# EUROPEAN PATENT SPECIFICATION

(11) **EP 4 004 548 B1**
(45) Date of publication and mention of the grant of the patent: **14.01.2026**
(21) Application number: 20715477.4
(22) Date of filing: 12.03.2020
(51) Int. Cl.: G01N 33/574, A61K 39/00, A61K 39/395

(54) **METHODS OF TREATING AND DIAGNOSING LUNG CANCER**
VERFAHREN ZUR BEHANDLUNG UND DIAGNOSE VON LUNGENKREBS
PROCÉDÉS DE TRAITEMENT ET DE DIAGNOSTIC DU CANCER DU POUMON

(30) Priority: 29.07.2019 US 201962879572 P; 28.11.2019 US 201962941754 P
(43) Date of publication of application: 01.06.2022
(73) Proprietor: Yeda Research and Development Co. Ltd, 7610002 Rehovot (IL)
(72) Inventor: MERBL, Yifat, 7610002 Rehovot (IL); JAVITT, Aaron, 7610002 Rehovot (IL); KACEN, Assaf, 7610002 Rehovot (IL); EISENBERG-LERNER, Avital, 7610002 Rehovot (IL); SHMUELI, Merav, 7610002 Rehovot (IL)
(74) Representative: Dennemeyer & Associates S.A.
(86) International application number: PCT/IL2020/050303
(87) International publication number: WO 2021/019526

(56) References cited:
- WO-A2-2005/011619
- US-A1- 2018 371 550
- V WELK ET AL: "Analyzing the role of proteasome activator 200 (PA200) in lung cancer", EUROPEAN RESPIRATORY JOURNAL, vol. 52, no. S62, 15 September 2018 (2018-09-15), pages pa2849, XP055698577

## Description

The present invention relates to a method of diagnosing non-small cell lung cancer in a subject comprising analyzing an
amount and/or an activity of PSME4 and at least one immunoproteasome catalytic subunit selected from the group consisting of PSMB8, PSMB9 and PSMB10 present in a non-small cell lung cancer tumor sample of said subject, wherein an increase in a ratio of amount and/or activity of said PSME4 to amount and/or activity of said at least one immunoproteasome catalytic subunit as compared to said ratio in a non-tumor sample is indicative of said non-small cell lung cancer.

The present invention relates also to a method of selecting a treatment for a subject diagnosed with a cancer, the method comprising determining an amount of PSME4 protein and an amount of at least one immunoproteasome catalytic subunit selected from the group consisting of PSMB8, PSM9, and PSMB10 present in cancer cells of said subject, wherein a ratio of amount of said PSME4 protein to amount of said at least one immunoproteasome catalytic subunit being below a predetermined threshold is indicative of suitability of said subject to treatment with an immune
checkpoint inhibitor. The two main types of lung cancer are small cell lung cancer (SCLC) and non-small cell lung cancer (NSCLC), the latter of which accounts for approximately 85% of all cases of lung cancer (Molina, J. R. et al., Mayo Clin Proc. 2008 May; 83(5): 584-594; Navada, S. et al., J Clin Oncol. 2006; 24(18S) suppl: 384S; Sher, T. et al., Mayo Clin Proc. 2008; 83(3): 355-367).

The primary risk factor for lung cancer is smoking, which accounts for more than 85% of all lung cancer-related deaths. The risk for lung cancer increases with the number of cigarettes smoked per day and the number of years spent smoking. In addition to the hazard of first-hand smoke, exposed nonsmokers have an increased relative risk for developing lung cancer. Radon gas, a radioactive gas that is produced by the decay of radium 226, is the second leading cause of lung cancer. The decay of this isotope leads to the production of substances that emit alpha-particles, which may cause cell damage and therefore increase the potential for malignant transformation.

There are five stages (Stage 0 to Stage IV) in NSCLC. Stages I, II and III are further subdivided into A and B subtypes. These stages are assigned based on a Tumor, Node and Metastasis (TMN) staging system.

Several biomarkers have emerged as prognostic and predictive markers for NSCLC (Ettinger, D. S. et al., J Natl Compr Canc Netw 2010; 8: 740-801). A prognostic biomarker, which is an indicator of innate tumor aggressiveness, is a biomolecule that indicates patient survival independent of the treatment received. A predictive biomarker is a biomolecule that indicates therapeutic efficacy, i.e., an interaction exists between the biomolecule and therapy that impacts patient outcome. Among these biomarkers, evidence is strongest for EGFR, the 5' endonuclease of the nucleotide excision repair complex (ERCC1), Kirsten rat sarcoma viral oncogene homolog (K-ras), and the regulatory subunit of ribonucleotide reductase (RRM1).

Specific targeted therapies have been developed for treating advanced lung cancer (Sandler, A. B. et al., Clin Cancer Res 2004; 10: 4258s-4262s; Giaccone, G. et al., J Clin Oncol 2005; 23: 3235-3242). Bevacizumab is a recombinant monoclonal antibody that blocksvascular endothelial growth factor (VEGF). Erlotinib is a small molecule inhibitor of EGFR. Cetuximab is a monoclonal antibody that targets EGFR. However, there is accumulating evidence that NSCLC acquires resistance to these specific targeted therapies (e.g., erlotinib and gefitinib) and how cancers such as NSCLC become resistant to, for example, EGFR inhibitors.

Background art includes WO2017/158610, US Patent Application No. 20160109453 and Wolf-Levy et al, 2018, Nat Bio., 10.1038/nbt.4279.

US 2018371550 relates to methods and compositions to determining suitability of immunotherapy for a subject having cancer, by determining whether tumor cells from a subject having cancer or one or more symptoms thereof have a loss of transcriptional fidelity (LTF) phenotype. It relates to methods of stratifying one or more subjects in a clinical trial by determining whether tumor cells from one or more subjects having cancer or one or more symptoms thereof have an LTF phenotype. It also relates to diagnostic kits, tests, or arrays to test for presence of a loss of transcriptional fidelity (LTF) phenotype in a sample.

WO 2005011619 relates to modulators of phosphatidic acid phosphatase type 2C and other polypeptides, highly expressed in cancers as compared to normal tissues, which are provided for treatment of proliferative disorders such as cancer. A method is provided for detecting polypeptides that are overexpressed in cancer, whereby antibodies or binding proteins that specifically recognize these molecules are contacted with a patient's bodily fluid. The method provides an early diagnosis of cancer, and can detect recurrence and metastasis following an initial diagnosis. It further provides methods of treating cancer with therapeutic agents directed toward these protein and peptide biomarkers.

### SUMMARY OF THE INVENTION

The present invention is set out in the appended set of claims.

Subject-matter that is marked herein as an aspect of the disclosure is not necessarily part of the invention as claimed.

### BRIEF DESCRIPTION OF THE SEVERAL VIEWS OF THE DRAWINGS

Some aspects of the disclosure are herein described, by way of example only, with reference to the accompanying drawings. With specific reference now to the drawings in detail, it is stressed that the particulars shown are by way of example and for purposes of illustrative discussion of aspects of the disclosure. In this regard, the description taken with the drawings makes apparent to those skilled in the art how aspects of the disclosure may be practiced.

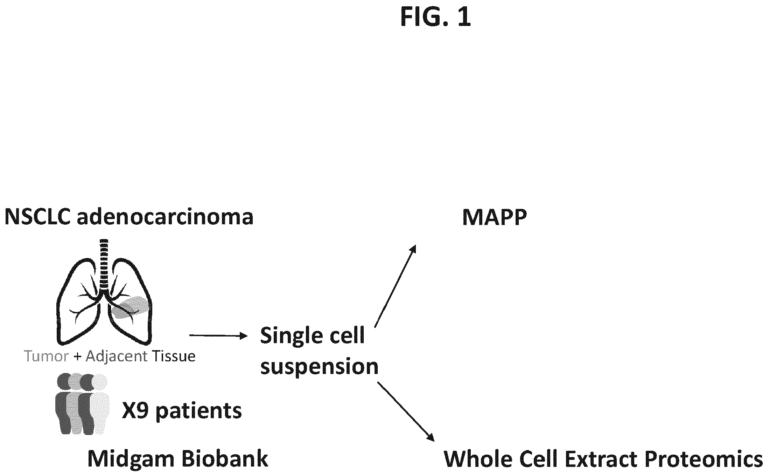

FIG. 1 Cohort description and project workflow. The lung adenocarcinoma tumor and adjacent lung tissue were obtained from 8 patients. Analysis was performed using standard whole cell extract proteomics to assess protein abundance and MAPP to study the degradomes of the sample.

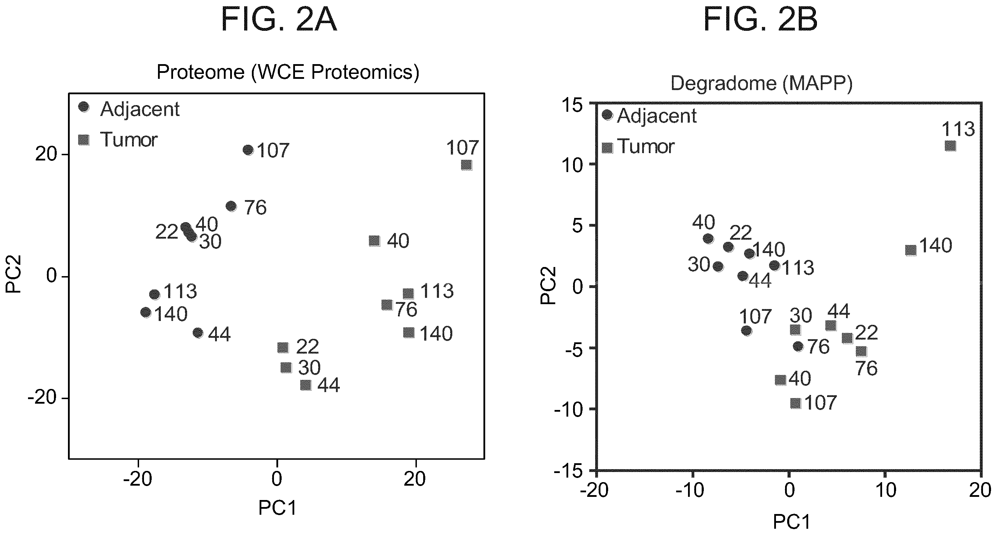

FIGs. 2A-B Proteome and Degradome landscapes classify tumor and adjacent tissues across patients. Principal Component Analysis based on the identities and abundances of the proteins identified by (A) Whole Cell Extract Proteomics or (B) MAPP are displayed.

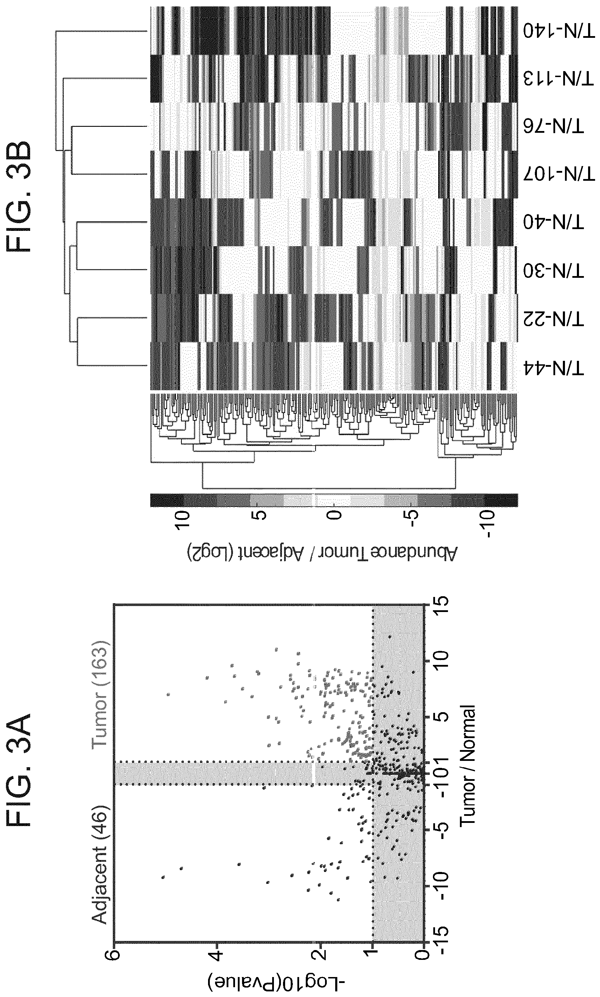

FIGs. 3A-B. Conserved interpatient degradation signature (A) The fold change in mean intensity between the tumor and adjacent samples for each protein identified by MAPP (x-axis) is plotted against the significance of the difference (y-axis). Proteins which are significantly enriched in the tumor (orange, n = 163) or adjacent samples (blue, n = 46) are colored. (B) The fold-change in intensity between tumor and adjacent samples for each patient are presented for the proteins which were identified as significantly enriched in A.

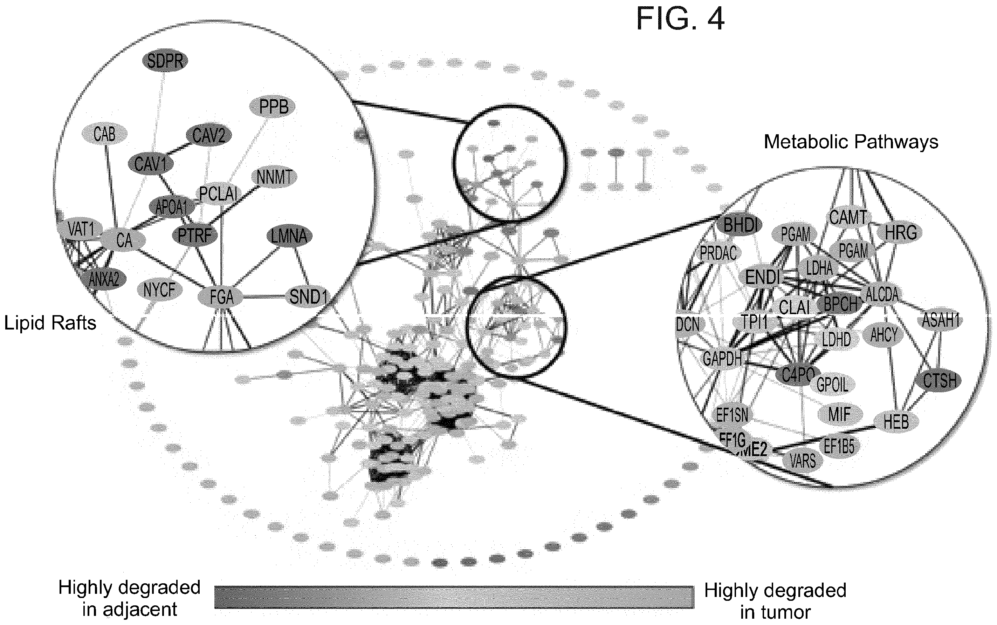

FIG. 4. Inter-patient conservation reveals altered degradation of proteins involved in lipid and metabolic pathways. For the proteins identified as significantly enriched in Figures 3A-B, a network based on their known interactions is generated. Two highly interconnected clusters were identified to be a group of proteins known to function in lipid raft assembly and metabolic pathways respectively. Color of the node indicates the mean fold-change in intensity as identified by MAPP between the tumor and adjacent samples.

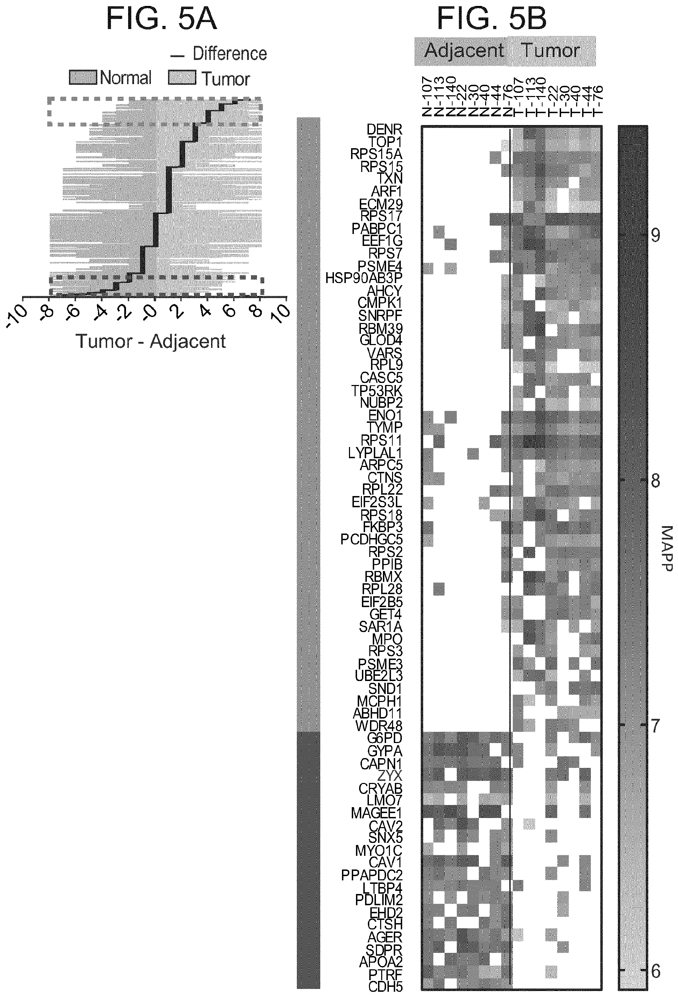

FIGs. 5A-B. Proteins selectively degraded in tumor or adjacent tissue across patients. (A) Each row is a protein, the orange bar indicates the number of patients that protein was identified as degraded in the tumor, blue bar the number of adjacent tissues. Proteins which were differential are boxed in orange or blue for tumor or adjacent respectively. The MAPP intensity across samples of the proteins identified is indicated in red.

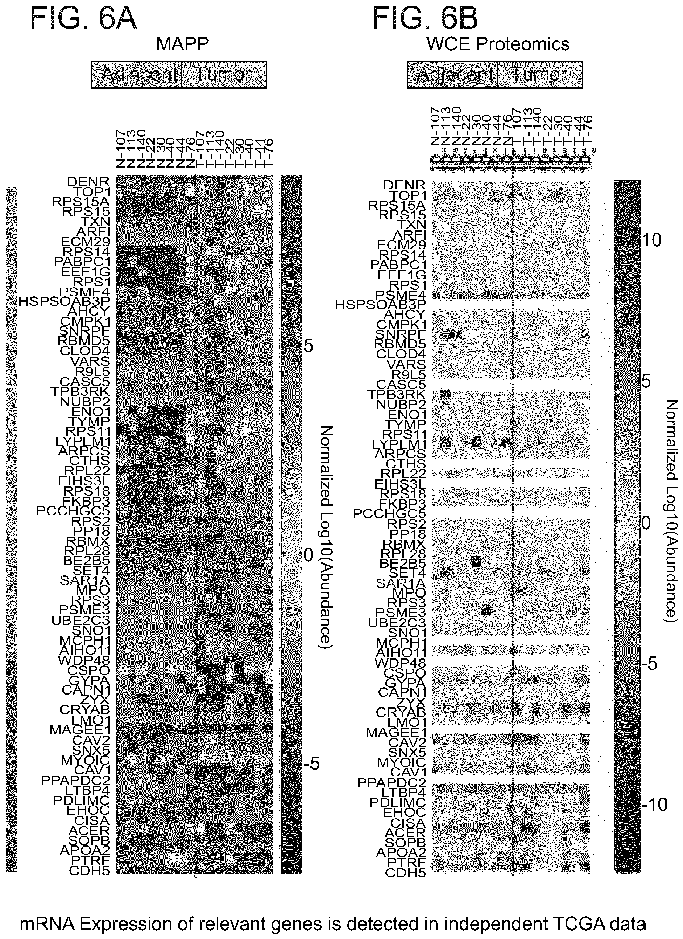

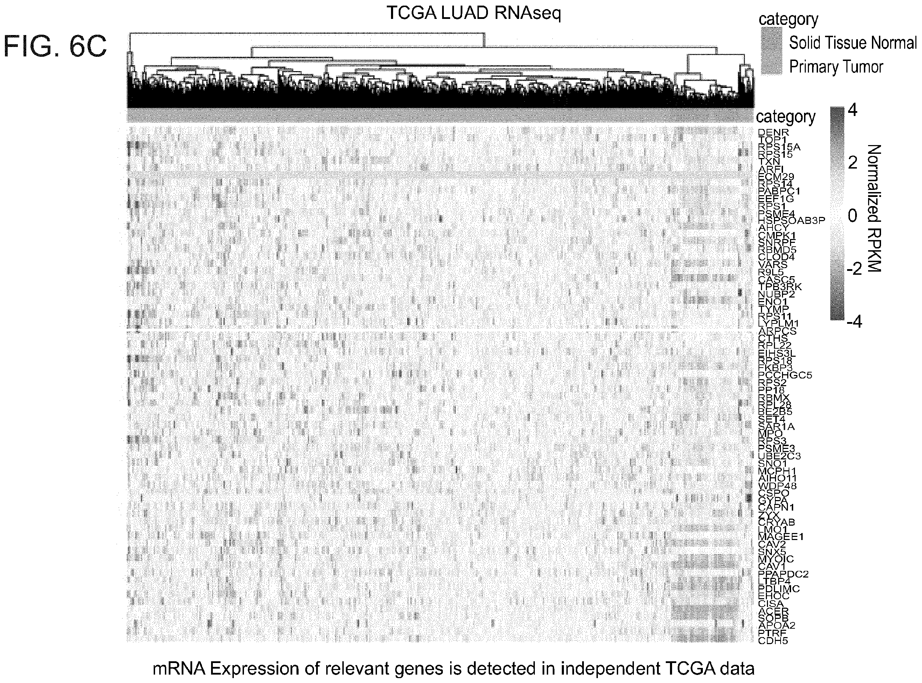

FIGs. 6A-C. Multi-level profiles of selectively degraded proteins. The (A) MAPP intensity, (B) WCE Proteomics Abundance or (C) mRNA expression for each protein identified as differential in Figures 5A-B. mRNA expression is from TCGA RNAseq of other lung adenocarcinoma samples (orange) or normal lung tissue (blue).

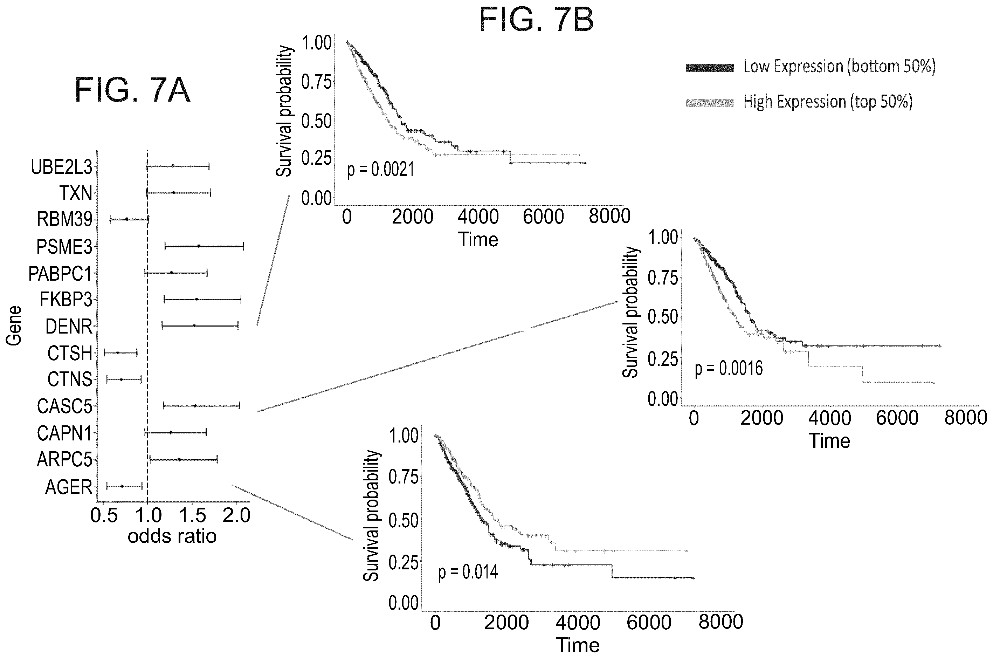

FIGs. 7A-B. Antigens presented from differential targets. (A) The number of antigens identified from select differential proteins as presented on MHC in the IEDB database. (B) Mutations identified in four of the differential proteins suggesting potential neoantigen targets.

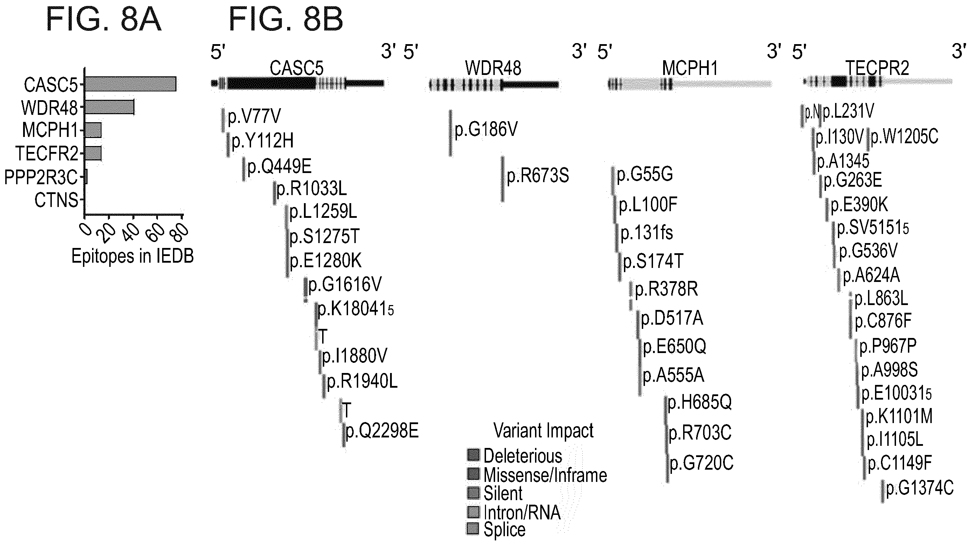

FIGs. 8A-B. Expression of differentially degraded proteins significantly impact patient survival rates. (A) Odds ratios of survival based on dividing the TCGA LUAD cohort into high and low expressors of each of the genes indicated. (B) The Kaplan-Meyer survival curves of the LUAD cohort stratified by the indicated gene. DENR and CASC5 expression reduce survival AGER expression improves survival.

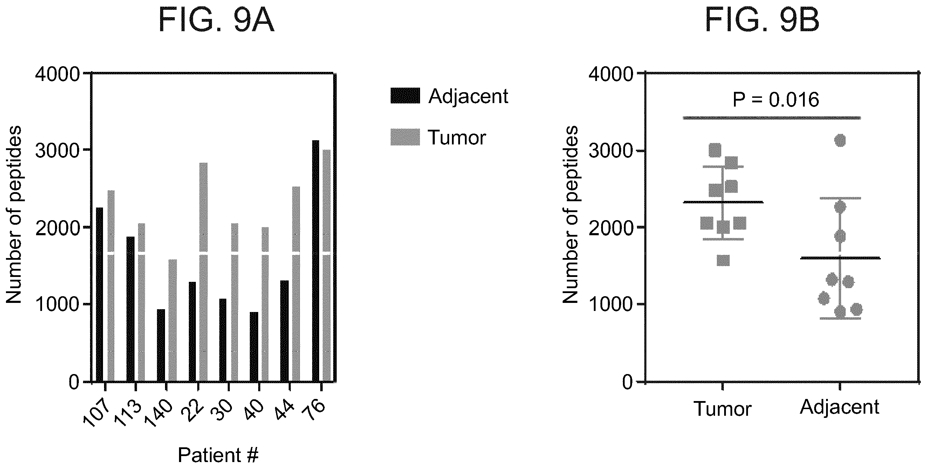

FIGs. 9A-B. More peptides identified in tumor degradome than adjacent controls. (A) The number of peptides identified by MAP for each of the samples. Sample 81 was excluded from all other analysis due to low peptide number. (B) There a significant increase in the number of peptides identified in the tumor samples compared to the adjacent tissue. (Students t-test, p = 0.016).

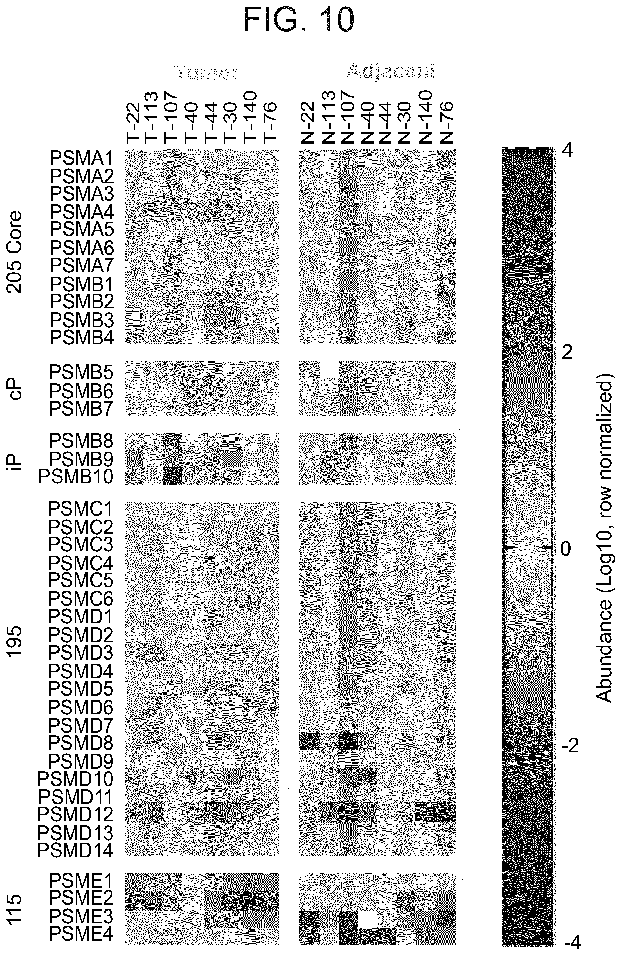

FIG. 10. Proteasome composition is altered between tumor and adjacent samples. The abundance of the proteasome subunits in each of the samples from the WCE proteomics data.

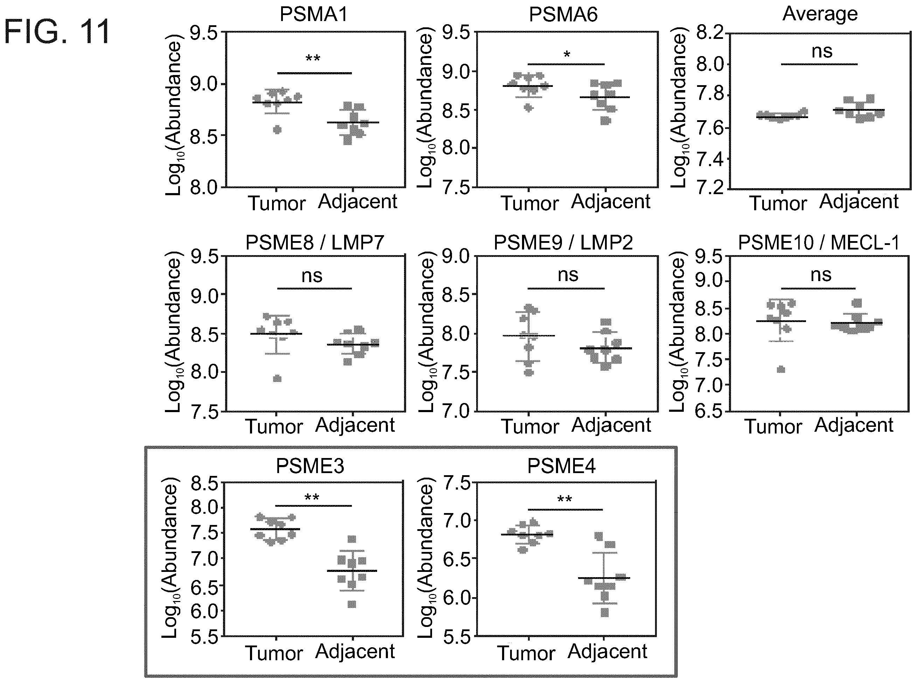

FIG. 11. Proteasome composition is altered between tumor and adjacent samples. There is no significant change in core subunits (first row), immunoproteasome catalytic subunits (second row) or average total protein abundance (top right) between the tumor and adjacent tissue. However, there is a highly significant increase in two regulators, PSME3 + PSME4 (bottom row, students t-test ** P<0.01).

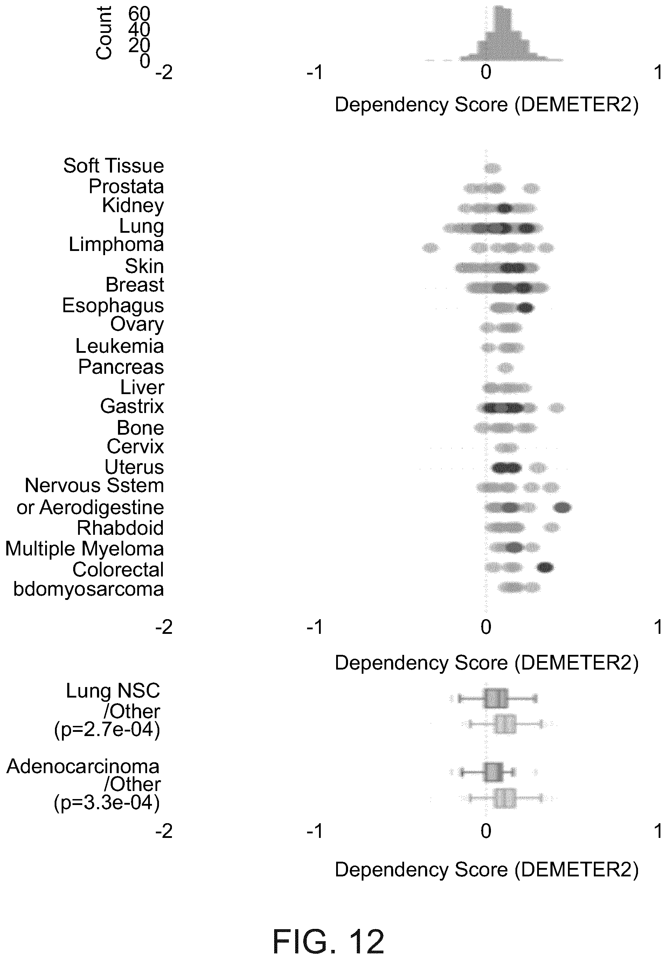

FIG. 12. NSCLC viability is affected by PSME4 knockdown. The DEMETER2 score for cell line or tissue dependency on PSME4 for the tissue groups listed. Only NSCLC and Lung Adenocarcinoma had a significant decrease in DEMETER2 score as compared to the other tissues.

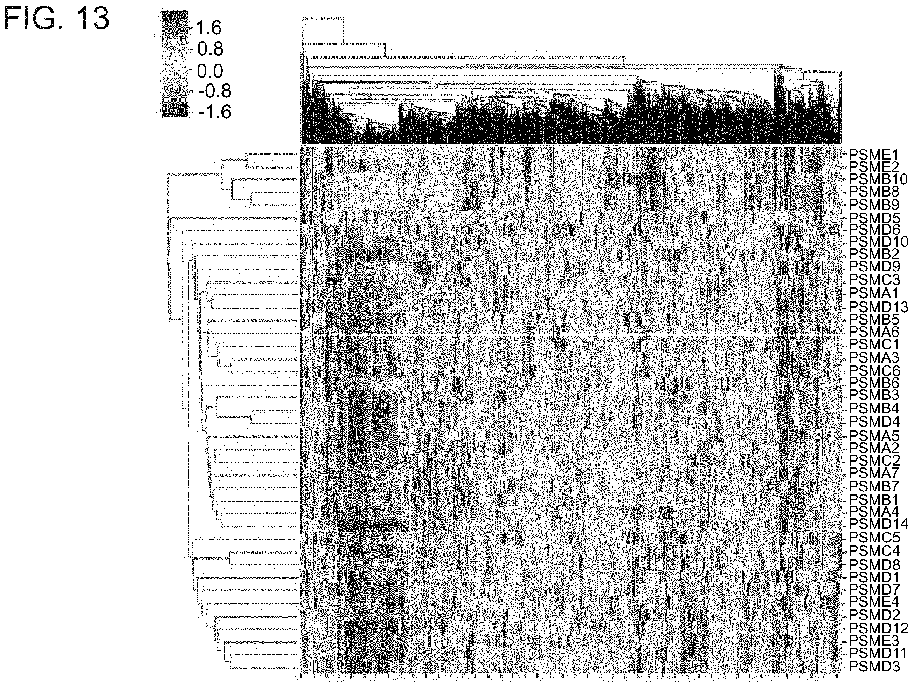

FIG. 13. Expression of proteasome subunits in LUAD cohort. The mRNA expression of all of the proteasome subunits across the samples in the TCGA LUAD cohort.

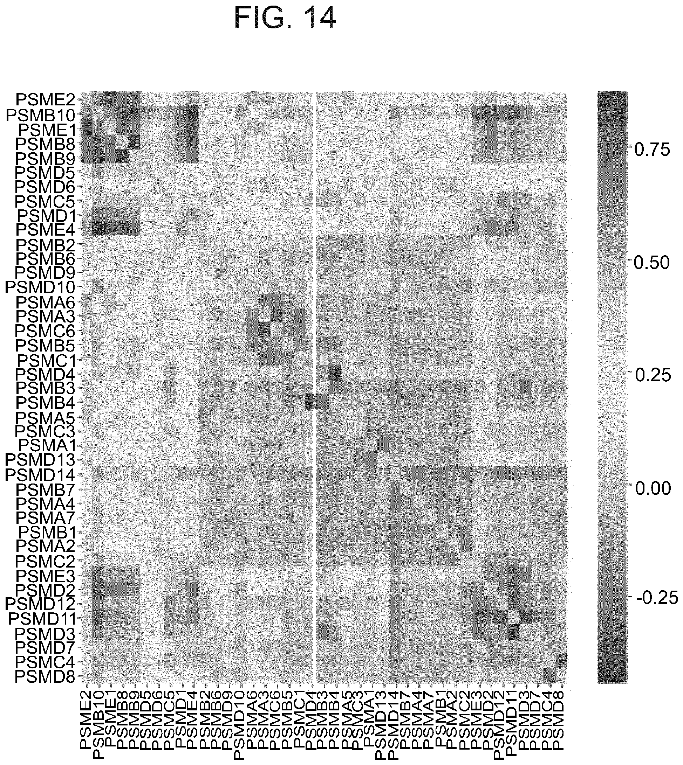

FIG. 14. Pairwise correlation of proteasome subunits in LUAD. The pairwise spearman correlation was calculated for all the proteasome subunits based on their expression in the LUAD cohort. Correlations were clustered to reveal groups of subunits which co-express.

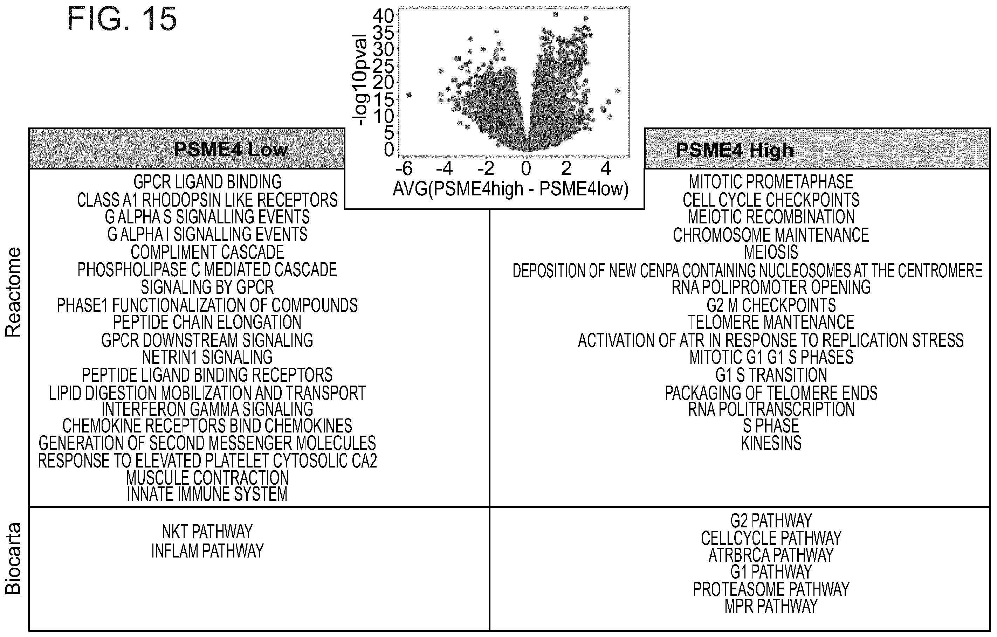

FIG. 15. Pathway enrichment of LUAD binned by PSME4 expression. The 20% of the LUAD cohort with the highest PSME4 expression was compared to the lowest 20%. Pathways in the reactome or biocarta annotation sets which were found by GSEA to be significantly enriched in the PSME4 high or low group are listed.

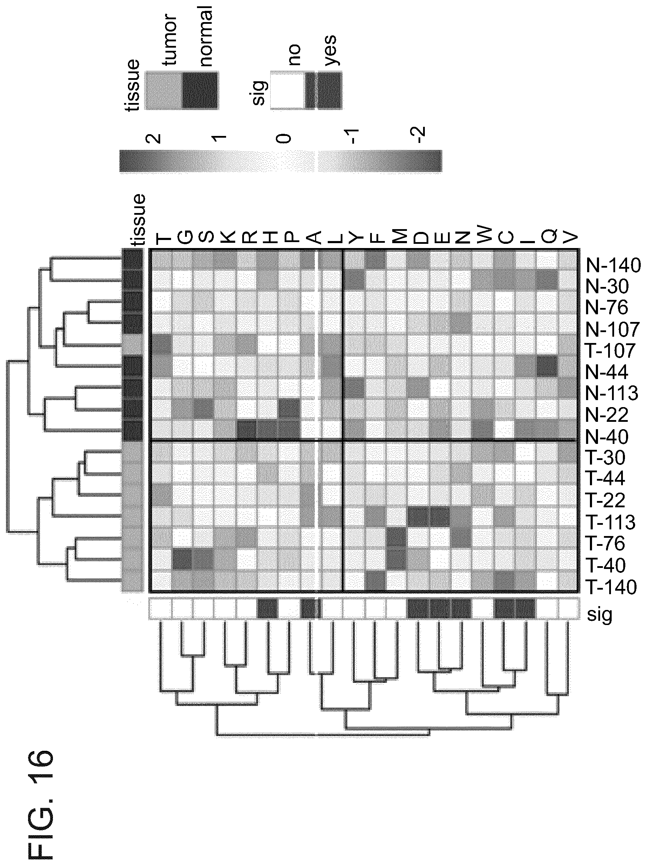

FIG. 16. Peptide carboxyl termini differ across healthy and tumor tissue. MAPP peptides were counted based on their carboxyl-terminal residue. The normalized count across samples for the number of peptides ending in each residue are displayed. With the exception of one sample, tumor (orange) and adjacent (blue) samples cluster together. Any amino acid where the count of peptides significantly differed (p <=0.05) between the tumor and adjacent samples was indicated in green.

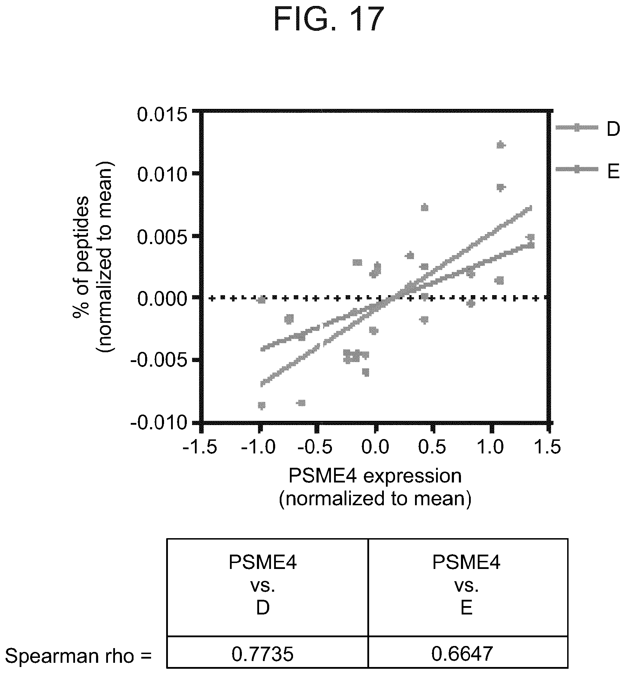

FIG. 17. Post glutamyl cleavage activity increases in tumor tissue in correlation with PSME4 levels. The abundance of PSME4 (x-axis) is plotted against the percentage of MAPP peptides with a D or E at their carboxyl-terminus in all the samples in the cohort. There is a high correlation between PSME4 abundance and D (rho = 0.77) or E (rho= 0.665) percentages.

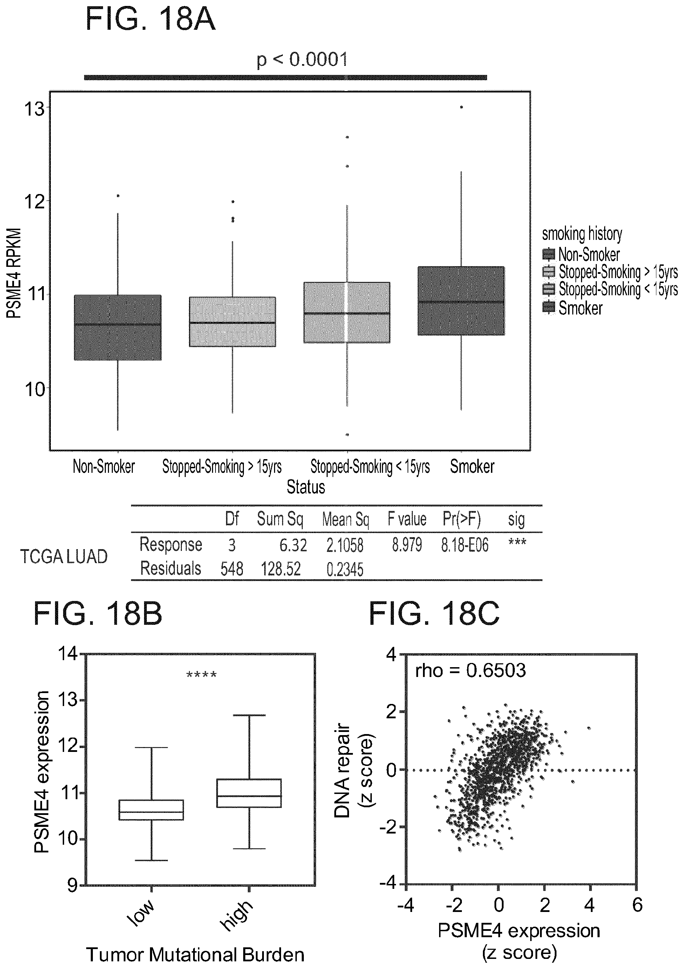

FIGs. 18A-C. Smoking increases PSME4 expression in NSCL. (A) Patients in the TCGA LUAD cohort were stratified based on their smoking history. A boxplot of the expression of PSME4 in each of the different groups is displayed. There is a significant increase in PSME4 in the tumors of patients who were Smokers versus Non-Smokers (Students T test, P<0.0001) and a significant connection between PSME4 level and smoking history (2 way Anova - details in figure). (B) Tumors were divided into those with high and low Tumor Mutational Burden and there was a significant difference in PSME4 expression between the 2 groups (student's T test ****P<0.0001). (C) A Z score was given to each tumor based on a genetic signature of DNA repair. This score correlated with the expression of PSME4 across the samples in the cohort (spearman rho = 0.6503).

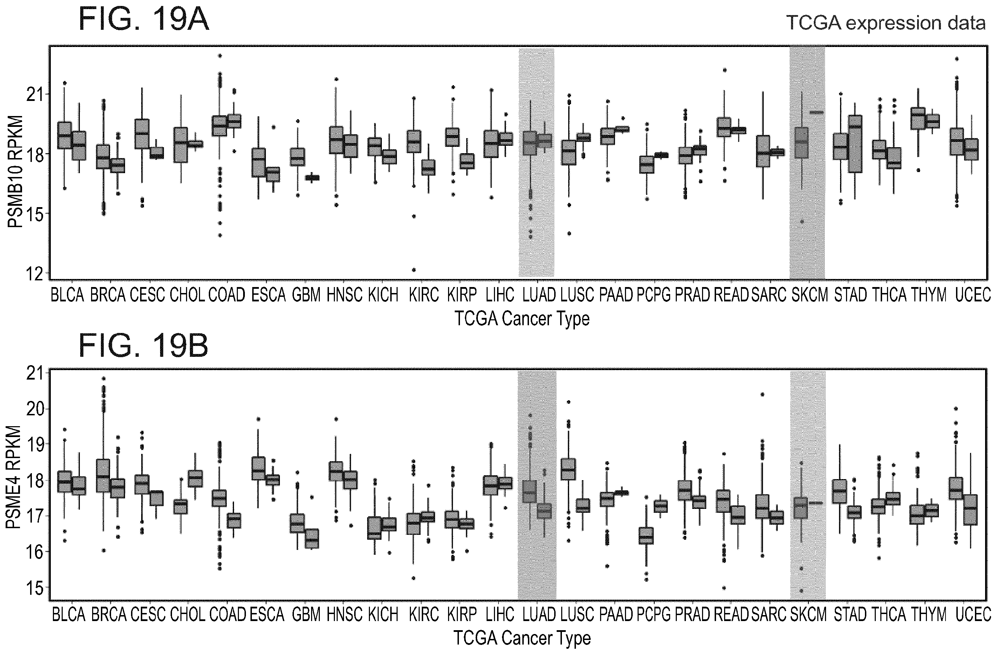

FIGs. 19A-B. Dynamic change in proteasome composition across cancers. The expression of (A) PSMB10 or (B) PSME4 across the TCGA PANCAN cohort for both tumor and normal/adjacent samples.

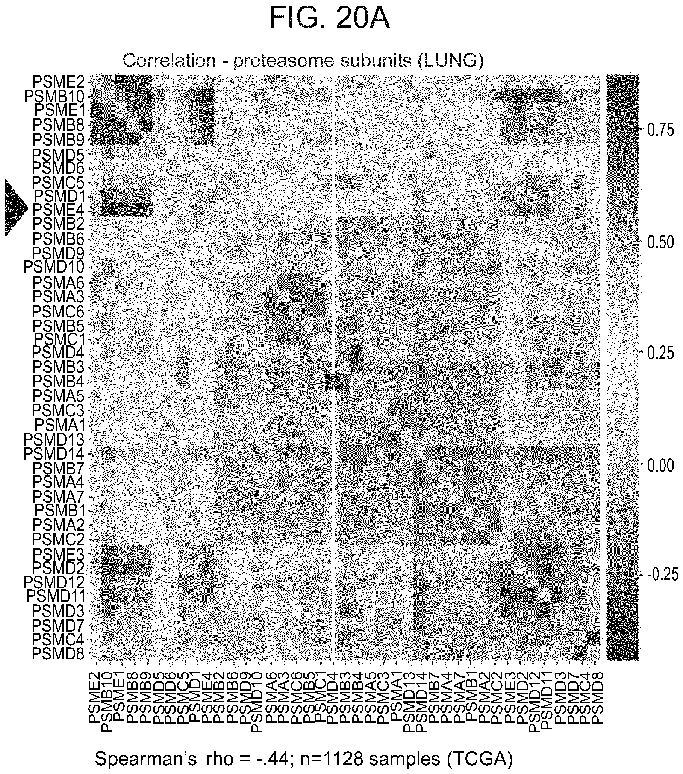

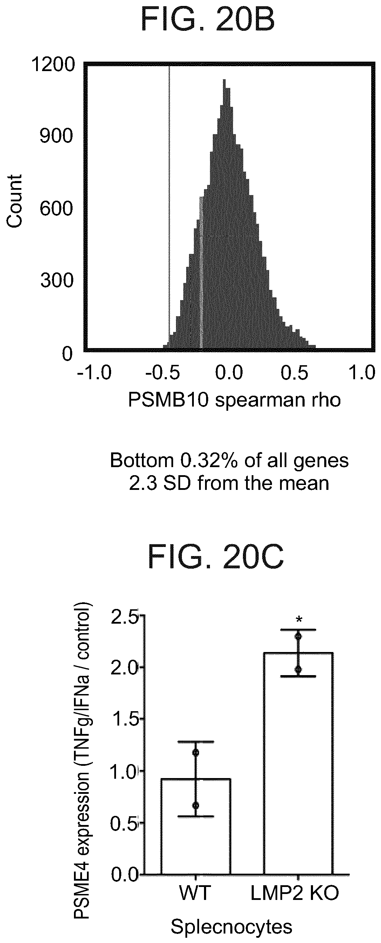

FIGs. 20A-C. PA200 and the immunoproteasome subunit PSMB10/β2i are anti-correlated. (A) Pairwise spearman correlation of the expression each of the proteasome subunits across the TCGA LUNG cohort. PSME4 and PSMB10 are the most anticorrelated (spearman rho = -0.44). (B) The distribution of the correlation between all of the genes in the TCGA cohort and PSMB10 (grey) compared to the correlation with PSME4 (red line). (C) The fold change in expression of PSME4 following stimulation with TNFα and IFNγ in splenocytes from either wild-type (WT) or immunoproteasome knockout (LMP2 KO) mice.

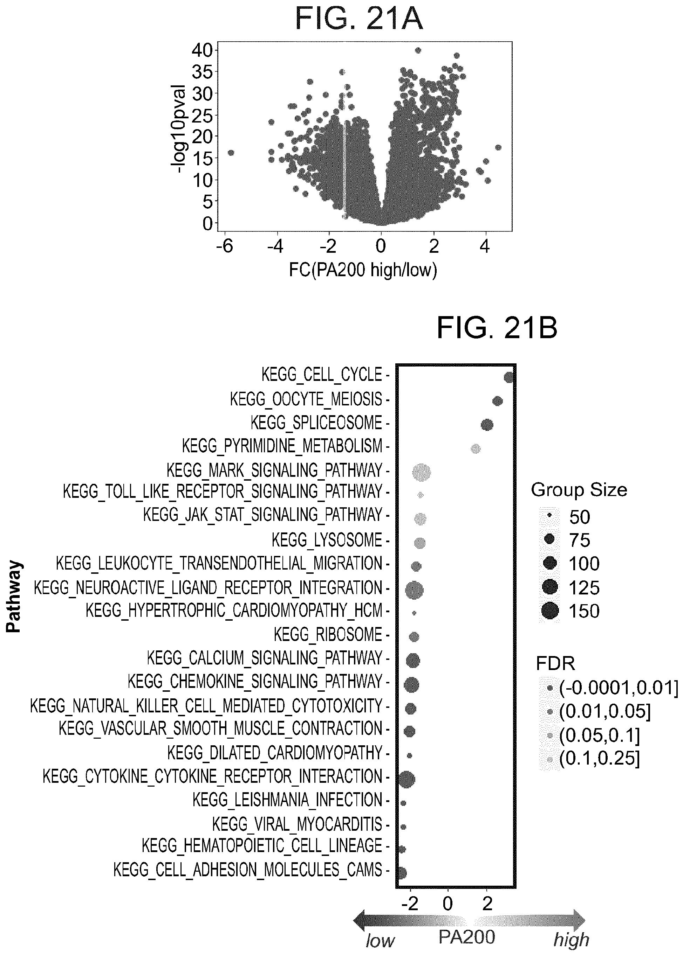

FIGs. 21A-B. Low expression of PSME4/PA200 is associated with increased inflammation. (A) The TCGA LUAD cohort was divided into the top and bottom 20% of PSME4 expressers. The fold change and significance of difference (student t-test) for each gene was plotted. (B) GSEA was used to find pathways that are enriched the cohort of high or low PSME4 expressers. The enrichment score of the pathway is displayed on the X axis, dot size corresponds to the size of the gene group and color indicates FDR score. Pathways of particular interest are marked in a black triangle.

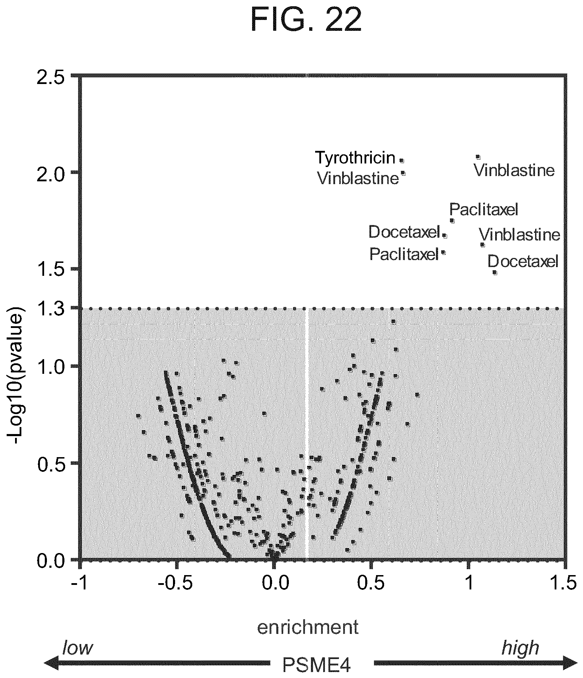

FIG. 22. Increased PSME4 expression sensitizes cells to cell cycle inhibitors. The change in IC50 between cell lines with high and low PSME4 expression (x axis) is plotted against the significance of the change (benjamini-hochberg corrected student's t-test; y axis).

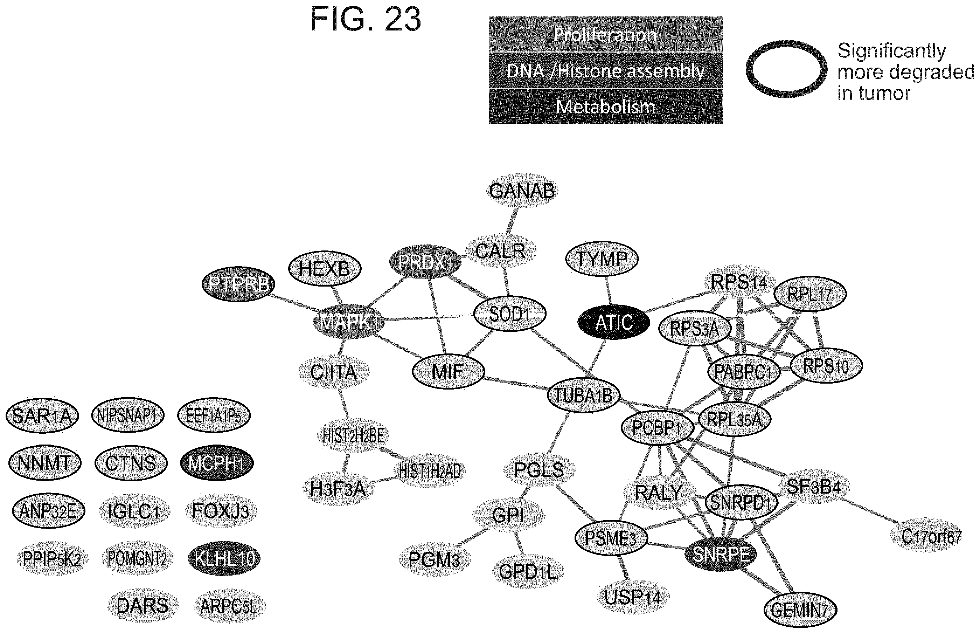

FIG. 23. PSME4 correlated substrates include regulators of metabolism and cell proliferation. Substrates whose degradation is highly correlated with PSME4 abundance across the tumor cohort are displayed. Proteins annotated to have a role in cell proliferation, DNA architecture or cell metabolism are colored in green, blue and black respectively. Of the substrates, those which are also significantly degraded more in the tumors than the adjacent samples are outlined in black.

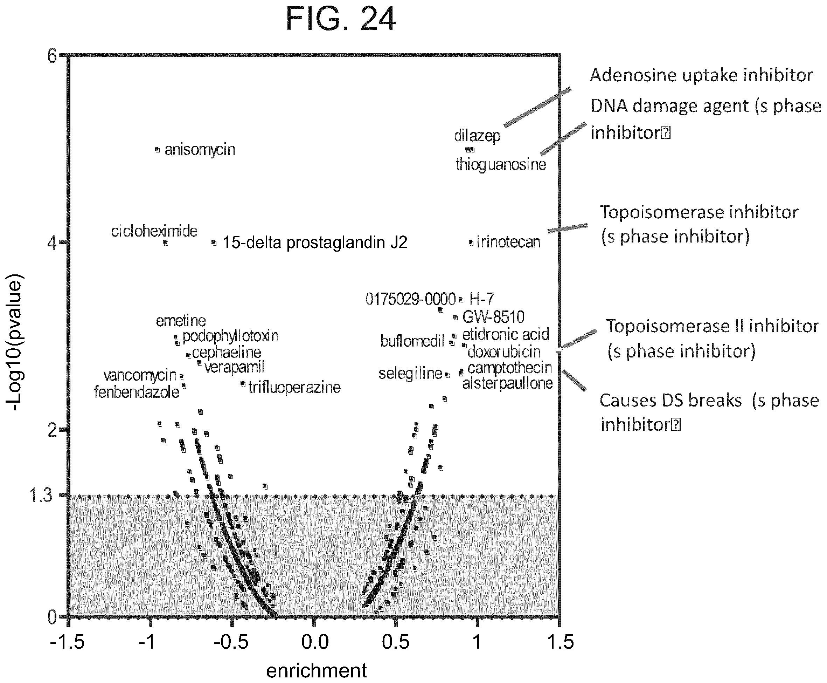

FIG. 24. PSME4 substrate expression sensitizes to DNA damage. The changes in IC50 of FDA approved drugs in CMAP that correlated with perturbations in the signature of PSME4 substrates in Fig23. Change in activity (x axis) is plotted against the significance of the change (benjamini-hochberg corrected student's t-test; y axis). Drugs which cause DNA damage are labeled on the graph with their M.O.A.

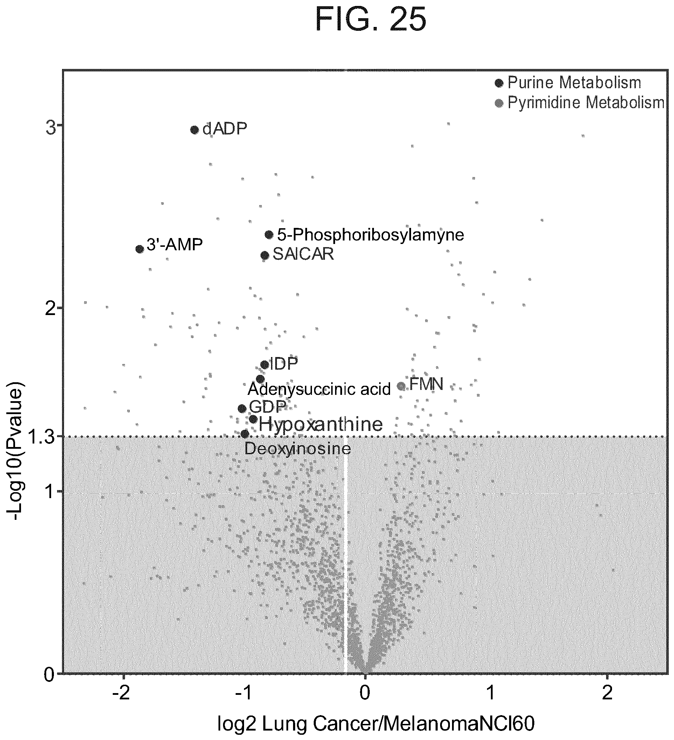

FIG. 25. Purine metabolism enriched in melanoma versus lung cancer metabolites. The log2 fold change in abundance of cellar metabolites in lung cancer or melanoma cell lines (x axis) is plotted against the significance of the change. Metabolites involved in purine or pyrimidine metabolism are labeled blue and orange respectively.

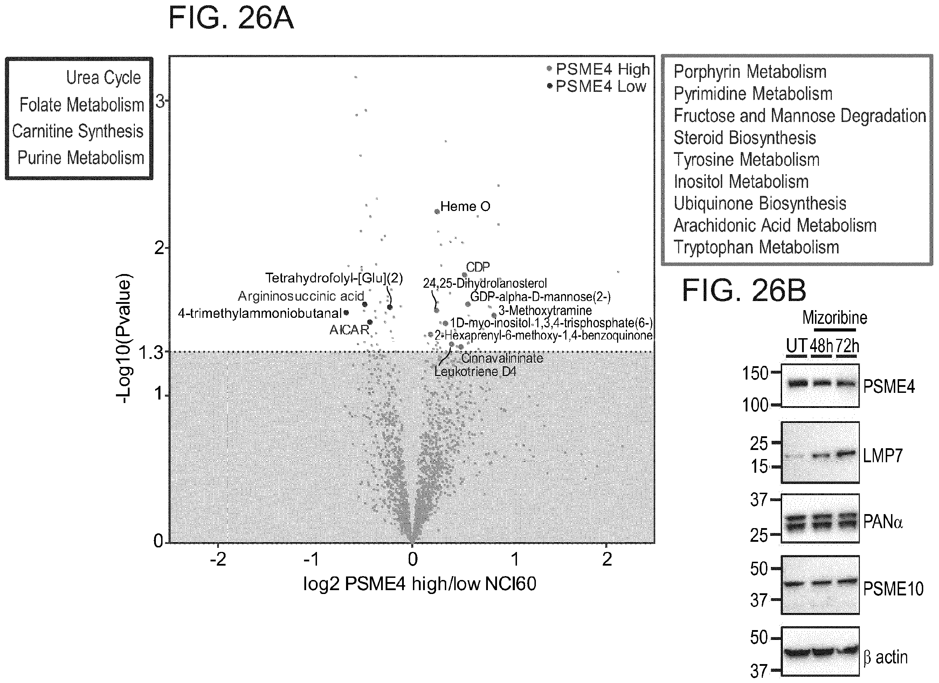

FIG. 26A-B. PSME4 expression correlates with increased Pyrimidine and decreased Purine metabolites. (A) The log2 fold change in abundance of cellar metabolites in PSME4 high or low expressing cell lines (x axis) is plotted against the significance of the change. The pathways that the metabolites are annotated to are displayed in blue and orange boxes. (B) A549 cells were treated with mizoribine for the time indicated and then separated by SDS-PAGE and analyzed by Western blot probed with the following antibodies: PSME4, LMP7 (PSMB8), PSMB10, panα (recognized all PSMA subunits), and β actin.

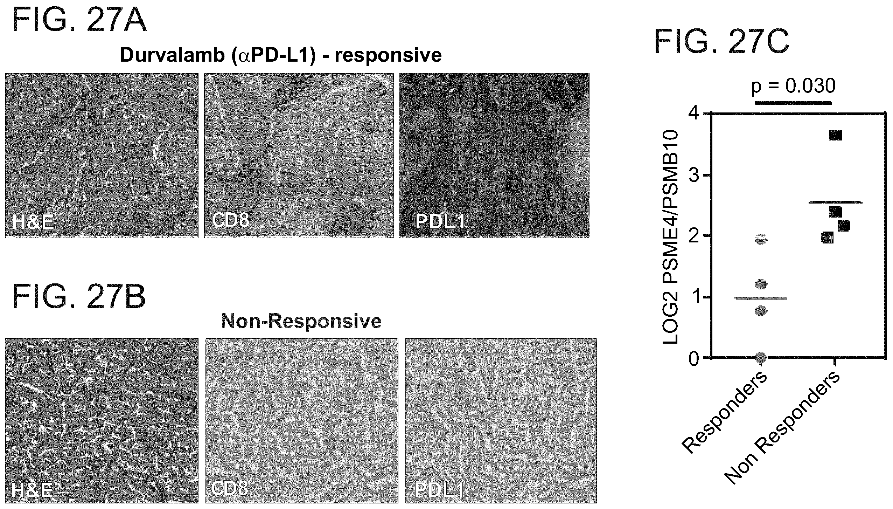

FIGs. 27A-C. PSME4/PSMB10 ratio is correlated with response to immunotherapy in NSCLC. Representative image of histology slice from a tumor that (A) responded or (B) did not respond to durvalamab therapy. H&E, anti CD8 and anti PDL1 stains are displayed. (C) The log2 ratio between PSME4 and PSMB10 expression as determined by qPCR from cDNA of 4 tumors which responded to therapy, and 4 that did not. There was a significant increase in the ratio in non-responders (students t-test, p = 0.030)

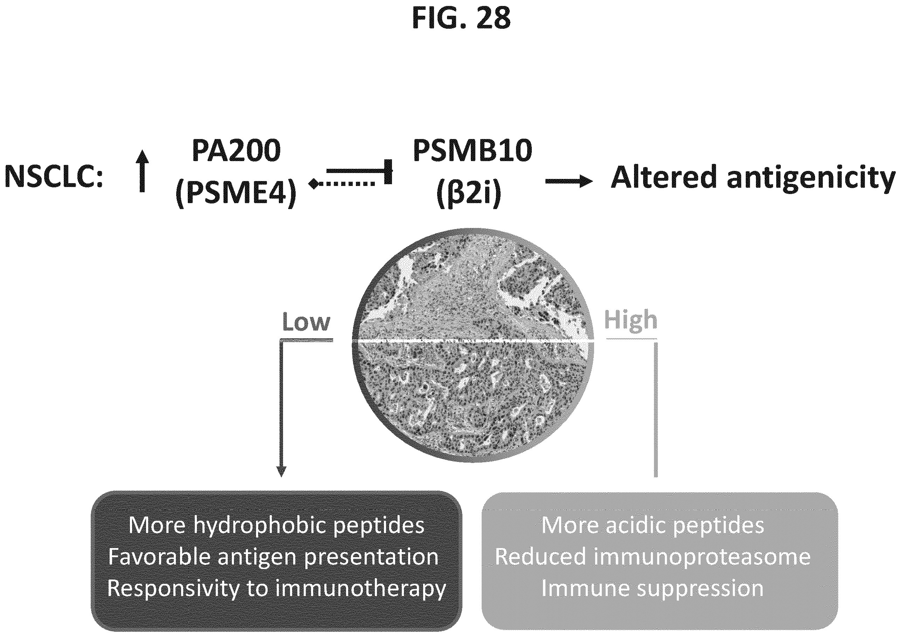

FIG. 28. Hypothesis: Increased PSME4 levels in NSCLC drive immune suppression and attenuate the response to immunotherapy.

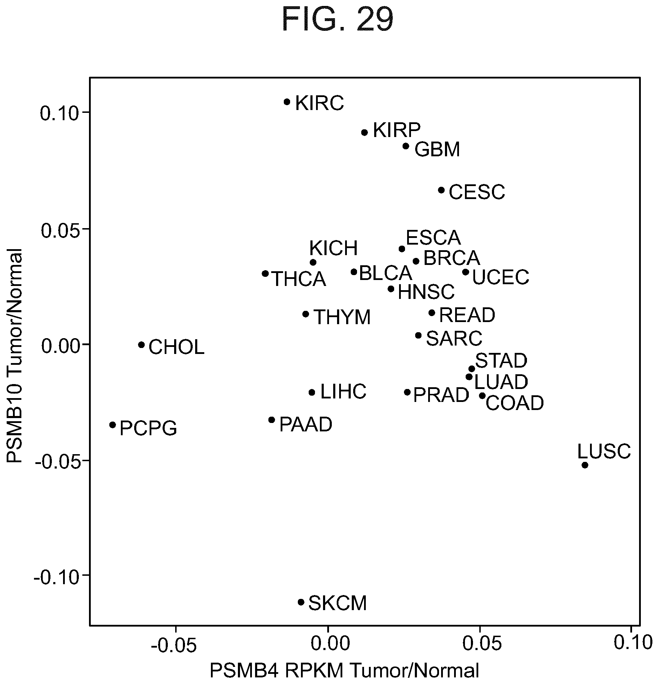

FIG. 29. Expression of PSME4 and PSMB10 across cancers. The ratio PSME4 (x-axis) or PSMB10 (y-axis) between mean expression in tumor or normal samples was plotted across cancers based on TCGA data.

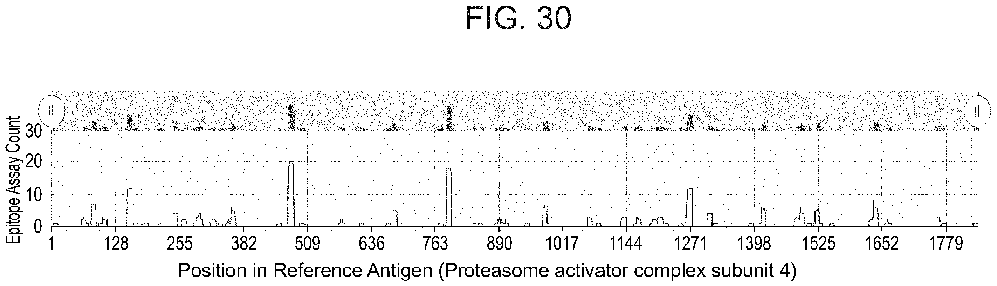

FIG. 30. Presented Peptides from PSME4. The 87 peptides identified as presented on MHC peptides in IEDB are displayed with their location in the protein sequence.

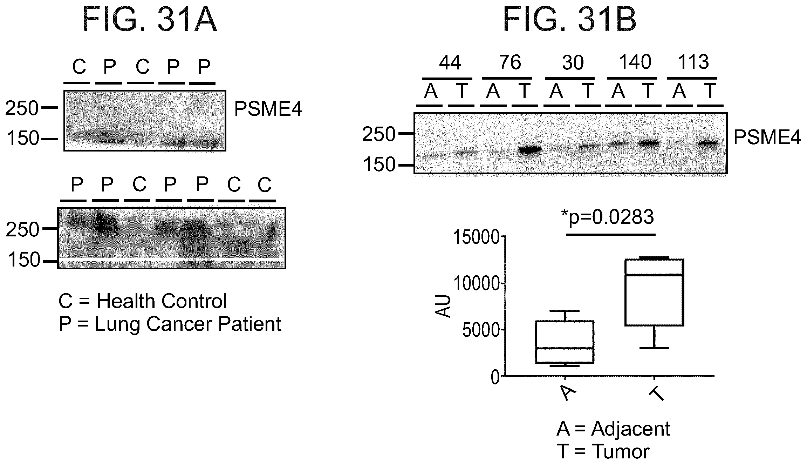

FIGs. 31A-B illustrates that PSME4 is a biomarker in serum (Figure 31A) and tumor samples (Figure 31B).

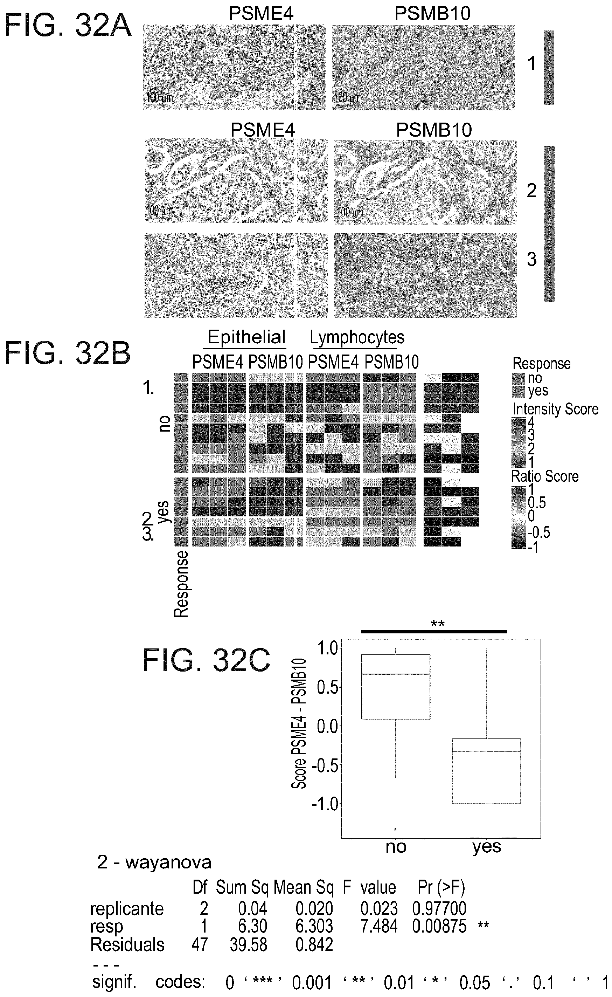

FIGs. 32A-C illustrate that non-responders have significantly higher PSME4/PSMB10 ratio in tumors. (Figure 32A) Representative images of the responders (from sample # 1) and non-responders (from samples # 2 and #3) with the PSME4 and PSMB10 staining, examples 1-3 from 32A are indicated. (Figure 32B) Heatmap of the scoring, and the ratio between PSME4 and PSMB10 from each tumor. (Figure 32C) The average ratio of the non-responders is significantly higher than the responders (2-way ANOVA p = 0.0088).

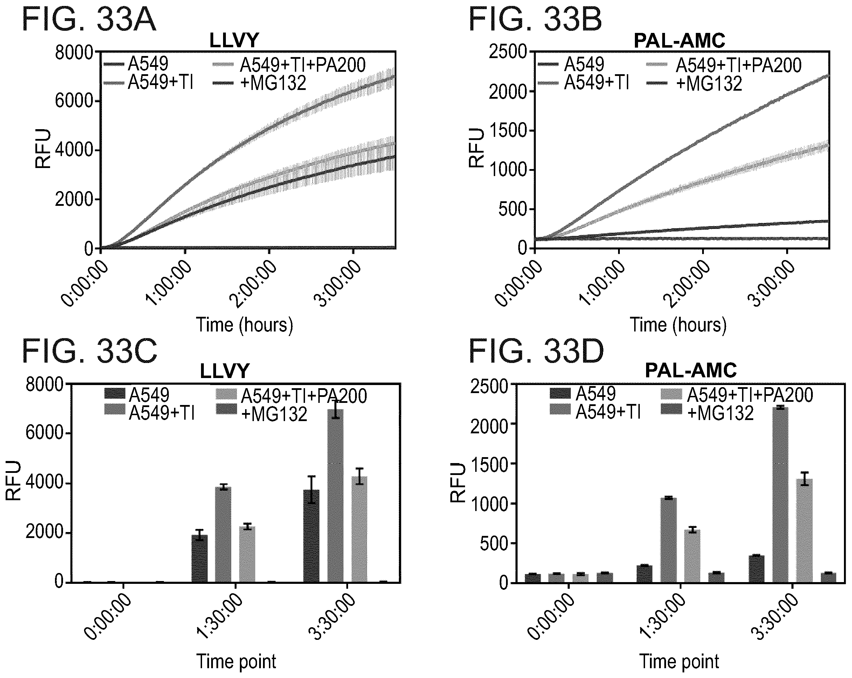

FIGs. 33A-D Active cell extract was prepared from A549 lung cancer cells, cells treated with TNFα and IFNγ (TI) and cells treated with TI and recombinant PA200. As a control, MG132 proteasome inhibitor was added. The extracts were then incubated with LLVY-AMC (SEQ ID NO: 1) (A) or PAL-AMC (SEQ ID NO: 2) (B) for 3.5 hours and the relative fluorescence units (RFU) was measured every minute. The RFU for each condition using LLVY-AMC (SEQ ID NO: 1) (C) or PAL-AMC (SEQ ID NO: 2) (D) is shown at 1.5 and 3.5 hours across the different repeats of the experiment.

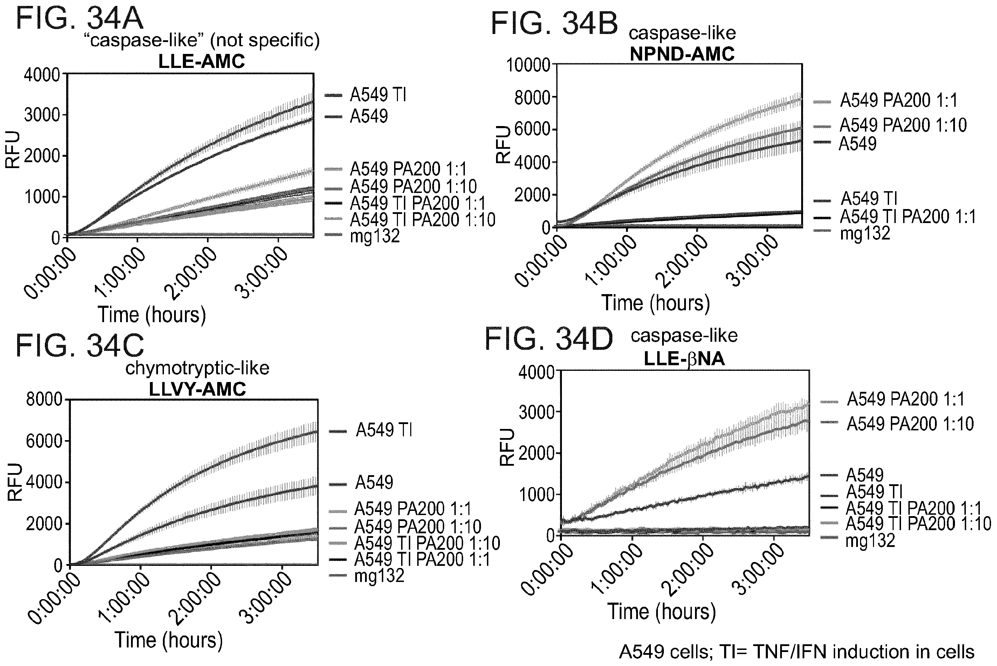

FIGs. 34A-D. Active cell extract was prepared from A549 lung cancer cells and cells treated with TNFα and IFNγ (TI). To each of these PA200 was added at a concentration of 4.7nm (1:1) or .47nm (1:10). As a control, MG132 proteasome inhibitor was added. The extracts were then incubated with LLE-AMC (SEQ ID NO: 3) (A), NPND-AMC (SEQ ID NO: 4) (B), LLVY-AMC (SEQ ID NO: 1) (C) or LLE-βNA (SEQ ID NO: 5) (D) for 3.5 hours and the relative fluorescence units (RFU) was measured every minute.

### DESCRIPTION OF SPECIFIC ASPECTS OF THE DISCLOSURE

The present disclosure, in some aspects thereof, relates to methods of treating and diagnosing lung cancer and, more particularly, but not exclusively, to methods of treating and diagnosing non-small cell lung cancer (NSCLC).

NSCLC is the leading cause of cancer mortality worldwide. Advanced NSCLC patients are currently treated with checkpoint inhibitors (CPI) (with or without chemotherapy) as a first-line treatment, based on recently reported advancements. While about half of patients respond to this treatment, the other half does not respond and within a year 80% of tumors have progressed. NSCLC patients with tumor progression on immune and chemo combination therapy have basically exhausted the currently validated treatment options. Thus, there is a great demand for novel therapeutic targets for NSCLC and revealing underlying mechanisms involved in the pathophysiology of NSCLC should shed new light on potential interventions.

Recently, the present inventors have developed a novel approach termed Mass Spectrometry Analysis of Proteolytic Peptides (MAPP), which allows detection of peptides that were endogenously cleaved by cellular proteasomes (WO2017/158610). While this system has already been applied and utilized in *in vitro* studies of cell lines in culture and liquid biopsies from lupus patients, application of MAPP to solid tumors has not been tried. The present inventors have now sought to examine the degradation landscape in clinical samples resected from patients by comparing MAPP-generated profiles from tumor tissues and an adjacent control tissue, based on pathologic examination.

Using this technology, the present inventors uncovered a myriad of proteins whose degradation pattern was unique to tumor tissue derived from NSCLC patients. The present inventors propose that these proteins can serve both as biomarkers for diagnosing the disease and as potential targets for therapy.

Thus, according to one aspect of the present disclosure there is provided a method of treating lung cancer of a subject in need thereof comprising administering to the subject a therapeutically effective amount of an agent that downregulates an amount and/or activity of a polypeptide selected from the group consisting of CASC5, MYOF, CTNS, FCGR2B, PCDHGC5, POMGNT2, ACSL1, CTAGE5, TECPR2, WDR48, MCPH1, PPP2R3C, ADRB1, JAG2, GEMIN7, PTPRB, PRMT9, PSME4, Ube2L3, TP53RK and PSME3, thereby treating the lung cancer.

Contemplated polypeptides which can be regulated or analyzed are summarized in Table 1, herein below.

**Table 1**

| **Entry name** | **Protein names** | **Gene names** | **Cross-reference (GeneID)** |
|---|---|---|---|
| KNL1_HUMAN | Kinetochore scaffold 1 (ALL1-fused gene from chromosome 15q14 protein) (AF15q14) (Bub-linking kinetochore protein) (Blinkin) (Cancer susceptibility candidate gene 5 protein) (Cancer/testis antigen 29) (CT29) (Kinetochore-null protein 1) (Protein CASC5) (Protein D40/AF15q14) | KNL1 | |
| | | CASC5 | |
| | | KIAA1570 | 57082 |
| | | MYOF | |
| | | FER1L3 | |
| MYOF HUMAN | Myoferlin (Fer-1-like protein 3) | KIAA1207 | 26509 |
| CTNS HUMAN | Cystinosin | CTNS | 1497 |
| | Low affinity immunoglobulin gamma Fc region receptor II-b (IgG Fc receptor II-b) (CDw32) (Fc-gamma RII-b) (Fc-gamma-RIIb) (FcRII-b) (CD antigen CD32) | FCGR2B | |
| | | CD32 | |
| | | FCG2 | |
| FCG2B HUMAN | | IGFR2 | 2213 |
| PCDGM HUMAN | Protocadherin gamma-C5 (PCDH-gamma-C5) | PCDHGC5 | 56097 |
| | Protein O-linked-mannose beta-1,4-N-acetylglucosaminyltransferase 2 (POMGnT2) (EC 2.4.1.312) (Extracellular O-linked N-acetylglucosamine transferase-like) (Glycosyltransferase-like domain-containing protein 2) | POMGNT2 | |
| | | AGO61 | |
| | | C3orf39 | |
| | | EOGTL | |
| PMGT2 HUMAN | | GTDC2 | 84892 |
| | Long-chain-fatty-acid--CoA ligase 1 (EC 6.2.1.3) (Acyl-CoA synthetase 1) (ACS1) (Arachidonate--CoA ligase) (EC 6.2.1.15) (Long-chain acyl-CoA synthetase 1) (LACS 1) (Long-chain acyl-CoA synthetase 2) (LACS 2) (Long-chain fatty acid-CoA ligase 2) (Palmitoyl-CoA ligase 1) (Palmitoyl-CoA ligase 2) (Phytanate--CoA ligase) (EC 6.2.1.24) | ACSL1 | |
| | | FACL1 | |
| | | FACL2 | |
| | | LACS | |
| | | LACS1 | |
| ACSL1 HUMAN | | LACS2 | 2180 |
| | Melanoma inhibitory activity protein 2 (CTAGE family member 5 ER export factor) (Meningioma-expressed antigen 6/11) | MIA2 | |
| | | CTAGE5 | |
| | | MEA 11 | |
| | | MEA6 | |
| | | MGEA 11 | |
| MIA2 HUMAN | | MGEA6 | 4253 |
| | Tectonin beta-propeller repeat-containing protein 2 (WD repeat-containing protein KIAA0329/KIAA0297) | TECPR2 | |
| | | KIAA0297 | |
| TCPR2 HUMAN | | KIAA0329 | 9895 |
| | WD repeat-containing protein 48 (USP1-associated factor 1) (WD repeat endosomal protein)(p80) | WDR48 | |
| | | KIAA1449 | |
| WDR48 HUMAN | | UAF1 | 57599 |
| MCPH1 HUMAN | Microcephalin | MCPH1 | 79648 |
| | Serine/threonine-protein phosphatase 2A regulatory subunit B" subunit gamma (Protein phosphatase subunit G5PR) (Rhabdomyosarcoma antigen MU-RMS-40.6A/6C) | PPP2R3C | |
| | | C14orf10 | |
| P2R3C_HUMAN | | G5PR | 55012 |
| | Beta-1 adrenergic receptor (Beta-1 adrenoreceptor)(Beta-1 adrenoceptor) | ADRB1 | |
| | | ADRB1R | |
| ADRB1_HUMAN | | B1AR | 153 |
| JAG2 HUMAN | Protein jagged-2 (Jagged2) (hJ2) | JAG2 | 3714 |
| GEMI7 HUMAN | Gem-associated protein 7 (Gemin-7) (SIP3) | GEMIN7 | 79760 |
| | Receptor-type tyrosine-protein phosphatase beta (Protein-tyrosine phosphatase beta) (R-PTP-beta) (EC 3.1.3.48) (Vascular endothelial protein tyrosine phosphatase) (VE-PTP) | PTPRB | |
| PTPRB HUMAN | | PTPB | 5787 |
| | Protein arginine N-methyltransferase 9 (Protein arginine N-methyltransferase 10) (EC 2.1.1.320) | PRMT9 | |
| ANM9 HUMAN | | PRMT10 | 90826 |
| | F-box only protein 11 (Protein arginine N-methyltransferase 9) (Vitiligo-associated protein 1) (VIT-1) | FBXO11 | |
| | | FBX11 | |
| | | PRMT9 | |
| | | VIT1 | |
| FBX11 HUMAN | | UG063H01 | 80204 |
| | Proteasome activator complex subunit 4 (Proteasome activator PA200) | PSME4 | |
| PSME4 HUMAN | | KIAA0077 | 23198 |
| | Ubiquitin-conjugating enzyme E2 L3 (EC 2.3.2.23) (E2 ubiquitin-conjugating enzyme L3) (L-UBC) (UbcH7) (Ubiquitin carrier protein L3) (Ubiquitin-conjugating enzyme E2-F1) (Ubiquitin-protein ligase L3) | UBE2L3 | |
| | | UBCE7 | |
| UB2L3 HUMAN | | UBCH7 | 7332 |
| | EKC/KEOPS complex subunit TP53RK (EC 3.6.-.-) (Atypical serine/threonine protein kinase TP53RK) (Nori-2) (TP53-regulating kinase) (EC 2.7.11.1) (p53-related protein kinase) | TP53RK | |
| | | C20orf64 | |
| PRPK HUMAN | | PRPK | 112858 |
| PSME3 HUMAN | Proteasome activator complex subunit 3 (11S regulator complex subunit gamma) (REG-gamma) (Activator of multicatalytic protease subunit 3) (Ki nuclear autoantigen) (Proteasome activator 28 subunit gamma) (PA28g) (PA28gamma) | PSME3 | 10197 |

As used herein, the term "subject" refers to a mammalian subject, typically a human.

As used herein, the term "lung cancer" refers to non-small cell lung cancer (NSCLC) and small cell lung cancer (SCLC).

According to a particular aspect, the cancer is NSCLC.

The term "non-small cell lung cancer" as used herein, refers to a group of lung cancers named for the kinds of cells found in the cancer and how the cells look microscopically. It is the most common type of lung cancer. The three main types of non-small cell lung cancer are squamous cell carcinoma, large cell carcinoma, and adenocarcinoma. The term "squamous cell carcinoma" as used herein refers to a non-small cell lung cancer that begins in squamous cells, which are thin, flat cells found in the tissue that forms the lining of the respiratory tract. Squamous cell carcinomas are found in the center of the lung next to a bronchus. The term "large cell carcinoma" refers to a lung cancer in which the cells are large and look abnormal when viewed microscopically. Large cell carcinomas can occur in any part of the lung, and tend to grow and spread faster than the other two types. The term "adenocarcinoma" as used herein refers to a cancer that begins in glandular (secretory) cells; adenocarcinomas are found in an outer area of the lung. The term "adenocarcinoma in situ" as used herein refers to a condition in which abnormal cells are found in the glandular tissue, which may become cancer and spread to nearby normal tissue.

The cancer may be at any stage of NSCLC (from Stage 0 to stage IV).

Altogether there are five stages (Stage 0 to Stage IV) in NSCLC. Stages I, II and III are further subdivided into A and B subtypes. These stages are assigned based on a Tumor, Node and Metastasis (TMN) staging system (See, cancer staging guidelines, www(dot)NCCN(dot)org). The TMN staging system of NSCLC is summarized in US Application No. 20160263187.

According to one aspect, the tumor is a primary tumor. According to another aspect, the tumor is a secondary tumor. According to another aspect, the tumor is a recurrent tumor. According to another aspect, the tumor is a tumor refractory to chemotherapy.

According to some aspects, the primary tumor is a squamous cell carcinoma, an adenocarcinoma, or a large cell carcinoma.

According to some aspects, the secondary tumor or site of metastasis is one or more of lung tissue, adrenal tissue, bone tissue, liver tissue or brain tissue.

According to one aspect, the downregulation of the polypeptide may, for example, be effective to reduce proliferation of the population of tumor cells, to reduce tumor size, to reduce tumor burden, to induce tumor cell death, or a combination thereof.

According to some aspects, cancer cell death may include, but is not limited to, apoptosis.

Downregulation of any of the proteins listed herein can be effected on the genomic and/or the transcript level using a variety of molecules which interfere with transcription and/or translation (e.g., RNA silencing agents, Ribozyme, DNAzyme and antisense, CRISPR system), or on the protein level using e.g., antagonists, antibodies, enzymes that cleave the polypeptide, enzyme inhibitors and the like.

Following is a list of agents capable of downregulating expression level and/or activity of the proteins disclosed herein.

One example, of an agent capable of downregulating one of the disclosed proteins is an inhibitory antibody (or antibody fragment) capable of specifically binding thereto. When the target is an intracellular target, preferably the antibody is capable of being internalized by the cell and entering the nucleus.

The term "antibody" as used in this disclosure includes intact molecules as well as functional fragments thereof, such as Fab, F(ab')2, and Fv that are capable of binding to macrophages. These functional antibody fragments are defined as follows: (1) Fab, the fragment which contains a monovalent antigen-binding fragment of an antibody molecule, can be produced by digestion of whole antibody with the enzyme papain to yield an intact light chain and a portion of one heavy chain; (2) Fab', the fragment of an antibody molecule that can be obtained by treating whole antibody with pepsin, followed by reduction, to yield an intact light chain and a portion of the heavy chain; two Fab' fragments are obtained per antibody molecule; (3) (Fab')2, the fragment of the antibody that can be obtained by treating whole antibody with the enzyme pepsin without subsequent reduction; F(ab')2 is a dimer of two Fab' fragments held together by two disulfide bonds; (4) Fv, defined as a genetically engineered fragment containing the variable region of the light chain and the variable region of the heavy chain expressed as two chains; and (5) Single chain antibody ("SCA"), a genetically engineered molecule containing the variable region of the light chain and the variable region of the heavy chain, linked by a suitable polypeptide linker as a genetically fused single chain molecule.

In one aspect, the antibody is a humanized antibody.

Humanized forms of non-human (e.g., murine) antibodies are chimeric molecules of immunoglobulins, immunoglobulin chains or fragments thereof (such as Fv, Fab, Fab', F(ab').sub.2 or other antigen-binding subsequences of antibodies) which contain minimal sequence derived from non-human immunoglobulin. Humanized antibodies include human immunoglobulins (recipient antibody) in which residues form a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues.

Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as import residues, which are typically taken from an import variable domain. Humanization can be essentially performed following the method of Winter and co-workers [Jones et al., Nature, 321:522-525 (1986); Riechmann et al., Nature 332:323-327 (1988); Verhoeyen et al., Science, 239:1534-1536 (1988)], by substituting rodent CDRs or CDR sequences for the corresponding sequences of a human antibody. Accordingly, such humanized antibodies are chimeric antibodies (U.S. Pat. No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)]. Similarly, human antibodies can be made by introduction of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Pat. Nos. 5,545,807; 5,545,806; 5,569,825; 5,625,126; 5,633,425; 5,661,016, and in the following scientific publications: Marks et al., Bio/Technology 10: 779-783 (1992); Lonberg et al., Nature 368: 856-859 (1994); Morrison, Nature 368 812-13 (1994); Fishwild et al., Nature Biotechnology 14, 845-51 (1996); Neuberger, Nature Biotechnology 14: 826 (1996); and Lonberg and Huszar, Intern. Rev. Immunol. 13, 65-93 (1995).

Downregulation of the proteins disclosed herein can be also achieved by RNA silencing. As used herein, the phrase "RNA silencing" refers to a group of regulatory mechanisms [e.g. RNA interference (RNAi), transcriptional gene silencing (TGS), post-transcriptional gene silencing (PTGS), quelling, co-suppression, and translational repression] mediated by RNA molecules which result in the inhibition or "silencing" of the expression of a corresponding protein-coding gene. RNA silencing has been observed in many types of organisms, including plants, animals, and fungi.

As used herein, the term "RNA silencing agent" refers to an RNA which is capable of inhibiting or "silencing" the expression of a target gene. In certain aspects, the RNA silencing agent is capable of preventing complete processing (e.g., the full translation and/or expression) of an mRNA molecule through a post-transcriptional silencing mechanism. RNA silencing agents include noncoding RNA molecules, for example RNA duplexes comprising paired strands, as well as precursor RNAs from which such small non-coding RNAs can be generated. Exemplary RNA silencing agents include dsRNAs such as siRNAs, miRNAs and shRNAs. In one aspect, the RNA silencing agent is capable of inducing RNA interference. In another aspect, the RNA silencing agent is capable of mediating translational repression.

RNA interference refers to the process of sequence-specific post-transcriptional gene silencing in animals mediated by short interfering RNAs (siRNAs). The corresponding process in plants is commonly referred to as post-transcriptional gene silencing or RNA silencing and is also referred to as quelling in fungi. The process of post-transcriptional gene silencing is thought to be an evolutionarily-conserved cellular defense mechanism used to prevent the expression of foreign genes and is commonly shared by diverse flora and phyla. Such protection from foreign gene expression may have evolved in response to the production of double-stranded RNAs (dsRNAs) derived from viral infection or from the random integration of transposon elements into a host genome via a cellular response that specifically destroys homologous single-stranded RNA or viral genomic RNA.

The presence of long dsRNAs in cells stimulates the activity of a ribonuclease III enzyme referred to as dicer. Dicer is involved in the processing of the dsRNA into short pieces of dsRNA known as short interfering RNAs (siRNAs). Short interfering RNAs derived from dicer activity are typically about 21 to about 23 nucleotides in length and comprise about 19 base pair duplexes. The RNAi response also features an endonuclease complex, commonly referred to as an RNA-induced silencing complex (RISC), which mediates cleavage of single-stranded RNA having sequence complementary to the antisense strand of the siRNA duplex. Cleavage of the target RNA takes place in the middle of the region complementary to the antisense strand of the siRNA duplex.

Accordingly, the present disclosure contemplates use of dsRNA to downregulate protein expression from mRNA.

According to one aspect, the dsRNA is greater than 30 bp. The use of long dsRNAs (i.e. dsRNA greater than 30 bp) has been very limited owing to the belief that these longer regions of double stranded RNA will result in the induction of the interferon and PKR response. However, the use of long dsRNAs can provide numerous advantages in that the cell can select the optimal silencing sequence alleviating the need to test numerous siRNAs; long dsRNAs will allow for silencing libraries to have less complexity than would be necessary for siRNAs; and, perhaps most importantly, long dsRNA could prevent viral escape mutations when used as therapeutics.

Various studies demonstrate that long dsRNAs can be used to silence gene expression without inducing the stress response or causing significant off-target effects - see for example [Strat et al., Nucleic Acids Research, 2006, Vol. 34, No. 13 3803-3810; Bhargava A et al. Brain Res. Protoc. 2004;13:115-125; Diallo M., et al., Oligonucleotides. 2003;13:381-392; Paddison P.J., et al., Proc. Natl Acad. Sci. USA. 2002;99:1443-1448; Tran N., et al., FEBS Lett. 2004;573:127-134].

In particular, the present disclosure also contemplates introduction of long dsRNA (over 30 base transcripts) for gene silencing in cells where the interferon pathway is not activated (e.g. embryonic cells and oocytes) see for example Billy et al., PNAS 2001, Vol 98, pages 14428-14433. and Diallo et al, Oligonucleotides, October 1, 2003, 13(5): 381-392. doi:10.1089/154545703322617069.

The present disclosure also contemplates introduction of long dsRNA specifically designed not to induce the interferon and PKR pathways for down-regulating gene expression. For example, Shinagwa and Ishii [Genes & Dev. 17 (11): 1340-1345, 2003] have developed a vector, named pDECAP, to express long double-strand RNA from an RNA polymerase II (Pol II) promoter. Because the transcripts from pDECAP lack both the 5'-cap structure and the 3'-poly(A) tail that facilitate ds-RNA export to the cytoplasm, long ds-RNA from pDECAP does not induce the interferon response.

Another method of evading the interferon and PKR pathways in mammalian systems is by introduction of small inhibitory RNAs (siRNAs) either via transfection or endogenous expression.

The term "siRNA" refers to small inhibitory RNA duplexes (generally between 18-30 basepairs) that induce the RNA interference (RNAi) pathway. Typically, siRNAs are chemically synthesized as 21mers with a central 19 bp duplex region and symmetric 2-base 3'-overhangs on the termini, although it has been recently described that chemically synthesized RNA duplexes of 25-30 base length can have as much as a 100-fold increase in potency compared with 21mers at the same location. The observed increased potency obtained using longer RNAs in triggering RNAi is theorized to result from providing Dicer with a substrate (27mer) instead of a product (21mer) and that this improves the rate or efficiency of entry of the siRNA duplex into RISC.

It has been found that position of the 3'-overhang influences potency of a siRNA and asymmetric duplexes having a 3'-overhang on the antisense strand are generally more potent than those with the 3'-overhang on the sense strand (Rose et al., 2005). This can be attributed to asymmetrical strand loading into RISC, as the opposite efficacy patterns are observed when targeting the antisense transcript.

The strands of a double-stranded interfering RNA (e.g., a siRNA) may be connected to form a hairpin or stem-loop structure (e.g., a shRNA). Thus, as mentioned the RNA silencing agent of the present disclosure may also be a short hairpin RNA (shRNA).

The term "shRNA", as used herein, refers to an RNA agent having a stem-loop structure, comprising a first and second region of complementary sequence, the degree of complementarity and orientation of the regions being sufficient such that base pairing occurs between the regions, the first and second regions being joined by a loop region, the loop resulting from a lack of base pairing between nucleotides (or nucleotide analogs) within the loop region. The number of nucleotides in the loop is a number between and including 3 to 23, or 5 to 15, or 7 to 13, or 4 to 9, or 9 to 11. Some of the nucleotides in the loop can be involved in base-pair interactions with other nucleotides in the loop. It will be recognized by one of skill in the art that the resulting single chain oligonucleotide forms a stem-loop or hairpin structure comprising a double-stranded region capable of interacting with the RNAi machinery.

According to another aspect the RNA silencing agent may be a miRNA. miRNAs are small RNAs made from genes encoding primary transcripts of various sizes. They have been identified in both animals and plants. The primary transcript (termed the "pri-miRNA") is processed through various nucleolytic steps to a shorter precursor miRNA, or "pre-miRNA." The pre-miRNA is present in a folded form so that the final (mature) miRNA is present in a duplex, the two strands being referred to as the miRNA (the strand that will eventually basepair with the target) The pre-miRNA is a substrate for a form of dicer that removes the miRNA duplex from the precursor, after which, similarly to siRNAs, the duplex can be taken into the RISC complex. It has been demonstrated that miRNAs can be transgenically expressed and be effective through expression of a precursor form, rather than the entire primary form (Parizotto et al. (2004) Genes & Development 18:2237-2242 and Guo et al. (2005) Plant Cell 17:1376-1386).

Unlike, siRNAs, miRNAs bind to transcript sequences with only partial complementarity (Zeng et al., 2002, Molec. Cell 9:1327-1333) and repress translation without affecting steady-state RNA levels (Lee et al., 1993, Cell 75:843-854; Wightman et al., 1993, Cell 75:855-862). Both miRNAs and siRNAs are processed by Dicer and associate with components of the RNA-induced silencing complex (Hutvagner et al., 2001, Science 293:834-838; Grishok et al., 2001, Cell 106: 23-34; Ketting et al., 2001, Genes Dev. 15:2654-2659; Williams et al., 2002, Proc. Natl. Acad. Sci. USA 99:6889-6894; Hammond et al., 2001, Science 293:1146-1150; Mourlatos et al., 2002, Genes Dev. 16:720-728). A recent report (Hutvagner et al., 2002, Sciencexpress 297:2056-2060) hypothesizes that gene regulation through the miRNA pathway versus the siRNA pathway is determined solely by the degree of complementarity to the target transcript. It is speculated that siRNAs with only partial identity to the mRNA target will function in translational repression, similar to a miRNA, rather than triggering RNA degradation.

It will be appreciated that the RNA silencing agent of the present disclosure need not be limited to those molecules containing only RNA, but further encompasses chemically-modified nucleotides and non-nucleotides.

In some aspects, the RNA silencing agent provided herein can be functionally associated with a cell-penetrating peptide." As used herein, a "cell-penetrating peptide" is a peptide that comprises a short (about 12-30 residues) amino acid sequence or functional motif that confers the energy-independent (i.e., non-endocytotic) translocation properties associated with transport of the membrane-permeable complex across the plasma and/or nuclear membranes of a cell. The cell-penetrating peptide used in the membrane-permeable complex of the present disclosure preferably comprises at least one non-functional cysteine residue, which is either free or derivatized to form a disulfide link with a double-stranded ribonucleic acid that has been modified for such linkage. Representative amino acid motifs conferring such properties are listed in U.S. Pat. No. 6,348,185. The cell-penetrating peptides of the present disclosure preferably include, but are not limited to, penetratin, transportan, pIsl, TAT(48-60), pVEC, MTS, and MAP.

Another agent capable of downregulating the proteins disclosed herein is a DNAzyme molecule capable of specifically cleaving an mRNA transcript or DNA sequence thereof. DNAzymes are single-stranded polynucleotides which are capable of cleaving both single and double stranded target sequences (Breaker, R.R. and Joyce, G. Chemistry and Biology 1995;2:655; Santoro, S.W. & Joyce, G.F. Proc. Natl, Acad. Sci. USA 1997;943:4262) A general model (the "10-23" model) for the DNAzyme has been proposed. "10-23" DNAzymes have a catalytic domain of 15 deoxyribonucleotides, flanked by two substrate-recognition domains of seven to nine deoxyribonucleotides each. This type of DNAzyme can effectively cleave its substrate RNA at purine: pyrimidine junctions (Santoro, S.W. & Joyce, G.F. Proc. Natl, Acad. Sci. USA 199; for rev of DNAzymes see Khachigian, LM [Curr Opin Mol Ther 4:119-21 (2002)].

Examples of construction and amplification of synthetic, engineered DNAzymes recognizing single and double-stranded target cleavage sites have been disclosed in U.S. Pat. No. 6,326,174 to Joyce et al. DNAzymes of similar design directed against the human Urokinase receptor were recently observed to inhibit Urokinase receptor expression, and successfully inhibit colon cancer cell metastasis (Itoh et al, 20002, Abstract 409, Ann Meeting Am Soc Gen Ther www(dot)asgt(dot)org). In another application, DNAzymes complementary to bcr-ab1 oncogenes were successful in inhibiting the oncogenes expression in leukemia cells, and lessening relapse rates in autologous bone marrow transplant in cases of CML and ALL.

Downregulation of the proteins disclosed herein can also be effected by using an antisense polynucleotide capable of specifically hybridizing with an mRNA transcript encoding the protein.

Design of antisense molecules which can be used to efficiently downregulate the protein must be effected while considering two aspects important to the antisense approach. The first aspect is delivery of the oligonucleotide into the cytoplasm of the appropriate cells, while the second aspect is design of an oligonucleotide which specifically binds the designated mRNA within cells in a way which inhibits translation thereof.

The prior art teaches of a number of delivery strategies which can be used to efficiently deliver oligonucleotides into a wide variety of cell types [see, for example, Luft J Mol Med 76: 75-6 (1998); Kronenwett et al. Blood 91: 852-62 (1998); Rajur et al. Bioconjug Chem 8: 935-40 (1997); Lavigne et al. Biochem Biophys Res Commun 237: 566-71 (1997) and Aoki et al. (1997) Biochem Biophys Res Commun 231: 540-5 (1997)].

In addition, algorithms for identifying those sequences with the highest predicted binding affinity for their target mRNA based on a thermodynamic cycle that accounts for the energetics of structural alterations in both the target mRNA and the oligonucleotide are also available [see, for example, Walton et al. Biotechnol Bioeng 65: 1-9 (1999)].

Such algorithms have been successfully used to implement an antisense approach in cells. For example, the algorithm developed by Walton et al. enabled scientists to successfully design antisense oligonucleotides for rabbit beta-globin (RBG) and mouse tumor necrosis factor-alpha (TNF alpha) transcripts. The same research group has more recently reported that the antisense activity of rationally selected oligonucleotides against three model target mRNAs (human lactate dehydrogenase A and B and rat gp130) in cell culture as evaluated by a kinetic PCR technique proved effective in almost all cases, including tests against three different targets in two cell types with phosphodiester and phosphorothioate oligonucleotide chemistries.

In addition, several approaches for designing and predicting efficiency of specific oligonucleotides using an in vitro system were also published (Matveeva et al., Nature Biotechnology 16: 1374 - 1375 (1998)].

Another agent capable of downregulating the disclosed proteins is a ribozyme molecule capable of specifically cleaving an mRNA transcript encoding the disclosed protein. Ribozymes are being increasingly used for the sequence-specific inhibition of gene expression by the cleavage of mRNAs encoding proteins of interest [Welch et al., Curr Opin Biotechnol. 9:486-96 (1998)]. The possibility of designing ribozymes to cleave any specific target RNA has rendered them valuable tools in both basic research and therapeutic applications. In the therapeutics area, ribozymes have been exploited to target viral RNAs in infectious diseases, dominant oncogenes in cancers and specific somatic mutations in genetic disorders [Welch et al., Clin Diagn Virol. 10:163-71 (1998)]. Most notably, several ribozyme gene therapy protocols for HIV patients are already in Phase 1 trials. More recently, ribozymes have been used for transgenic animal research, gene target validation and pathway elucidation. Several ribozymes are in various stages of clinical trials. ANGIOZYME was the first chemically synthesized ribozyme to be studied in human clinical trials. ANGIOZYME specifically inhibits formation of the VEGF-r (Vascular Endothelial Growth Factor receptor), a key component in the angiogenesis pathway. Ribozyme Pharmaceuticals, Inc., as well as other firms have demonstrated the importance of anti-angiogenesis therapeutics in animal models. HEPTAZYME, a ribozyme designed to selectively destroy Hepatitis C Virus (HCV) RNA, was found effective in decreasing Hepatitis C viral RNA in cell culture assays (Ribozyme Pharmaceuticals, Incorporated - WEB home page).

An additional method of regulating the expression of one of the disclosed proteins in cells is via triplex forming oligonuclotides (TFOs). Recent studies have shown that TFOs can be designed which can recognize and bind to polypurine/polypirimidine regions in double-stranded helical DNA in a sequence-specific manner. These recognition rules are outlined by Maher III, L. J., et al., Science, 1989;245:725-730; Moser, H. E., et al., Science,1987;238:645-630; Beal, P. A., et al, Science,1992;251:1360-1363; Cooney, M., et al., Science, 1988;241:456-459; and Hogan, M. E., et al., EP Publication 375408. Modification of the oligonuclotides, such as the introduction of intercalators and backbone substitutions, and optimization of binding conditions (pH and cation concentration) have aided in overcoming inherent obstacles to TFO activity such as charge repulsion and instability, and it was recently shown that synthetic oligonucleotides can be targeted to specific sequences (for a recent review see Seidman and Glazer, J Clin Invest 2003; 112:487-94).

In general, the triplex-forming oligonucleotide has the sequence correspondence:

| | | | | |
|---|---|---|---|---|
| oligo | 3'--A | G | G | T |
| duplex | 5'--A | G | C | T |
| duplex | 3'--T | C | G | A |

However, it has been shown that the A-AT and G-GC triplets have the greatest triple helical stability (Reither and Jeltsch, BMC Biochem, 2002, Sept12, Epub). The same authors have demonstrated that TFOs designed according to the A-AT and G-GC rule do not form non-specific triplexes, indicating that the triplex formation is indeed sequence specific.

Thus for any given sequence of the regulatory region of one of the disclosed proteins, a triplex forming sequence may be devised. Triplex-forming oligonucleotides preferably are at least 15, more preferably 25, still more preferably 30 or more nucleotides in length, up to 50 or 100 bp.

Transfection of cells (for example, via cationic liposomes) with TFOs, and formation of the triple helical structure with the target DNA induces steric and functional changes, blocking transcription initiation and elongation, allowing the introduction of desired sequence changes in the endogenous DNA and resulting in the specific downregulation of gene expression. Examples of such suppression of gene expression in cells treated with TFOs include knockout of episomal supFG1 and endogenous HPRT genes in mammalian cells (Vasquez et al., Nucl Acids Res. 1999;27:1176-81, and Puri, et al, J Biol Chem, 2001;276:28991-98), and the sequence- and target specific downregulation of expression of the Ets2 transcription factor, important in prostate cancer etiology (Carbone, et al, Nucl Acid Res. 2003;31:833-43), and the pro-inflammatory ICAM-1 gene (Besch et al, J Biol Chem, 2002;277:32473-79). In addition, Vuyisich and Beal have recently shown that sequence specific TFOs can bind to dsRNA, inhibiting activity of dsRNA-dependent enzymes such as RNA-dependent kinases (Vuyisich and Beal, Nuc. Acids Res 2000;28:2369-74).

Additionally, TFOs designed according to the abovementioned principles can induce directed mutagenesis capable of effecting DNA repair, thus providing both downregulation and upregulation of expression of endogenous genes (Seidman and Glazer, J Clin Invest 2003; 112:487-94). Detailed description of the design, synthesis and administration of effective TFOs can be found in U.S. Patent Application Nos. 2003 017068 and 2003 0096980 to Froehler et al, and 2002 0128218 and 2002 0123476 to Emanuele et al, and U.S. Pat. No. 5,721,138 to Lawn.

Downregulation of the disclosed proteins can be effected using a meganuclease system such as the CRISPR-Cas system (as further described herein below).

*CRISPR-Cas system* - Many bacteria and archea contain endogenous RNA-based adaptive immune systems that can degrade nucleic acids of invading phages and plasmids. These systems consist of clustered regularly interspaced short palindromic repeat (CRISPR) genes that produce RNA components and CRISPR associated (Cas) genes that encode protein components. The CRISPR RNAs (crRNAs) contain short stretches of homology to specific viruses and plasmids and act as guides to direct Cas nucleases to degrade the complementary nucleic acids of the corresponding pathogen. Studies of the type II CRISPR/Cas system of *Streptococcus pyogenes* have shown that three components form an RNA/protein complex and together are sufficient for sequence-specific nuclease activity: the Cas9 nuclease, a crRNA containing 20 base pairs of homology to the target sequence, and a trans-activating crRNA (tracrRNA) (Jinek et al. Science (2012) 337: 816-821.). It was further demonstrated that a synthetic chimeric guide RNA (gRNA) composed of a fusion between crRNA and tracrRNA could direct Cas9 to cleave DNA targets that are complementary to the crRNA in vitro. It was also demonstrated that transient expression of Cas9 in conjunction with synthetic gRNAs can be used to produce targeted double-stranded brakes in a variety of different species (Cho *et al.,* 2013; Cong *et al.,* 2013; DiCarlo *et al.,* 2013; Hwang *et al.,* 2013a,b; Jinek *et al.,* 2013; Mali *et al.,* 2013).

The CRIPSR/Cas system for genome editing contains two distinct components: a gRNA and an endonuclease e.g. Cas9.

The gRNA is typically a 20 nucleotide sequence encoding a combination of the target homologous sequence (crRNA) and the endogenous bacterial RNA that links the crRNA to the Cas9 nuclease (tracrRNA) in a single chimeric transcript. The gRNA/Cas9 complex is recruited to the target sequence by the base-pairing between the gRNA sequence and the complement genomic DNA. For successful binding of Cas9, the genomic target sequence must also contain the correct Protospacer Adjacent Motif (PAM) sequence immediately following the target sequence. The binding of the gRNA/Cas9 complex localizes the Cas9 to the genomic target sequence so that the Cas9 can cut both strands of the DNA causing a double-strand break. Just as with ZFNs and TALENs, the double-stranded brakes produced by CRISPR/Cas can undergo homologous recombination or NHEJ.

The Cas9 nuclease has two functional domains: RuvC and HNH, each cutting a different DNA strand. When both of these domains are active, the Cas9 causes double strand breaks in the genomic DNA.

A significant advantage of CRISPR/Cas is that the high efficiency of this system coupled with the ability to easily create synthetic gRNAs enables multiple genes to be targeted simultaneously. In addition, the majority of cells carrying the mutation present biallelic mutations in the targeted genes.

However, apparent flexibility in the base-pairing interactions between the gRNA sequence and the genomic DNA target sequence allows imperfect matches to the target sequence to be cut by Cas9.

Modified versions of the Cas9 enzyme containing a single inactive catalytic domain, either RuvC- or HNH-, are called 'nickases'. With only one active nuclease domain, the Cas9 nickase cuts only one strand of the target DNA, creating a single-strand break or 'nick'. A single-strand break, or nick, is normally quickly repaired through the HDR pathway, using the intact complementary DNA strand as the template. However, two proximal, opposite strand nicks introduced by a Cas9 nickase are treated as a double-strand break, in what is often referred to as a 'double nick' CRISPR system. A double-nick can be repaired by either NHEJ or HDR depending on the desired effect on the gene target. Thus, if specificity and reduced off-target effects are crucial, using the Cas9 nickase to create a double-nick by designing two gRNAs with target sequences in close proximity and on opposite strands of the genomic DNA would decrease off-target effect as either gRNA alone will result in nicks that will not change the genomic DNA.

Modified versions of the Cas9 enzyme containing two inactive catalytic domains (dead Cas9, or dCas9) have no nuclease activity while still able to bind to DNA based on gRNA specificity. The dCas9 can be utilized as a platform for DNA transcriptional regulators to activate or repress gene expression by fusing the inactive enzyme to known regulatory domains. For example, the binding of dCas9 alone to a target sequence in genomic DNA can interfere with gene transcription.

There are a number of publically available tools available to help choose and/or design target sequences as well as lists of bioinformatically determined unique gRNAs for different genes in different species such as the Feng Zhang lab's Target Finder, the Michael Boutros lab's Target Finder (E-CRISP), the RGEN Tools: Cas-OFFinder, the CasFinder: Flexible algorithm for identifying specific Cas9 targets in genomes and the CRISPR Optimal Target Finder.

In order to use the CRISPR system, both gRNA and Cas9 should be expressed in a target cell. The insertion vector can contain both cassettes on a single plasmid or the cassettes are expressed from two separate plasmids. CRISPR plasmids are commercially available such as the px330 plasmid from Addgene.

Polynucleotide agents for down-regulating an amount or activity of one of the disclosed proteins are typically administered as part of an expression construct. In this case, the polynucleotide agent is ligated in a nucleic acid construct under the control of a cis-acting regulatory element (e.g. promoter) capable of directing an expression of the agent capable of downregulating one of the disclosed proteins in a constitutive or inducible manner.

The nucleic acid agent may be delivered using an appropriate gene delivery vehicle/method (transfection, transduction, etc.). Optionally an appropriate expression system is used. Examples of suitable constructs include, but are not limited to, pcDNA3, pcDNA3.1 (+/-), pGL3, PzeoSV2 (+/-), pDisplay, pEF/myc/cyto, pCMV/myc/cyto each of which is commercially available from Invitrogen Co. (www(dot)Invitrogen(dot)com).

The expression construct may also be a virus. Examples of viral constructs include but are not limited to adenoviral vectors, retroviral vectors, vaccinia viral vectors, adeno-associated viral vectors, polyoma viral vectors, alphaviral vectors, rhabdoviral vectors, lenti viral vectors and herpesviral vectors.

A viral construct such as a retroviral construct includes at least one transcriptional promoter/enhancer or locus-defining element(s), or other elements that control gene expression by other means such as alternate splicing, nuclear RNA export, or post-transcriptional modification of messenger. Such vector constructs also include a packaging signal, long terminal repeats (LTRs) or portions thereof, and positive and negative strand primer binding sites appropriate to the virus used, unless it is already present in the viral construct. In addition, such a construct typically includes a signal sequence for secretion of the peptide from a host cell in which it is placed. Preferably, the signal sequence for this purpose is a mammalian signal sequence or the signal sequence of the peptide variants of the present disclosure. Optionally, the construct may also include a signal that directs polyadenylation, as well as one or more restriction site and a translation termination sequence. By way of example, such constructs will typically include a 5' LTR, a tRNA binding site, a packaging signal, an origin of second-strand DNA synthesis, and a 3' LTR or a portion thereof.

Preferably the viral dose for infection is at least 10³, 10⁴, 10⁵, 10⁶, 10⁷, 10⁸, 10⁹, 10¹⁰, 10¹¹, 10¹², 10¹³, 10¹⁴, 10¹⁵ or higher pfu or viral particles.

Double stranded RNA may be synthesized by adding two opposing promoters to the ends of the gene segments, wherein one promoter is placed immediately 5' to the gene and the opposing promoter is placed immediately 3' to the gene segment. The dsRNA may then be transcribed with the appropriate polymerase.

The application of small polynucleotide agents (e.g. siRNAs) as potential therapeutic agents requires delivery approaches that will enhance their pharmacological properties. These delivery approaches aim to: (1) increase the retention time of the small polynucleotide agents in the circulatory system by reducing the rate of renal clearance; (2) protect the small polynucleotide agents from serum nucleases; (3) ensure effective biodistribution; (4) facilitate targeting to and uptake of the small polynucleotide agents into the target cells; and (5) promote trafficking to the cytoplasm and uptake into RISC. A variety of approaches have been developed that promote small polynucleotide agent delivery in vivo, including cationic nanoparticles, lipids and liposomes, antibody (Ab)-fusion molecules [Ab-protamine and Ab-poly-arginine, as well as cholesterol and aptamer-conjugated agents. On their own, small polynucleotide agents such as siRNAs fall below the size threshold for renal filtration and are rapidly cleared from the circulatory system. Complexes of small polynucleotide agents and the various delivery reagents remain in the circulation for longer, either because they exceed the size cut-off for renal clearance or because the delivery agents promote association with serum proteins (e.g. serum albumin).

In addition, the encapsidation of the small polynucleotide agents into nanoparticles (using either lipid- or cationic-polymer-based systems) helps to shield them from serum nucleases. Ab-fusion molecules have been used to effectively deliver naked, unmodified small polynucleotide agents to specific cell types following intravenous injection. Although the siRNAs are thought to be exposed on the surface of these recombinant Ab-fusion molecules, they were effectively delivered to the target cells, suggesting that complexation with these molecules provides some protection from nucleolytic degradation. The incorporation of chemical modifications to the phosphate backbone, the sugar moiety and the nucleoside bases of the small polynucleotide agents increases its resistance to degradation by serum nucleases. As some of these modifications are detrimental to the silencing efficacy, however, a balance must be maintained between the incorporation of chemical modifications and the inhibitory activity of the small polynucleotide agents. An attractive strategy for decreasing the dosage of the small polynucleotide agents needed to achieve effective silencing and minimizing off-target silencing in bystander cells is the use of delivery agents that target the small polynucleotide agents to specific cell types and tissues. This has been achieved using Abs or ligands that are fused to highly positively charged peptides or proteins, with which the small polynucleotide agents can associate by electrostatic interactions, or by directly conjugating aptamers or ligands to the small polynucleotide agents. These reagents (Abs, ligands and aptamers) can bind with high affinity to cell-surface molecules and deliver the small polynucleotide agents specifically to cells expressing these markers. By combining these targeting reagents with nanoparticles (e.g. immunoliposomes containing lipid nanoparticles coated with specific Abs), the quantity of small polynucleotide agents delivered and, as a consequence, the efficacy of silencing can be increased.

Accordingly, the present disclosure contemplates use of lipid-based systems for the delivery of these agents. Useful lipids for lipid-mediated transfer of the gene are, for example, DOTMA, DOPE, and DC-Chol [Tonkinson et al., Cancer Investigation, 14(1): 54-65 (1996)]. Recently, it has been shown that Chitosan can be used to deliver nucleic acids to the intestine cells (Chen J. (2004) World J Gastroenterol 10(1):112-116). Other non-lipid based vectors that can be used according to this aspect of the present disclosure include but are not limited to polylysine and dendrimers, carbon nanotubes, nanogels, polymer based particles.

As mentioned, when the target protein is an enzyme (e.g. PSME4, Ube2L3, TP53K or PSME3), the present disclosure contemplates the use of enzyme inhibitors to down-regulation the activity thereof.

The present inventors also contemplate the use of vaccines which target one of the disclosed proteins. Indirectly, it is perceived that vaccines may be capable of down-regulating the amount of the above disclosed proteins, in particular, but not limited to TECPR2, CASC5, WDR48, MCPH1, PPP2R3C, JAG2, GEMIN7, PTPRB and PRMT9.

As used herein, the term "vaccine" refers to a pharmaceutical preparation (pharmaceutical composition) or product that upon administration induces an immune response, in particular a cellular immune response, which recognizes and attacks a pathogen or a diseased cell such as a cancer cell.

A vaccine may be used for the prevention or treatment of a disease such as cancer (e.g. lung cancer). The term "personalized cancer vaccine" or "individualized cancer vaccine" concerns a particular cancer patient and means that a cancer vaccine is adapted to the needs or special circumstances of an individual cancer patient.

In one aspect, the vaccine comprises a peptide of one of the above disclosed polypeptides or a nucleic acid, preferably RNA, encoding the peptide or polypeptide.

The cancer vaccines provided according to the disclosure when administered to a patient provide one or more T cell epitopes suitable for stimulating, priming and/or expanding T cells specific for the patient's tumor. The T cells are preferably directed against cells expressing antigens from which the T cell epitopes are derived. Thus, the vaccines described herein are preferably capable of inducing or promoting a cellular response, preferably cytotoxic T cell activity, against a cancer disease characterized by presentation of one or more peptides of one of the above disclosed proteins with class I MHC.

The vaccine can comprise one or more T cell epitopes derived from the above disclosed proteins, such as 2 or more, 5 or more, 10 or more, 15 or more, 20 or more, 25 or more, 30 or more and preferably up to 60, up to 55, up to 50, up to 45, up to 40, up to 35 or up to 30 T cell epitopes.

Presentation of these epitopes by cells of a patient, in particular antigen presenting cells, preferably results in T cells targeting the epitopes when bound to MHC and thus, the patient's tumor, preferably the primary tumor as well as tumor metastases, expressing antigens from which the T cell epitopes are derived and presenting the same epitopes on the surface of the tumor cells.

The vaccines of the present disclosure may further comprise an adjuvant.

The term "adjuvant" as used herein refers to an agent that nonspecifically increases an immune response to a particular antigen thereby reducing the quantity of antigen necessary in any given vaccine and/or the frequency of injection necessary in order to generate an adequate immune response to the antigen of interest. Suitable adjuvants for use herein include, but are not limited to, poly IC; synthetic oligodeoxynucleotides (ODNs) with a CpG motif; modified polyinosinic:polycytidylic acid (Poly-IC) including, but not limited to, Poly-IC/LC (Hiltonol) and Poly-IC12U (Ampligen); Poly-K; carboxymethyl cellulose (CMC); Adjuvant 65 (containing peanut oil, mannide monooleate, an aluminum monostearate); Freund's complete or incomplete adjuvant; mineral gels such as aluminum hydroxide, aluminum phosphate, and alum; surfactants such as hexadecylamine, octadecylamine, lysolecithin, dimethyldioctadecylammonium bromide, N,N-dioctadecyl-N',N"-bis(2-hydroxymethyl)propanediamine, methoxyhexadecylglyerol and pluronic polyols; polyanions such as pyran, dextran sulfate, polyacrylic acid, and carbopol; peptides such as muramyl dipeptide, dimethylglycine and tuftsin; and oil emulsions. The adjuvants of the present disclosure may include nucleic acids based on inosine and cytosine such as poly I:poly C; poly IC; poly dC; poly dI; poly dIC; Poly-IC/LC; Poly-K; and Poly-IC12U as well as oligodeoxynucleotides (ODNs) with a CpG motif, CMC and any other combinations of complementary double stranded IC sequences or chemically modified nucleic acids such as thiolated poly IC as described in U.S. Pat. Nos. 6,008,334; 3,679,654 and 3,725,545.

The present inventors further contemplate use of T cell populations that are capable of binding to peptide epitopes of any of the above described proteins (in particular TECPR2, CASC5, WDR48, MCPH1, PPP2R3C, JAG2, GEMIN7, PTPRB, PRMT9) for adoptive cell therapy (ACT) - e.g. CAR-T cell therapy.

ACT refers to the transfer of cells, most commonly immune-derived cells, back into the same patient or into a new recipient host with the goal of transferring the immunologic functionality and characteristics into the new host. If possible, use of autologous cells helps the recipient by minimizing GVHD issues. The adoptive transfer of autologous tumor infiltrating lymphocytes (TIL) (Besser et al., (2010) Clin. Cancer Res 16 (9) 2646-55; Dudley et al., (2002) Science 298 (5594): 850-4; and Dudley et al., (2005) Journal of Clinical Oncology 23 (10): 2346-57.) or genetically re-directed peripheral blood mononuclear cells (Johnson et al., (2009) Blood 114 (3): 535-46; and Morgan et al., (2006) Science 314(5796) 126-9) has been used to successfully treat patients with advanced solid tumors, including melanoma and colorectal carcinoma, as well as patients with CD19-expressing hematologic malignancies (Kalos et al., (2011) Science Translational Medicine 3 (95): 95ra73).

In one aspect TCRs are selected for administering to a subject based on binding to neoantigens as identified herein. In one aspect T cells are expanded using methods known in the art. Expanded T cells that express tumor specific TCRs may be administered back to a subject. In another aspect PBMCs are transduced or transfected with polynucleotides for expression of TCRs and administered to a subject. T cells expressing TCRs specific to peptides derived from any or the above described proteins are expanded and administered back to a subject. In one aspect T cells that express TCRs for peptides derived any of the above disclosed proteins, that result in cytolytic activity when incubated with autologous tumor tissue are expanded and administered to a subject.

According to a particular aspect, the T cells express chimeric antigen receptors that target peptides derived from any of the above disclosed proteins (CAR-T cells).

The T cells can be administered by any suitable route as known in the art. Preferably, the T cells are administered as an intra-arterial or intravenous infusion, which preferably lasts approximately 30-60 min. Other examples of routes of administration include intraperitoneal, intrathecal and intralymphatic. T cells may also be administered by injection. T cells may be introduced at the site of the tumor.

For purposes of the disclosure, the dose, e.g., number of cells in the inventive cell population expressing subject specific TCRs, administered should be sufficient to effect, e.g., a therapeutic or prophylactic response, in the subject over a reasonable time frame. For example, the number of cells should be sufficient to bind to a cancer antigen, or detect, treat or prevent cancer in a period of from about 2 hours or longer, e.g., 12 to 24 or more hours, from the time of administration. In certain aspects, the time period could be even longer. The number of cells will be determined by, e.g., the efficacy of the particular cells and the condition of the subject (e.g., human), as well as the body weight of the subject (e.g., human) to be treated.

Many assays for determining an administered number of cells from the inventive cell population expressing subject specific TCRs are known in the art. For purposes of the disclosure, an assay, which comprises comparing the extent to which target cells are lysed or one or more cytokines such as, e.g., IFN-.gamma and IL-2 are secreted upon administration of a given number of such cells to a subject, could be used to determine a starting number to be administered to a mammal. The extent to which target cells are lysed, or cytokines such as, e.g., IFN-gamma and IL-2 are secreted, upon administration of a certain number of cells, can be assayed by methods known in the art. Secretion of cytokines such as, e.g., IL-2, may also provide an indication of the quality (e.g., phenotype and/or effectiveness) of a cell preparation.

The number of the cells administered from the inventive cell population expressing subject specific TCRs may also be determined by the existence, nature and extent of any adverse side effects that might accompany the administration of a particular cell population.

Another contemplated agent which can be used to downregulate any of the above described proteins includes a proteolysis-targeting chimaera (PROTAC). Such agents are heterobifunctional, comprising a ligand which binds to an ubiquitin ligase (such as E3 ubiquitin ligase) and a ligand to one of the above described proteins (e.g. PSME4) and optionally a linker connecting the two ligands. Binding of the PROTAC to the target protein leads to the ubiquitination of an exposed lysine on the target protein, followed by ubiquitin proteasome system (UPS)-mediated protein degradation.

According to another aspect of the present disclosure there is provided a method of targeting a pharmaceutical agent to a lung cancer cell in a subject comprising administering the pharmaceutical agent to the subject, wherein the pharmaceutical agent is attached to an affinity moiety, the affinity moiety being capable of binding specifically to a polypeptide selected from the group consisting of MYOF, CTNS, FCGR2B, PCDHGC5, POMGNT2, ACSL1, CTAGE5 and ADRB1, thereby targeting the pharmaceutical agent to the lung cancer cell.

In one aspect, the targeting is effected *in vivo.*

In another aspect, the targeting is effected *in vitro.*

Agents which may be targeted to the lung cancer cells include but are not limited to therapeutic agents and diagnostic agents.

Exemplary therapeutic agents include nucleic acid, polypeptides e.g. antibodies, anticancer agent (e.g., chemotherapy, radioisotopes, immunotherapy), antibiotic, enzyme, antioxidant, lipid intake inhibitor, hormone, anti-inflammatory, steroid, vasodilator, angiotensin converting enzyme inhibitor, angiotensin receptor antagonist, inhibitor for smooth muscle cell growth and migration, platelet aggregation inhibitor, anticoagulant, inhibitor for release of chemical mediator, promoter or inhibitor for endothelial cell growth, aldose reductase inhibitor, inhibitor for mesangium cell growth, lipoxygenase inhibitor, immunosuppressive, immunostimulant, antiviral agent, Maillard reaction suppressor, amyloidosis inhibitor, nitric oxide synthetic inhibitor, AGEs (Advanced glycation end-products) inhibitor, radical scavenger, protein, peptide; glycosaminoglycan and derivatives thereof; and oligosaccharide, polysaccharide, and derivatives thereof.

According to a particular aspect, the pharmaceutical agent is a cytotoxic agent.

As used herein, the term "cytotoxic agent" refers to refers to a substance that inhibits or prevents the function of cells and/or causes destruction of cells. The term is intended to include radioactive isotopes (e.g., ²¹¹At, ¹³¹I, ¹²⁵I, ³²P, ³⁵S and radioactive isotopes of Lu, including ¹⁷⁷Lu, ⁸⁶Y, ⁹⁰Y, ¹¹¹In, ¹⁷⁷Lu, ²²⁵Ac, ²¹²Bi, ²¹³Bi, ⁶⁶Ga, ⁶⁷Ga, ⁶⁸Ga , ⁶⁴Cu, ⁶⁷Cu, ⁷¹As, ⁷²As, ⁷⁶As, ⁷⁷As, ⁶⁵Zn, ⁴⁸V, ²⁰³Pb, ²⁰⁹Pb , ²¹²Pb, ¹⁶⁶Ho, ¹⁴⁹Pm, ¹⁵³Sm, ²⁰¹Tl, ¹⁸⁸Re, ¹⁸⁶Re and ⁹⁹mTc), anticancer agents as otherwise described herein, including chemotherapeutic (anticancer drugs e.g. methotrexate, adriamicin, vinca alkaloids (vincristine, vinblastine, etoposide), taxol, doxoruicin, cisplatin, 5-fluorouridine, melphalan, ethidium bromide, mitomycin C, chlorambucil, daunorubicin and other intercalating agents, enzymes and fragments thereof such as nucleolytic enzymes, antibiotics, therapeutic RNA molecules (e.g., siRNA, antisense oligonucleotides, microRNA, ribozymes, RNA decoys, aptamers), DNAzymes, antibodies, proteins and polynucleotides encoding same, and toxins such as small molecule toxins or enzymatically active toxins of bacterial, fungal, plant or animal origin, such as pokeweed antiviral protein (PAP), ricin toxin A, abrin, gelonin, saporin, cholera toxin A, diphtheria toxin, Pseudomonas exotoxin, and alpha-sarcin, including fragments and/or variants thereof.

According to another aspect, the pharmaceutical agent is a diagnostic agent.

Exemplary diagnostic drugs include in vivo diagnostics such as an X ray contrast medium, a diagnostic agent for ultrasound, an isotope-labeled agent for diagnosis by nuclear medicine, and an agent for diagnosis by nuclear magnetic resonance.

As mentioned, the pharmaceutical agents of this aspect of the present disclosure are attached either directly or indirectly to an affinity moiety.

The affinity moiety may comprise a chemical (non-peptide) molecule, an aptamer, a peptide or an antibody (e.g. antibody-derived epitope binding domain) which is capable of specifically binding to the above disclosed proteins.

In one aspect binding or specifically binding means a binding affinity (KD) of 10⁻⁸ mol/l or less, preferably 10⁻⁹ M to 10⁻¹³ mol/1.

As mentioned, the affinity moiety of this aspect of the present disclosure may also be an aptamer.

As used herein, the term "aptamer" refers to a nucleic acid that specifically binds to a target, such as a protein, through interactions other than Watson-Crick base pairing. In a particular aspect, the aptamer specifically binds to one or more targets (e.g., a protein or protein complex) to the general exclusion of other molecules in a sample. The aptamer may be a nucleic acid such as an RNA, a DNA, a modified nucleic acid, or a mixture thereof. The aptamer may also be a nucleic acid in a linear or circular form and may be single stranded or double stranded. The aptamer may comprise oligonucleotides that are at least 5, at least 10, at least 15, at least 20, at least 25, at least 30, at least 35, at least 40 or more nucleotides in length. Aptamers may comprise sequences that are up to 40, up to 60, up to 80, up to 100, up to 150, up to 200 or more nucleotides in length. Aptamers may be from about 5 to about 150 nucleotides, from about 10 to about 100 nucleotides, or from about 20 to about 75 nucleotides in length. While aptamers are discussed herein as nucleic acid molecules (e.g., oligonucleotides) aptamers, aptamer equivalents may also be used in place of the nucleic acid aptamers, such as peptide aptamers.

According to one aspect, the pharmaceutical agent and the affinity moiety (e.g. antibody or aptamer) are attached directly to one another.

The pharmaceutical agent of the disclosure may be attached or conjugated to the affinity moiety of the disclosure in various ways, depending on the context, application and purpose.

When both the pharmaceutical agent and the affinity moiety are polypeptides, the conjugate may be produced by recombinant means. For example, the nucleic acid sequence encoding a toxin (e.g., PE38KDEL) or a fluorescent protein [e.g., green fluorescent protein (GFP), red fluorescent protein (RFP) or yellow fluorescent protein (YFP)] may be ligated in-frame with the nucleic acid sequence encoding an antibody of the disclosure and be expressed in a host cell to produce a recombinant conjugated antibody. Alternatively, at least one of the affinity moiety or pharmaceutical agent may be chemically synthesized by, for example, the stepwise addition of one or more amino acid residues in defined order such as solid phase peptide synthetic techniques.

A pharmaceutical agent may also be attached to the affinity moiety of the disclosure using standard chemical synthesis techniques widely practiced in the art [see e.g., hypertexttransferprotocol://worldwideweb (dot) chemistry (dot) org/portal/Chemistry)], such as using any suitable chemical linkage, direct or indirect, as via a peptide bond (when the functional moiety is a polypeptide), or via covalent bonding to an intervening linker element, such as a linker peptide or other chemical moiety, such as an organic polymer. Chimeric peptides may be linked via bonding at the carboxy (C) or amino (N) termini of the peptides, or via bonding to internal chemical groups such as straight, branched or cyclic side chains, internal carbon or nitrogen atoms, and the like.

Exemplary methods for conjugating peptide pharmaceutical agent to polypeptide affinity moieties (e.g. antibodies) are described herein below:
***SPDP conjugation*** - A non-limiting example of a method of SPDP conjugation is described in Cumber et al. (1985, Methods of Enzymology 112: 207-224). Briefly, a peptide, such as a detectable or therapeutic moiety (e.g., 1.7 mg/ml) is mixed with a 10-fold excess of SPDP (50 mM in ethanol); the antibody is mixed with a 25-fold excess of SPDP in 20 mM sodium phosphate, 0.10 M NaCl pH 7.2 and each of the reactions is incubated for about 3 hours at room temperature. The reactions are then dialyzed against PBS. The peptide is reduced, e.g., with 50 mM DTT for 1 hour at room temperature. The reduced peptide is desalted by equilibration on G-25 column (up to 5 % sample/column volume) with 50 mM KH₂PO₄ pH 6.5. The reduced peptide is combined with the SPDP-antibody in a molar ratio of 1:10 antibody: peptide and incubated at 4 °C overnight to form a peptide-antibody conjugate.

***Glutaraldehyde conjugation** -* A non-limiting example of a method of glutaraldehyde conjugation is described in G.T. Hermanson (1996, "Antibody Modification and Conjugation, in Bioconjugate Techniques, Academic Press, San Diego). Briefly, the antibody and the peptide (1.1 mg/ml) are mixed at a 10-fold excess with 0.05 % glutaraldehyde in 0.1 M phosphate, 0.15 M NaCl pH 6.8, and allowed to react for 2 hours at room temperature. 0.01 M lysine can be added to block excess sites. After-the reaction, the excess glutaraldehyde is removed using a G-25 column equilibrated with PBS (10 % v/v sample/column volumes).

***Carbodiimide conjugation** -* Conjugation of a peptide with an antibody can be accomplished using a dehydrating agent such as a carbodiimide, e.g., in the presence of 4-dimethyl aminopyridine. Carbodiimide conjugation can be used to form a covalent bond between a carboxyl group of peptide and a hydroxyl group of an antibody (resulting in the formation of an ester bond), or an amino group of an antibody (resulting in the formation of an amide bond) or a sulfhydryl group of an antibody (resulting in the formation of a thioester bond). Likewise, carbodiimide coupling can be used to form analogous covalent bonds between a carbon group of an antibody and an hydroxyl, amino or sulfhydryl group of the peptide [see, J. March, Advanced Organic Chemistry: Reaction's, Mechanism, and Structure, pp. 349-50 & 372-74 (3d ed.), 1985]. For example, the peptide can be conjugated to an antibody via a covalent bond using a carbodiimide, such as dicyclohexylcarbodiimide [B. Neises et al. (1978), Angew Chem., Int. Ed. Engl. 17:522; A. Hassner et al. (1978, Tetrahedron Lett. 4475); E.P. Boden et al. (1986, J. Org. Chem. 50:2394) and L.J. Mathias (1979, Synthesis 561)].

When both the pharmaceutical agent and the affinity moiety are antibodies, the present disclosure contemplates generation of bispecific antibodies wherein each arm of the antibody recognizes a different antigen.

It will be appreciated that the affinity moiety and the pharmaceutical agent of this aspect of the present disclosure may be attached indirectly - e.g. via a particle, wherein the pharmaceutical agent is inside the particle or on the outer surface thereof and the affinity moiety is on the outer surface of the particle.

As used herein, "particles" refers to nano - micro structures which are not biological cells.

The particle may be a synthetic carrier, gel or other object or material having an external surface which is capable of being loadable with (e.g., encapsulating) a pharmaceutical agent. The particle may be either polymeric or non-polymeric preparations.

Exemplary particles that may be used according to this aspect of the present disclosure include, but are not limited to polymeric particles, microcapsules, liposomes, microspheres, microemulsions, nanoparticles, nanocapsules, nano-spheres, nano-liposomes, nano-emulsions and nanotubes.

According to a particular aspect, the particles are nanoparticles.

As used herein, the term "nanoparticle" refers to a particle or particles having an intermediate size between individual atoms and macroscopic bulk solids. Generally, nanoparticle has a characteristic size (*e.g*., diameter for generally spherical nanoparticles, or length for generally elongated nanoparticles) in the sub-micrometer range, *e.g.,* from about 1 nm to about 500 nm, or from about 1 nm to about 200 nm, or of the order of 10 nm, *e.g.,* from about 1 nm to about 100 nm. The nanoparticles may be of any shape, including, without limitation, elongated particle shapes, such as nanowires, or irregular shapes, in addition to more regular shapes, such as generally spherical, hexagonal and cubic nanoparticles. According to one aspect, the nanoparticles are generally spherical.

The particles of this aspect of the present disclosure may have a charged surface (i.e., positively charged or negatively charged) or a neutral surface.

Agents which are used to fabricate the particles may be selected according to the desired charge required on the outer surface of the particles.

Thus, for example if a negatively charged surface is desired, the particles may be fabricated from negatively charged lipids (i.e. anionic phospholipids) such as described herein below.

When a positively charged surface is desired, the particles may be fabricated from positively charged lipids (i.e. cationic phospholipids), such as described herein below.

As mentioned, non-charged particles are also contemplated by the present disclosure. Such particles may be fabricated from neutral lipids such as phosphatidylethanolamine or dioleoylphosphatidylethanolamine (DOPE).

It will be appreciated that combinations of different lipids may be used to fabricate the particles of the present disclosure, including a mixture of more than one cationic lipid, a mixture of more than one anionic lipid, a mixture of more than one neutral lipid, a mixture of at least one cationic lipid and at least one anionic lipid, a mixture of at least one cationic lipid and at least one neutral lipid, a mixture of at least one anionic lipid and at least one neutral lipid and additional combinations of the above. In addition, polymer-lipid based formulations may be used.

There are numerous polymers which may be attached to lipids. Polymers typically used as lipid modifiers include, without being limited thereto: polyethylene glycol (PEG), polysialic acid, polylactic (also termed polylactide), polyglycolic acid (also termed polyglycolide), poly-(lactic-co-glycolic)poly-(vinyl-alcohol), polyvinylpyrrolidone, polyethyloxazoline, polyllydroxyetlyloxazolille, solyhydroxypryloxazoline, polyhydroxypropyl methacrylamide, polymethacrylamide, polydimethylacrylamide, polyvinylmethylether, polyhydroxyethyl acrylate, derivatized celluloses such as hydroxymethylcellulose or hydroxyethylcellulose.

The polymers may be employed as homopolymers or as block or random copolymers.

The particles may also include other components. Examples of such other components includes, without being limited thereto, fatty alcohols, fatty acids, and/or cholesterol esters or any other pharmaceutically acceptable excipients which may affect the surface charge, the membrane fluidity and assist in the incorporation of the biologically active lipid into the lipid assembly. Examples of sterols include cholesterol, cholesterol hemisuccinate, cholesterol sulfate, or any other derivatives of cholesterol. Preferred lipid assemblies according the disclosure include either those which form a micelle (typically when the assembly is absent from a lipid matrix) or those which form a liposome (typically, when a lipid matrix is present).

The particles of the present disclosure may be modified. According modified to enhance their circulatory half-life (e.g. by PEGylation) to reduce their clearance, to prolong their scavenging time-frame and to allow antibody binding. The PEG which is incorporated into the articles may be characterized by of any of various combinations of chemical composition and/or molecular weight, depending on the application and purpose.

Methods of coupling affinity moieties (e.g. antibodies) on particle's outer surface (e.g., liposomes) are known in the art.

As used herein "coupling" or "coupled on" refers to covalent or non-covalent attachment of the affinity moiety to the particle.

Antibody conjugation methods which can be used in accordance with the teachings of the present disclosure can be divided to direct binding or indirect binding. Some methods are provided hereinbelow. While specifically referring to liposomes, the procedures described hereinbelow may be applied to a variety of particles, while using modified protocols simply applied by the ordinary artisan.

Direct conjugation methods are well known to those of skill in the art. See for example, G. Gregoriadis, (1984) "Liposome Technology" CRC Press, Boca Raton, Fla. and D. D. Lasic, "Liposomes: from physics to applications" (1993) Elsevier, Amsterdam; N.Y. Particularly preferred is conjugation through a thioether linkage. This may be accomplished by reacting the antibody with a maleimide derivatized lipid such as maleimide derivatized phosphatidylethanolamine (M-PE) or dipalmitoylethanolamine (M-DEP). This approach is described in detail by Martin et al. J. Biol. Chem., 257: 286-288 (1982).

In another preferred aspect, the antibody can be coupled to a hydrophilic polymer (e.g., a PEG). Means of attaching targeting molecules to polymer linkers are well known to those of skill in the art (see, e.g., chapter 4 in Monoclonal Antibodies: Principles and Applications, Birch and Lennox, eds., John Wiley & Sons, Inc., New York (1995); and Blume et al. Biochem. Biophys. Acta. 1149: 180-184 (1993). In a particularly preferred aspect, an antibody or a fragment thereof (e.g., Fab' fragment) is linked to a maleimide derivatized PEG through the --SII group of the antibody. The maleimide-derivative of PEG-PE is included in the liposome preparation as described above and below and the antibody can be conjugated with the liposome via the sulfhydryl group at pH 7.2.

Amine modifications making use of cross-linking agents such as EDC are taught in Endoh et al. 1981 J. Immun. Meth. 44:79-85; Dunnick 1975 J. Nuclear. Med. 16:483-487; Alternatively, direct modification of antibodies with activated fatty acids, such as N-hydroxysuccinimide (NHS) eater or palmitic acid, prior to incorporation into a liposome membrane, typically by detergent dialysis procedures (Huang et al. 1980, J. Biol. Chem. 255:8015-8018. Reagents, such as EDC, are used in conjunction with NHS to activate acidic functions on liposomes, which are then conjugated to the amino groups on antibodies. Better control of the conjugation reaction can be achieved using heterobifunctional cross-linkers which efficiently introduce a unique and selective reactive function, such as a protected thiol or maleimide group. Examples of these crosslinkers are SPDP (Barbet et al. 1981 J. Supramolec. Struct. Cell. Biochem. 16:243-258), S-acetylthioglycolic acid N-hydroxysuccinimide ester (SATA, Jones 1993 Biochim. Biophys. Acta. 1152:23:1-32; Schwendener 1990 Biochim. Biophys. Acta. 1026:69-79 and 4-(p-maleimidophenyl)butyric acid N-hydroxysuccinimide ester(SMPB (Hansen 1995 Biochim. Biophys. Acta. 1239:133-144). Antibodies which have been activated by these crosslinkers can, after deprotection where appropriate, react with activated lip- ids in liposome bilayers. Maleimide and protected thiol-derivatized lipids are available from commercial sources for this purpose.

Deprotection of 3-pyridyl disulfides is usually effected by DTT and occasionally by some other mercaptan. Once deprotected, sulfhydryl groups can react with maleimide (for example SMPB-modified conjugates) or iodo (for example, iodoacetic acid N-hydroxysuccinimide ester (SIAA)-modified conjugates) groups. Maleimide groups are recommended since iodo functions can react with amino groups in either of the substrates, leading to undesirable side products. Deprotection is not required for these reagents.

Indirect conjugation methods:
Biotin-avidin - For example, a biotin conjugated antibody may be bound to a particle (e.g., liposome) containing a streptavidin. Alternatively, the biotinylated antibody may be conjugated to a biotin derivatized liposome by an avidin or streptavidin linker. Ahmad et al., Cancer Res., 52: 4817-4820 (1992), describes such a mode of coupling. When monovalent Fab molecules are used, typically about 30 to 125 and more typically about 50 to 100 Fab' molecules per liposome are used.

Binding via protein A/G/L-liposome conjugates targeted to the Fc chain of antibodies is taught in Matthay et al. 1986 Cancer Res. 46:4904-4910; Machy et al. 1983 Biochem. Biophys. Acta. 901:157-160.

Loading of the particle with the pharmaceutical agent can be effected concomitant with, or following particle assembly.

Agents which increase the amount of the above disclosed proteins include agents which are capable of increasing the transcription (for example a transcription factor known to interact with the 5'untranslated region of the protein) of the protein, the translation of the protein or the stability of the protein. Additionally, the agent which increases the amount of the protein, may be a polynucleotide which encodes the protein itself or an active peptide thereof.

Recombinant techniques are typically used to generate the polypeptides of the present disclosure. These techniques may be preferred due to the number of amino acids of the polypeptides and the large amounts required thereof. Such recombinant techniques are described by Bitter et al., (1987) Methods in Enzymol. 153:516-544, Studier et al. (1990) Methods in Enzymol. 185:60-89, Brisson et al. (1984) Nature 310:511-514, Takamatsu et al. (1987) EMBO J. 6:307-311, Coruzzi et al. (1984) EMBO J. 3:1671-1680 and Brogli et al., (1984) Science 224:838-843, Gurley et al. (1986) Mol. Cell. Biol. 6:559-565 and Weissbach & Weissbach, 1988, Methods for Plant Molecular Biology, Academic Press, NY, Section VIII, pp 421-463.

To produce an expression vector for the expression of one of the proteins disclosed herein above, a polynucleotide encoding the protein is ligated into a nucleic acid expression vector, which comprises the polynucleotide sequence under the transcriptional control of a cis-regulatory sequence (e.g., promoter sequence) suitable for directing constitutive, tissue specific or inducible transcription of the protein of the present disclosure in the host cells.

The phrase "an isolated polynucleotide" refers to a single or double stranded nucleic acid sequence which is isolated and provided in the form of an RNA sequence, a complementary polynucleotide sequence (cDNA), a genomic polynucleotide sequence and/or a composite polynucleotide sequences (e.g., a combination of the above).

As used herein the phrase "complementary polynucleotide sequence" refers to a sequence, which results from reverse transcription of messenger RNA using a reverse transcriptase or any other RNA dependent DNA polymerase. Such a sequence can be subsequently amplified *in vivo* or *in vitro* using a DNA dependent DNA polymerase.

As used herein the phrase "genomic polynucleotide sequence" refers to a sequence derived (isolated) from a chromosome and thus it represents a contiguous portion of a chromosome.

As used herein the phrase "composite polynucleotide sequence" refers to a sequence, which is at least partially complementary and at least partially genomic. A composite sequence can include some exonal sequences required to encode the protein of the present disclosure, as well as some intronic sequences interposing there between. The intronic sequences can be of any source, including of other genes, and typically will include conserved splicing signal sequences. Such intronic sequences may further include cis acting expression regulatory elements.

As mentioned hereinabove, polynucleotide sequences of the present disclosure are inserted into expression vectors (i.e., a nucleic acid construct) to enable expression of the recombinant protein. The expression vector of the present disclosure may include additional sequences which render this vector suitable for replication and integration in prokaryotes, eukaryotes, or preferably both (e.g., shuttle vectors). Typical cloning vectors contain transcription and translation initiation sequences (e.g., promoters, enhances) and transcription and translation terminators (e.g., polyadenylation signals).

A variety of prokaryotic or eukaryotic cells can be used as host-expression systems to express the protein of the present disclosure. These include, but are not limited to, microorganisms, such as bacteria transformed with a recombinant bacteriophage DNA, plasmid DNA or cosmid DNA expression vector containing the protein coding sequence; yeast transformed with recombinant yeast expression vectors containing the protein coding sequence; plant cell systems infected with recombinant virus expression vectors (e.g., cauliflower mosaic virus, CaMV; tobacco mosaic virus, TMV) or transformed with recombinant plasmid expression vectors, such as Ti plasmid, containing the protein coding sequence.

Other than containing the necessary elements for the transcription and translation of the inserted coding sequence, the expression construct of the present disclosure can also include sequences engineered to optimize stability, production, purification, yield or activity of the expressed protein.

Various methods can be used to introduce the expression vector of the present disclosure into the host cell system. Such methods are generally described in Sambrook et al., Molecular Cloning: A Laboratory Manual, Cold Springs Harbor Laboratory, New York (1989, 1992), in Ausubel et al., Current Protocols in Molecular Biology, John Wiley and Sons, Baltimore, Md. (1989), Chang et al., Somatic Gene Therapy, CRC Press, Ann Arbor, Mich. (1995), Vega et al., Gene Targeting, CRC Press, Ann Arbor Mich. (1995), Vectors: A Survey of Molecular Cloning Vectors and Their Uses, Butterworths, Boston Mass. (1988) and Gilboa et al. [Biotechniques 4 (6): 504-512, 1986] and include, for example, stable or transient transfection, lipofection, electroporation and infection with recombinant viral vectors. In addition, see U.S. Pat. Nos. 5,464,764 and 5,487,992 for positive-negative selection methods.

Transformed cells are cultured under effective conditions, which allow for the expression of high amounts of recombinant protein. Effective culture conditions include, but are not limited to, effective media, bioreactor, temperature, pH and oxygen conditions that permit protein production. An effective medium refers to any medium in which a cell is cultured to produce the recombinant protein of the present disclosure. Such a medium typically includes an aqueous solution having assimilable carbon, nitrogen and phosphate sources, and appropriate salts, minerals, metals and other nutrients, such as vitamins. Cells of the present disclosure can be cultured in conventional fermentation bioreactors, shake flasks, test tubes, microtiter dishes and petri plates. Culturing can be carried out at a temperature, pH and oxygen content appropriate for a recombinant cell. Such culturing conditions are within the expertise of one of ordinary skill in the art.

Depending on the vector and host system used for production, resultant protein of the present disclosure may either remain within the recombinant cell, secreted into the fermentation medium, secreted into a space between two cellular membranes, such as the periplasmic space in *E. coli;* or retained on the outer surface of a cell or viral membrane.

Following a predetermined time in culture, recovery of the recombinant protein is affected.

The phrase "recovering the recombinant protein" used herein refers to collecting the whole fermentation medium containing the protein and need not imply additional steps of separation or purification.

Thus, the polypeptides of the present disclosure can be purified using a variety of standard protein purification techniques, such as, but not limited to, affinity chromatography, ion exchange chromatography, filtration, electrophoresis, hydrophobic interaction chromatography, gel filtration chromatography, reverse phase chromatography, concanavalin A chromatography, chromatofocusing and differential solubilization.

To facilitate recovery, the expressed coding sequence can be engineered to encode the protein fused to a cleavable moiety. Such a fusion protein can be designed so that the protein can be readily isolated by affinity chromatography; e.g., by immobilization on a column specific for the cleavable moiety. Where a cleavage site is engineered between the protein and the cleavable moiety, the protein can be released from the chromatographic column by treatment with an appropriate enzyme or agent that specifically cleaves the fusion protein at this site [e.g., see Booth et al., Immunol. Lett. 19:65-70 (1988); and Gardella et al., J. Biol. Chem. 265:15854-15859 (1990)].

The protein of the present disclosure is preferably retrieved in "substantially pure" form.

As used herein, the phrase "substantially pure" refers to a purity that allows for the effective use of the protein in the applications described herein.

In addition to being synthesizable in host cells, the protein of the present disclosure can also be synthesized using *in vitro* expression systems. These methods are well known in the art and the components of the system are commercially available.

As mentioned, the protein may be administered to the subject in need thereof as polynucleotides where they are expressed in vivo i.e. gene therapy.

The phrase "gene therapy" as used herein refers to the transfer of genetic material (e.g. DNA or RNA) of interest into a host to treat or prevent a genetic or acquired disease or condition or phenotype. The genetic material of interest encodes a product (e.g. a protein, polypeptide, peptide, functional RNA, antisense) whose production *in vivo* is desired. For example, the genetic material of interest can encode a hormone, receptor, enzyme, polypeptide or peptide of therapeutic value. For review see, in general, the text "Gene Therapy" (Advanced in Pharmacology 40, Academic Press, 1997).

Two basic approaches to gene therapy have evolved: (1) ex vivo and (2) *in vivo* gene therapy. In ex vivo gene therapy cells are removed from a patient, and while being cultured are treated *in vitro.* Generally, a functional replacement gene is introduced into the cell via an appropriate gene delivery vehicle/method (transfection, transduction, homologous recombination, etc.) and an expression system as needed and then the modified cells are expanded in culture and returned to the host/patient. These genetically reimplanted cells have been shown to express the transfected genetic material in situ. The cells may be autologous or non-autologous to the subject. Since non-autologous cells are likely to induce an immune reaction when administered to the body several approaches have been developed to reduce the likelihood of rejection of non-autologous cells. These include either suppressing the recipient immune system or encapsulating the non-autologous cells in immunoisolating, semipermeable membranes before transplantation.

In *in vivo* gene therapy, target cells are not removed from the subject rather the genetic material to be transferred is introduced into the cells of the recipient organism in situ, which is within the recipient. These genetically altered cells have been shown to express the transfected genetic material in situ.

To confer specificity, preferably the nucleic acid constructs used to express the protein of the present disclosure comprise cell-specific promoter sequence elements.

Recombinant viral vectors are useful for *in vivo* expression of the protein of the present disclosure since they offer advantages such as lateral infection and targeting specificity. Lateral infection is inherent in the life cycle of, for example, retrovirus and is the process by which a single infected cell produces many progeny virions that bud off and infect neighboring cells. The result is that a large area becomes rapidly infected, most of which was not initially infected by the original viral particles. This is in contrast to vertical-type of infection in which the infectious agent spreads only through daughter progeny. Viral vectors can also be produced that are unable to spread laterally. This characteristic can be useful if the desired purpose is to introduce a specified gene into only a localized number of targeted cells.

It will be appreciated that up-regulation of expression of the above described proteins can also be effected at the polynucleotide level - e.g. use of specific miRNAs or anti-miRNAs which are known to up-regulate expression.

The agents of the present disclosure can be administered to an organism per se, or in a pharmaceutical composition where it is mixed with suitable carriers or excipients.

As used herein a "pharmaceutical composition" refers to a preparation of one or more of the active ingredients described herein with other chemical components such as physiologically suitable carriers and excipients. The purpose of a pharmaceutical composition is to facilitate administration of a compound to an organism.

Herein the term "active ingredient" refers to the agent which regulates one of the disclosed proteins accountable for the biological effect.

Hereinafter, the phrases "physiologically acceptable carrier" and "pharmaceutically acceptable carrier" which may be interchangeably used refer to a carrier or a diluent that does not cause significant irritation to an organism and does not abrogate the biological activity and properties of the administered compound. An adjuvant is included under these phrases.

Herein the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils and polyethylene glycols.

Techniques for formulation and administration of drugs may be found in "Remington's Pharmaceutical Sciences," Mack Publishing Co., Easton, PA, latest edition.

Pharmaceutical compositions of some aspects of the disclosure may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with some aspects of the disclosure thus may be formulated in conventional manner using one or more physiologically acceptable carriers comprising excipients and auxiliaries, which facilitate processing of the active ingredients into preparations which, can be used pharmaceutically. Proper formulation is dependent upon the route of administration chosen.

For injection, the active ingredients of the pharmaceutical composition may be formulated in aqueous solutions, preferably in physiologically compatible buffers such as Hank's solution, Ringer's solution, or physiological salt buffer. For transmucosal administration, penetrants appropriate to the barrier to be permeated are used in the formulation. Such penetrants are generally known in the art.

For oral administration, the pharmaceutical composition can be formulated readily by combining the active compounds with pharmaceutically acceptable carriers well known in the art. Such carriers enable the pharmaceutical composition to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carbomethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active compound doses.

Pharmaceutical compositions which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration.

For buccal administration, the compositions may take the form of tablets or lozenges formulated in conventional manner.

For administration by nasal inhalation, the active ingredients for use according to some aspects of the disclosure are conveniently delivered in the form of an aerosol spray presentation from a pressurized pack or a nebulizer with the use of a suitable propellant, e.g., dichlorodifluoromethane, trichlorofluoromethane, dichloro-tetrafluoroethane or carbon dioxide. In the case of a pressurized aerosol, the dosage unit may be determined by providing a valve to deliver a metered amount. Capsules and cartridges of, e.g., gelatin for use in a dispenser may be formulated containing a powder mix of the compound and a suitable powder base such as lactose or starch.

The pharmaceutical composition described herein may be formulated for parenteral administration, e.g., by bolus injection or continuos infusion. Formulations for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the active preparation in water-soluble form. Additionally, suspensions of the active ingredients may be prepared as appropriate oily or water based injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents which increase the solubility of the active ingredients to allow for the preparation of highly concentrated solutions.

Alternatively, the active ingredient may be in powder form for constitution with a suitable vehicle, e.g., sterile, pyrogen-free water based solution, before use.

The pharmaceutical composition of some aspects of the disclosure may also be formulated in rectal compositions such as suppositories or retention enemas, using, e.g., conventional suppository bases such as cocoa butter or other glycerides.

Pharmaceutical compositions suitable for use in context of some aspects of the disclosure include compositions wherein the active ingredients are contained in an amount effective to achieve the intended purpose. More specifically, a therapeutically effective amount means an amount of active ingredients effective to prevent, alleviate or ameliorate symptoms of a disorder (e.g., lung cancer) or prolong the survival of the subject being treated.

Determination of a therapeutically effective amount is well within the capability of those skilled in the art, especially in light of the detailed disclosure provided herein.

For any preparation used in the methods of the disclosure, the therapeutically effective amount or dose can be estimated initially from in vitro and cell culture assays. For example, a dose can be formulated in animal models to achieve a desired concentration or titer. Such information can be used to more accurately determine useful doses in humans.

Toxicity and therapeutic efficacy of the active ingredients described herein can be determined by standard pharmaceutical procedures in vitro, in cell cultures or experimental animals. The data obtained from these in vitro and cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.1).

Dosage amount and interval may be adjusted individually to provide pulmonary levels of the active ingredient are sufficient to induce or suppress the biological effect (minimal effective concentration, MEC). The MEC will vary for each preparation, but can be estimated from in vitro data. Dosages necessary to achieve the MEC will depend on individual characteristics and route of administration. Detection assays can be used to determine plasma concentrations.

Depending on the severity and responsiveness of the condition to be treated, dosing can be of a single or a plurality of administrations, with course of treatment lasting from several days to several weeks or until cure is effected or diminution of the disease state is achieved.

The amount of a composition to be administered will, of course, be dependent on the subject being treated, the severity of the affliction, the manner of administration, the judgment of the prescribing physician, etc.

Compositions of some aspects of the disclosure may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert. Compositions comprising a preparation of the disclosure formulated in a compatible pharmaceutical carrier may also be prepared, placed in an appropriate container, and labeled for treatment of an indicated condition, as is further detailed above.

According to any of the above described aspects of the present disclosure, the step of administering occurs by pulmonary delivery. According to some aspects, the step of administering may occur systemically either orally, buccally, parenterally, topically, by inhalation, by insufflation, or rectally, or may occur locally by means such as, but not limited to, injection, implantation, grafting, topical application, or parenterally. Additional administration may be performed, for example, intravenously, transmucosally, transdermally, intramuscularly, subcutaneously, intratracheally (including by pulmonary inhalation), intraperitoneally, intrathecally, intralymphatically, intralesionally, or epidurally. Administering can be performed, for example, once, a plurality of times, and/or over one or more extended periods either as individual unit doses or in the form of a treatment regimen comprising multiple unit doses of multiple drugs and/or substances.

The term "inhalation" as used herein refers to the act of drawing in a medicated vapor with the breath.

The term "inhalation delivery device" as used herein refers to a machine/apparatus or component that produces small droplets or an aerosol from a liquid or dry powder aerosol formulation and is used for administration through the mouth in order to achieve pulmonary administration of a drug, e.g., in solution, powder, and the like. Examples of inhalation delivery device include, but are not limited to, a nebulizer, a metered-dose inhaler, and a dry powder inhaler (DPI).

According to some other aspects, the step of administering occurs at one time as a single dose. According to some other aspects, the step of administering is performed as a plurality of doses over a period of time. According to some such aspects, the period of time is a day, a week, a month, a year, or multiples thereof. According to some aspects, the step of administering is performed daily for a period of at least one week. According to some aspects, the step of administering is performed weekly for a period of at least one month. According to some aspects, the step of administering is performed monthly for a period of at least two months. According to another aspect, the step of administering is performed repeatedly over a period of at least one year. According to another aspect, the step of administering is performed at least once monthly. According to another aspect, the step of administering is performed at least once weekly. According to another aspect, the step of administering is performed at least once daily.

According to some other aspects, the therapeutic amount of the pharmaceutical composition is administered via an inhalation device. Examples of the inhalation device that can be used for administering the pharmaceutical composition include, but are not limited to, a nebulizer, a metered-dose inhaler (MDI), a dry powder inhaler (DPI), and a dry powder nebulizer.

According to another aspect, the dry powder comprises microparticles with Mass Median Aerodynamic Diameter (MMAD) of 1 to 5 microns. According to another aspect, the dry powder comprises microparticles with Mass Median Aerodynamic Diameter (MMAD) of about 2 micron.

Any of the therapeutic agents of the present disclosure may be administered together with other agents known to be therapeutic for lung cancer.

According to some such aspects, the additional therapeutic agent is a chemotherapeutic agent. Examples of chemotherapeutic agents include, but are not limited to, Abitrexate (Methotrexate), Abraxane (Paclitaxel Albumin-stabilized Nanoparticle Formulation), Afatinib Dimaleate, Alimta (Pemetrexed Disodium), Avastin (Bevacizumab), Bvacizumab, Carboplatin, Ceritinib, Cisplatin, Crizotinib, Cyramza (Ramucirumab), Docetaxel, Erlotinib Hydrochloride, Folex PFS (Methotrexate), Gefitinib, Gilotrif (Afatinib Dmaleate), Gemcitabine Hydrochloride, Gemzar (Gemcitabine Hydrochloride), Iressa (Gefitinib), Mechlorethamine Hydrochloride, Methotrexate, Methotrexate LPF (Methotrexate), Mexate (Methotrexate), Mexate-AQ (Methotrexate), Mustargen (mechlorethamine Hydrochloride), Navelbine (Vinorelbine Tartrate), Paclitaxel, Paclitaxel Albumin-stabilized nanoparticle Formulation, Paraplat (Carboplatin), Paraplatin (Carboplatin), Pemetrexed Disodium, Platinol (Cisplatin), Platinol-AQ (Cisplatin), Ramucirumab, Tarceva (Erlotinib Hydrochloride), Taxol (Paclitaxel), Taxotere (Docetaxel), Vinorelbine Tartrate, Xalkori (Crizotinib), Zykadia (Ceritinib), Carboplatin-Taxol, and Gemcitabine-Cisplatin.

According to another aspect, the additional therapeutic agent is an analgesic agent. According to some aspects, the analgesic agent relieves pain by elevating the pain threshold without disturbing consciousness or altering other sensory modalities. According to some such aspects, the analgesic agent is a non-opioid analgesic. "Non-opioid analgesics" are natural or synthetic substances that reduce pain but are not opioid analgesics. Examples of non-opioid analgesics include, but are not limited to, etodolac, indomethacin, sulindac, tolmetin, nabumetone, piroxicam, acetaminophen, fenoprofen, flurbiprofen, ibuprofen, ketoprofen, naproxen, naproxen sodium, oxaprozin, aspirin, choline magnesium trisalicylate, diflunisal, meclofenamic acid, mefenamic acid, and phenylbutazone. According to some other aspects, the analgesic is an opioid analgesic. "Opioid analgesics", "opioid", or "narcotic analgesics" are natural or synthetic substances that bind to opioid receptors in the central nervous system, producing an agonist action. Examples of opioid analgesics include, but are not limited to, codeine, fentanyl, hydromorphone, levorphanol, meperidine, methadone, morphine, oxycodone, oxymorphone, propoxyphene, buprenorphine, butorphanol, dezocine, nalbuphine, and pentazocine.

According to another aspect, the additional therapeutic agent is an anti-infective agent. According to another aspect, the anti-infective agent is an antibiotic agent. The term "antibiotic agent" as used herein means any of a group of chemical substances having the capacity to inhibit the growth of, or to destroy bacteria and other microorganisms, used chiefly in the treatment of infectious diseases. Examples of antibiotic agents include, but are not limited to, Penicillin G; Methicillin; Nafcillin; Oxacillin; Cloxacillin; Dicloxacillin; Ampicillin; Amoxicillin; Ticarcillin; Carbenicillin; Mezlocillin; Azlocillin; Piperacillin; Imipenem; Aztreonam; Cephalothin; Cefaclor; Cefoxitin; Cefuroxime; Cefonicid; Cefmetazole; Cefotetan; Cefprozil; Loracarbef; Cefetamet; Cefoperazone; Cefotaxime; Ceftizoxime; Ceftriaxone; Ceftazidime; Cefepime; Cefixime; Cefpodoxime; Cefsulodin; Fleroxacin; Nalidixic acid; Norfloxacin; Ciprofloxacin; Ofloxacin; Enoxacin; Lomefloxacin; Cinoxacin; Doxycycline; Minocycline; Tetracycline; Amikacin; Gentamicin; Kanamycin; Netilmicin; Tobramycin; Streptomycin; Azithromycin; Clarithromycin; Erythromycin; Erythromycin estolate; Erythromycin ethyl succinate; Erythromycin glucoheptonate; Erythromycin lactobionate; Erythromycin stearate; Vancomycin; Teicoplanin; Chloramphenicol; Clindamycin; Trimethoprim; Sulfamethoxazole; Nitrofurantoin; Rifampin; Mupirocin; Metronidazole; Cephalexin; Roxithromycin; Co-amoxiclavuanate; combinations of Piperacillin and Tazobactam; and their various salts, acids, bases, and other derivatives. Antibacterial antibiotic agents include, but are not limited to, penicillins, cephalosporins, carbacephems, cephamycins, carbapenems, monobactams, aminoglycosides, glycopeptides, quinolones, tetracyclines, macrolides, and fluoroquinolones.

According to some other aspects, the additional therapeutic agent comprises a bronchodilator including, but not limited to, a leukotriene modifier, an anticholinergic bronchodilator, a β2-agonist, or a combination thereof.

According to another aspect, the additional therapeutic agent comprises a corticosteroid including, but not limited to, prednisone, budesonide, mometasone, beclemethasone, or a combination thereof.

According to another aspect, the additional therapeutic agent is an anti-inflammatory agent.

According to another aspect, the anti-inflammatory agent is a nonsteroidal anti-inflammatory agent. Mixtures of non-steroidal anti-inflammatory agents also may be employed, as well as the dermatologically acceptable salts and esters of these agents. For example, etofenamate, a flufenamic acid derivative, is particularly useful for topical application.

According to another aspect, wherein the nonsteroidal anti-inflammatory agent comprises Transforming Growth Factor-.beta.3 (TGF-β.3), an anti-Tumor Necrosis Factor-alpha (TNF-α) agent, or a combination thereof.

According to another aspect, the anti-inflammatory agent is a steroidal anti-inflammatory agent. According to another aspect, the steroidal anti-inflammatory agent comprises at least one corticosteroid selected from the group consisting of prednisone, budesonide, mometasone, beclemethasone, and a combination thereof.

According to another aspect, the additional therapeutic agent comprises a methylxanthine.

According to another aspect, the additional therapeutic agent comprises a neutrophil elastase inhibitor.

According to another aspect, the additional therapeutic agent is at least one neutrophil elastase inhibitor, including, but not limited to, ICI 200355, ONO-5046, MR-889, L-694,458, CE-1037, GW-311616, TEI-8362, ONO-6818, AE-3763, FK-706, ICI-200,880, ZD-0892, ZD-8321, and a combination thereof.

According to another aspect, the additional therapeutic agent comprises at least one phosphodiesterase inhibitor, including, but not limited to, phosphodiesterase 4 inhibitor. Examples of phosphodiesterase 4 inhibitors include, but are not limited to, roflumilast, cilomilast or a combination thereof.

As well as treating lung cancer, the present disclosure also contemplates diagnosing lung cancer.

Thus, according to yet another aspect of the present disclosure, there is provided a method of diagnosing lung cancer in a subject comprising analyzing amount and/or activity of at least one polypeptide selected from the group consisting of MYOF, CTNS, FCGR2B, PCDHGC5, POMGNT2, ACSL1, CTAGE5, ADRB1, TECPR2, CASC5, WDR48, MCPH1, PPP2R3C, JAG2, GEMIN7, PTPRB, PRMT9, Ube2L3, TP53RK, PSME3, CDH5, PAPDC2, AGER, GYPA, CAV1, PPAPDC2 and MAGEE1 present in a lung tumor sample of the subject, wherein a change in the amount and/or activity as compared to the amount and/or activity of the at least one polypeptide in a non-tumor sample is indicative of lung cancer.

The term "diagnosing" as used herein refers to determining the presence of a disease, classifying a disease, staging a disease, determining a severity of a disease, monitoring disease progression, forecasting an outcome of the disease, predicting survival and/or prospects of recovery (i.e. prognosis).

The subject may be a healthy animal or human subject undergoing a routine well-being checkup. Alternatively, the subject may be at risk of having the disease (e.g., a genetically predisposed subject, a subject with medical and/or family history of cancer, a subject who has been exposed to carcinogens, occupational hazard, environmental hazard] and/or a subject who exhibits suspicious clinical signs of the disease [e.g., blood in the stool or melena, coughing, shortness of breath, unexplained pain, sweating, unexplained fever, unexplained loss of weight up to anorexia, changes in bowel habits (constipation and/or diarrhea), tenesmus, anemia and/or general weakness).

Determining an expression of any of the polypeptides listed above may be effected on the RNA or protein level as detailed below.

According to one aspect, the determining is effected *ex vivo.*

According to another aspect, the determining is effected *in vivo.*

### Methods of detecting expression of the polypeptides on the RNA level

In order to detect expression of the polypeptides on the RNA level, typically polynucleotide probes (e.g. oligonucleotides or primers) are used that are capable of specifically hybridizing to their RNA or cDNA generated therefrom.

Preferably, the oligonucleotide probes and primers utilized by the various hybridization techniques described hereinabove are capable of hybridizing to their targets under stringent hybridization conditions.

By way of example, hybridization of short nucleic acids (below 200 bp in length, e.g. 17-40 bp in length) can be effected by the following hybridization protocols depending on the desired stringency; (i) hybridization solution of 6 x SSC and 1 % SDS or 3 M TMACl, 0.01 M sodium phosphate (pH 6.8), 1 mM EDTA (pH 7.6), 0.5 % SDS, 100 µg/ml denatured salmon sperm DNA and 0.1 % nonfat dried milk, hybridization temperature of 1 - 1.5 °C below the Tm, final wash solution of 3 M TMACl, 0.01 M sodium phosphate (pH 6.8), 1 mM EDTA (pH 7.6), 0.5 % SDS at 1 - 1.5 °C below the Tm (stringent hybridization conditions) (ii) hybridization solution of 6 x SSC and 0.1 % SDS or 3 M TMACI, 0.01 M sodium phosphate (pH 6.8), 1 mM EDTA (pH 7.6), 0.5 % SDS, 100 µg/ml denatured salmon sperm DNA and 0.1 % nonfat dried milk, hybridization temperature of 2 - 2.5 °C below the Tm, final wash solution of 3 M TMACl, 0.01 M sodium phosphate (pH 6.8), 1 mM EDTA (pH 7.6), 0.5 % SDS at 1 - 1.5 °C below the Tm, final wash solution of 6 x SSC, and final wash at 22 °C (stringent to moderate hybridization conditions); and (iii) hybridization solution of 6 x SSC and 1 % SDS or 3 M TMACI, 0.01 M sodium phosphate (pH 6.8), 1 mM EDTA (pH 7.6), 0.5 % SDS, 100 µg/ml denatured salmon sperm DNA and 0.1 % nonfat dried milk, hybridization temperature at 2.5-3 °C below the Tm and final wash solution of 6 x SSC at 22 °C (moderate hybridization solution).

***Northern Blot analysis:*** This method involves the detection of a particular RNA in a mixture of RNAs. An RNA sample is denatured by treatment with an agent (e.g., formaldehyde) that prevents hydrogen bonding between base pairs, ensuring that all the RNA molecules have an unfolded, linear conformation. The individual RNA molecules are then separated according to size by gel electrophoresis and transferred to a nitrocellulose or a nylon-based membrane to which the denatured RNAs adhere. The membrane is then exposed to labeled DNA probes. Probes may be labeled using radio-isotopes or enzyme linked nucleotides. Detection may be using autoradiography, colorimetric reaction or chemiluminescence. This method allows both quantitation of an amount of particular RNA molecules and determination of its identity by a relative position on the membrane which is indicative of a migration distance in the gel during electrophoresis.

***RT-PCR analysis:*** This method uses PCR amplification of relatively rare RNAs molecules. First, RNA molecules are purified from the cells and converted into complementary DNA (cDNA) using a reverse transcriptase enzyme (such as an MMLV-RT) and primers such as, oligo dT, random hexamers or gene specific primers. Then by applying gene specific primers and Taq DNA polymerase, a PCR amplification reaction is carried out in a PCR machine. Those of skills in the art are capable of selecting the length and sequence of the gene specific primers and the PCR conditions (*i.e.,* annealing temperatures, number of cycles and the like) which are suitable for detecting specific RNA molecules. It will be appreciated that a semi-quantitative RT-PCR reaction can be employed by adjusting the number of PCR cycles and comparing the amplification product to known controls.

***RNA in situ hybridization stain:*** In this method DNA or RNA probes are attached to the RNA molecules present in the cells. Generally, the cells are first fixed to microscopic slides to preserve the cellular structure and to prevent the RNA molecules from being degraded and then are subjected to hybridization buffer containing the labeled probe. The hybridization buffer includes reagents such as formamide and salts (e.g., sodium chloride and sodium citrate) which enable specific hybridization of the DNA or RNA probes with their target mRNA molecules *in situ* while avoiding non-specific binding of probe. Those of skills in the art are capable of adjusting the hybridization conditions (*i.e.,* temperature, concentration of salts and formamide and the like) to specific probes and types of cells. Following hybridization, any unbound probe is washed off and the slide is subjected to either a photographic emulsion which reveals signals generated using radio-labeled probes or to a colorimetric reaction which reveals signals generated using enzyme-linked labeled probes.

***In situ RT-PCR stain:*** This method is described in Nuovo GJ, et al. [Intracellular localization of polymerase chain reaction (PCR)-amplified hepatitis C cDNA. Am J Surg Pathol. 1993, 17: 683-90] and Komminoth P, et al. [Evaluation of methods for hepatitis C virus detection in archival liver biopsies. Comparison of histology, immunohistochemistry, in situ hybridization, reverse transcriptase polymerase chain reaction (RT-PCR) and in situ RT-PCR. Pathol Res Pract. 1994, 190: 1017-25]. Briefly, the RT-PCR reaction is performed on fixed cells by incorporating labeled nucleotides to the PCR reaction. The reaction is carried on using a specific *in situ* RT-PCR apparatus such as the laser-capture microdissection PixCell I LCM system available from Arcturus Engineering (Mountainview, CA).

***Oligonucleotide microarray*** - In this method oligonucleotide probes capable of specifically hybridizing with the polynucleotides of the present disclosure are attached to a solid surface (e.g., a glass wafer). Each oligonucleotide probe is of approximately 20-25 nucleic acids in length. To detect the expression pattern of the polynucleotides of the present disclosure in a specific cell sample (e.g., blood cells), RNA is extracted from the cell sample using methods known in the art (using e.g., a TRIZOL solution, Gibco BRL, USA). Hybridization can take place using either labeled oligonucleotide probes (e.g., 5'-biotinylated probes) or labeled fragments of complementary DNA (cDNA) or RNA (cRNA). Briefly, double stranded cDNA is prepared from the RNA using reverse transcriptase (RT) (e.g., Superscript II RT), DNA ligase and DNA polymerase I, all according to manufacturer's instructions (Invitrogen Life Technologies, Frederick, MD, USA). To prepare labeled cRNA, the double stranded cDNA is subjected to an *in vitro* transcription reaction in the presence of biotinylated nucleotides using e.g., the BioArray High Yield RNA Transcript Labeling Kit (Enzo, Diagnostics, Affymetix Santa Clara CA). For efficient hybridization the labeled cRNA can be fragmented by incubating the RNA in 40 mM Tris Acetate (pH 8.1), 100 mM potassium acetate and 30 mM magnesium acetate for 35 minutes at 94 °C. Following hybridization, the microarray is washed and the hybridization signal is scanned using a confocal laser fluorescence scanner which measures fluorescence intensity emitted by the labeled cRNA bound to the probe arrays.

For example, in the Affymetrix microarray (Affymetrix^{®}, Santa Clara, CA) each gene on the array is represented by a series of different oligonucleotide probes, of which, each probe pair consists of a perfect match oligonucleotide and a mismatch oligonucleotide. While the perfect match probe has a sequence exactly complimentary to the particular gene, thus enabling the measurement of the level of expression of the particular gene, the mismatch probe differs from the perfect match probe by a single base substitution at the center base position. The hybridization signal is scanned using the Agilent scanner, and the Microarray Suite software subtracts the non-specific signal resulting from the mismatch probe from the signal resulting from the perfect match probe.

### Methods of detecting the polypeptides on the protein level

Determining expression of the polypeptides on the protein level is typically effected using an antibody capable of specifically interacting with same. Methods of detecting the above described proteins include immunoassays which include but are not limited to competitive and non-competitive assay systems using techniques such as Western blots, radioimmunoassays, ELISA (enzyme linked immunosorbent assay), "sandwich" immunoassays, and immunoprecipitation assays and immunohistochemical assays as detailed herein below.

Below is a list of techniques which may be used to determine the level of the proteins described herein above on the protein level.

***Enzyme linked immunosorbent assay (ELISA):*** This method involves fixation of a sample (e.g., fixed cells or a proteinaceous solution) containing a protein substrate to a surface such as a well of a microtiter plate. A substrate specific antibody coupled to an enzyme is applied and allowed to bind to the substrate. Presence of the antibody is then detected and quantitated by a colorimetric reaction employing the enzyme coupled to the antibody. Enzymes commonly employed in this method include horseradish peroxidase and alkaline phosphatase. If well calibrated and within the linear range of response, the amount of substrate present in the sample is proportional to the amount of color produced. A substrate standard is generally employed to improve quantitative accuracy.

***Western blot:*** This method involves separation of a substrate from other protein by means of an acrylamide gel followed by transfer of the substrate to a membrane (e.g., nylon or PVDF). Presence of the substrate is then detected by antibodies specific to the substrate, which are in turn detected by antibody binding reagents. Antibody binding reagents may be, for example, protein A, or other antibodies. Antibody binding reagents may be radiolabeled or enzyme linked as described hereinabove. Detection may be by autoradiography, colorimetric reaction or chemiluminescence. This method allows both quantitation of an amount of substrate and determination of its identity by a relative position on the membrane which is indicative of a migration distance in the acrylamide gel during electrophoresis.

***Radio-immunoassay (RIA):*** In one version, this method involves precipitation of the desired protein (*i.e.,* the substrate) with a specific antibody and radiolabeled antibody binding protein (e.g., protein A labeled with I¹²⁵) immobilized on a precipitable carrier such as agarose beads. The number of counts in the precipitated pellet is proportional to the amount of substrate.

In an alternate version of the RIA, a labeled substrate and an unlabelled antibody binding protein are employed. A sample containing an unknown amount of substrate is added in varying amounts. The decrease in precipitated counts from the labeled substrate is proportional to the amount of substrate in the added sample.

***Fluorescence activated cell sorting (FACS):*** This method involves detection of a substrate *in situ* in cells by substrate specific antibodies. The substrate specific antibodies are linked to fluorophores. Detection is by means of a cell sorting machine which reads the wavelength of light emitted from each cell as it passes through a light beam. This method may employ two or more antibodies simultaneously. It will be appreciated that when the protein is not expressed selectively on the cell membrane, care should be taken to avoid the antibody penetrating the cell membrane.

***Immunohistochemical analysis:*** This method involves detection of a substrate *in situ* in fixed cells by substrate specific antibodies. The substrate specific antibodies may be enzyme linked or linked to fluorophores. Detection is by microscopy and subjective or automatic evaluation. If enzyme linked antibodies are employed, a colorimetric reaction may be required. It will be appreciated that immunohistochemistry is often followed by counterstaining of the cell nuclei using for example Hematoxyline or Giemsa stain. It will be appreciated that when the protein is not expressed selectively on the cell membrane, care should be taken to avoid the antibody penetrating the cell membrane.

***In situ activity assay:*** According to this method, a chromogenic substrate is applied on the cells containing an active enzyme and the enzyme catalyzes a reaction in which the substrate is decomposed to produce a chromogenic product visible by a light or a fluorescent microscope.

As mentioned, the identifying/diagnosing/staging is carried out by analyzing an amount or activity of the polypeptides in a cell sample of the subject, wherein a difference in an amount or activity thereof beyond a predetermined threshold with respect to a control cell sample is indicative of the disease. It will be appreciated that the amount of change may correspond with a degree or a stage of the disease. Thus, larger differences may indicate a later stage of the disease with a poorer prognosis, whereas lower differences may indicate an early stage of the disease with a better prognosis.

Specifically, when the analyzed polypeptide is MYOF, CTNS, FCGR2B, PCDHGC5, POMGNT2, ACSL1, CTAGE5. ADRB1, TECPR2, CASC5, CTNS, PCDHGC5, WDR48, MCPH1, PPP2R3C, ADRB1, JAG2, GEMIN7, PTPRB, PRMT9, Ube2L3, TP53RK, PSME3, a statistically significant increase in its amount and/or activity above a predetermined level is indicative of lung cancer.

When the analyzed polypeptide is CDH5, PAPDC2, AGER, GYPA, CAV1, PPAPDC2 and MAGEE1, a statistically significant decrease in its amount and/or activity above a predetermined level is indicative of lung cancer.

The patient sample typically comprises cells. It may be part of a tissue sample, retrieved during a biopsy. Alternatively, the sample may be a bodily fluid, e.g. blood, urine, saliva, CSF, plasma etc.

For diagnosis of cancer, the cell sample may comprise cells of the primary tumor and/or metastatic effusion thereof.

The predetermined level may be established based on results from control (non-diseased) cells.

The control cell sample typically depends on the patient sample being analyzed. Thus, for example, in the case of lung cancer, the control sample may comprise lung cells of a healthy individual (or at least one not suffering from lung cancer) or from a known stage of lung cancer (e.g. non-metastatic stage).

The control cells are typically normally differentiated cells, preferably of the same tissue and specimen as the tested cells. Typically, the amount of change in expression of the polypeptides is statistically significant.

Preferably, the difference is at least 10 %, 20 %, 30 %, 40 %, 50 %, 80 %, 100 % (i.e., twofold), 3 fold, 5 fold or 10 fold different as compared to the control cells.

It will be appreciated that the control data may also be taken from databases and literature.

On obtaining the results of the analysis, the subject is typically informed. Additional diagnostic tests may also be performed so as to corroborate the results of the diagnosing (e.g. gold standard tests, assessing the aggressiveness of the tumor, the patient's health and susceptibility to treatment, etc.).

Imaging studies such as CT and/or MRI may be obtained to further diagnose the disease.

In addition, the diagnosis or choice of therapy may be determined by further assessing the size of the tumor, or the lymph node stage or both, optionally together or in combination with other risk factors.

As described in the Examples section herein below, PSME4 is the most anti-correlated gene to the immunoproteasome subunits and most particularly to PSMB10. Thus, according to still another aspect of the present disclosure there is provided a method of diagnosing cancer in a subject comprising analyzing amount and/or activity of PSME4 (GeneID 23198) and at least one immunoproteasome catalytic subunit present in a sample of the subject, wherein an increase in the ratio of said PSME4: said at least one immunoproteasome catalytic subunit as compared to said ratio in a sample derived from a non-diseased subject is indicative of the cancer, wherein said at least one immunoproteasome catalytic subunit is selected from the group consisting of PSMB8 (GeneID 5696), PSMB9 (GeneID 5698) and PSMB10 (GeneID 5699).

Exemplary samples that may be analyzed include a tumor sample itself (e.g. from a biopsy) or a liquid sample of the subject such as blood, serum, plasma, urine or CSF.

Methods of analyzing the expression of the immunoproteasome catalytic subunits may be carried out as described for other targets mentioned herein above.

In one aspect, when the ratio of PSME4:PSMB10 is greater than 2:1, it is indicative of cancer.

More specifically, when the ratio of PSME4: PSMB10 is greater than at least 3:1, it is indicative of cancer.

More specifically, when the ratio of PSME4: PSMB10 is greater than at least 4:1, it is indicative of cancer.

In one aspect, when the ratio of PSME4:PSMB10 in the tested cells is at least twice or three times the ratio in a sample (preferably derived from the subject, known not to be cancerous), it is indicative of cancer.

Particular cancers that are relevant for this method of diagnosis include lung cancer such as non-small cell lung cancer (NSCLC) and specifically, Lung Adenocarcinoma, stomach adenocarcinoma and colon adenocarcinoma.

According to a particular aspect, the cancer is not melanoma.

The subject may be treated according to the results of the diagnosis using a medication known to be effective for treating lung cancer (as detailed herein above).

The present inventors propose that the ratio of PSME4: at least one immunoproteasome catalytic subunit (e.g. PSMB10) may also be used to select a suitable therapy for the treatment of cancer e.g. personalized medicine.

In another aspect, when the ratio of the amount of PSME4: at least one immunoproteasome catalytic subunit is below a predetermined threshold, it is indicative that the patient is suitable for immunotherapy treatment e.g. durvalamab.

Thus, for example, when the ratio of PSME4: PSMB10 is greater than at least 2:1, the patient is deemed suitable for immunotherapy treatment.

More specifically, when the ratio of PSME4: PSMB10 is greater than at least 3:1, the patient is deemed suitable for immunotherapy treatment.

More specifically, when the ratio of PSME4: PSMB10 is greater than at least 4:1, the patient is deemed suitable for immunotherapy treatment.

An example of an immunotherapy treatment includes an immune checkpoint inhibitor. As used herein, the phrase "immune checkpoint inhibitor" refers to a compound capable of inhibiting the function of an immune checkpoint protein. Inhibition includes reduction of function and full blockade. In particular the immune checkpoint protein is a human immune checkpoint protein. Thus the immune checkpoint protein inhibitor preferably is an inhibitor of a human immune checkpoint protein. Immune checkpoint proteins are described in the art (see for instance Pardoll, 2012. Nature Rev. cancer 12: 252-264). The designation immune checkpoint includes the experimental demonstration of stimulation of an antigen-receptor triggered T lymphocyte response by inhibition of the immune checkpoint protein in vitro or in vivo, e.g. mice deficient in expression of the immune checkpoint protein demonstrate enhanced antigen-specific T lymphocyte responses or signs of autoimmunity (such as disclosed in Waterhouse et al., 1995. Science 270:985-988; Nishimura et al., 1999. Immunity 11:141-151). It may also include demonstration of inhibition of antigen-receptor triggered CD4+ or CD8+ T cell responses due to deliberate stimulation of the immune checkpoint protein in vitro or in vivo (e.g. Zhu et al., 2005. Nature Immunol. 6:1245-1252).

Preferred immune checkpoint protein inhibitors are antibodies that specifically recognize immune checkpoint proteins. A number of CTLA-4, PD1, PDL-1, PD-L2, LAG-3, BTLA, B7H3, B7H4, TIM3 and KIR inhibitors are known and in analogy of these known immune checkpoint protein inhibitors, alternative immune checkpoint inhibitors may be developed in the (near) future. For example ipilimumab is a fully human CTLA-4 blocking antibody presently marketed under the name Yervoy (Bristol-Myers Squibb). A second CTLA-4 inhibitor is tremelimumab (referenced in Ribas et al, 2013, J. Clin. Oncol. 31:616-22).

Examples of PD-1 inhibitors include without limitation humanized antibodies blocking human PD-1 such as lambrolizumab (e.g. disclosed as hPD109A and its humanized derivatives h409A11, h409A16 and h409A17 in WO2008/156712; Hamid et al., N. Engl. J. Med. 369: 134-144 2013,), or pidilizumab (disclosed in Rosenblatt et al., 2011. J. Immunother. 34:409-18), as well as fully human antibodies such as nivolumab (previously known as MDX-1106 or BMS-936558, Topalian et al., 2012. N. Eng. J. Med. 366:2443-2454, disclosed in U.S. Pat. No. 8,008,449 B2). Other PD-1 inhibitors may include presentations of soluble PD-1 ligand including without limitation PD-L2 Fc fusion protein also known as B7-DC-Ig or AMP-244 (disclosed in Mkrtichyan M, et al. J Immunol. 189:2338-47 2012) and other PD-1 inhibitors presently under investigation and/or development for use in therapy.

In addition, immune checkpoint inhibitors may include without limitation humanized or fully human antibodies blocking PD-L such as MEDI-4736 (disclosed in WO2011066389 A1), MPDL3280A (disclosed in U.S. Pat. No. 8,217,149 B2) and MIH1 (Affymetrix obtainable via eBioscience (16.5983.82)) and other PD-L1 inhibitors presently under investigation. According to this disclosure an immune checkpoint inhibitor is preferably selected from a CTLA-4, PD-1 or PD-L1 inhibitor, such as selected from the known CTLA-4, PD-1 or PD-L1 inhibitors mentioned above (ipilimumab, tremelimumab, labrolizumab, nivolumab, pidilizumab, AMP-244, MEDI-4736, MPDL3280A and MIH1). Known inhibitors of these immune checkpoint proteins may be used as such or analogues may be used, in particular chimerized, humanized or human forms of antibodies.

Other agents used in the arsenal of cancer immunotherapy agents include T cell populations that are capable of binding to the peptide epitopes of the tumor for adoptive cell therapy (ACT).

ACT refers to the transfer of cells, most commonly immune-derived cells, back into the same patient or into a new recipient host with the goal of transferring the immunologic functionality and characteristics into the new host. If possible, use of autologous cells helps the recipient by minimizing GVHD issues. The adoptive transfer of autologous tumor infiltrating lymphocytes (TIL) (Besser et al., (2010) Clin. Cancer Res 16 (9) 2646-55; Dudley et al., (2002) Science 298 (5594): 850-4; and Dudley et al., (2005) Journal of Clinical Oncology 23 (10): 2346-57.) or genetically re-directed peripheral blood mononuclear cells (Johnson et al., (2009) Blood 114 (3): 535-46; and Morgan et al., (2006) Science 314(5796) 126-9) has been used to successfully treat patients with advanced solid tumors, including melanoma and colorectal carcinoma, as well as patients with CD19-expressing hematologic malignancies (Kalos et al., (2011) Science Translational Medicine 3 (95): 95ra73).In one aspect TCRs are selected for administering to a subject based on binding to neoantigens. In one aspect T cells are expanded using methods known in the art. Expanded T cells that express tumor specific TCRs may be administered back to a subject. In another aspect PBMCs are transduced or transfected with polynucleotides for expression of TCRs and administered to a subject. T cells expressing TCRs specific to neoantigens are expanded and administered back to a subject.

Other immunotherapy treatments include T cell populations expressing chimeric antibodies (CAR-T cells) on the surface thereof that can bind to at least one peptide epitope of the tumor.

### As used herein the term "about" refers to ± 10 %

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various aspects of this disclosure may be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the disclosure, which are, for clarity, described in the context of separate aspects, may also be provided in combination in a single aspect. Conversely, various features of the disclosure, which are, for brevity, described in the context of a single aspect, may also be provided separately or in any suitable subcombination or as suitable in any other described aspect of the disclosure. Certain features described in the context of various aspects are not to be considered essential features of those aspects, unless the aspect is inoperative without those elements.

Various aspects and aspects of the present disclosure as delineated hereinabove and as claimed in the claims section below find experimental support in the following examples.

### EXAMPLES

Reference is now made to the following examples, which together with the above descriptions illustrate some aspects of the disclosure in a non-limiting fashion.

Generally, the nomenclature used herein and the laboratory procedures utilized in the present disclosure include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document. The procedures therein are believed to be well known in the art and are provided for the convenience of the reader.

### GENERAL MATERIALS AND METHODS

***Cell Culture and Treatments:*** A549 cells were grown in DMEM supplemented with 10% fetal bovine serum, 1% Penicillin/streptomycin and L-glutamine (2 mM) (Biological industries) at 37 °C with 5% CO₂. Cells were treated with 400 U*mL-1 TNFα and/or 200 U*mL-1 IFNγ (peprotech) for the indicated amount of time.

***Antibodies for Western blotting, immunoprecipitation and immunohistochemistry:*** Primary antibodies to the following were used: PSMA1 (hybridoma). PANα (PSMA1-7; Enzo, BML-PW8195-0025), PSME4(Millipore Sigma HPA060922 and Proteintech 18799-1-AP), LMP7(Cell signaling 13635), PSMB10 (Thermo Fisher PA519146) and β - Actin (Abcam ab170325). The following secondary antibodies were used: goat anti-mouse HRP (115-035-003) and goat anti-rabbit HRP (111-035-003) (Jackson Labs).

***Purification of proteasome complexes:*** Tumors were mechanically disrupted and passed through a mesh. Cells were lysed with 25 mM HEPES, pH 7.4, 10 % glycerol, 5 mM MgCl₂, 1 mM ATP, and 1:400 protease-inhibitor mixture (Calbiochem), then homogenized through freeze-thaw cycles and passed through a 25-gauge needle. The lysates were cleared by 30-min centrifugation at 21,130g at 4 °C. Lysates were treated with 1 mM PMSF (Sigma) and 2 mM 1,10-phenanthroline (Sigma), cross-linked with 0.5 mM DSP (Thermo Fisher) for 30 minutes at room temperature, and quenched in 20 mM Tris-HCl, pH 7.4, 5 mM L-cysteine for 10 minutes at room temperature. For immunoprecipitation, the lysates were then incubated with Protein G-Sepharose beads (Santa Cruz) with antibodies to PSMA1 and eluted with 50 mM DTT for 30 min at 37 °C. Subsequently, 0.5 % trifluoroacetic acid (TFA) was added. Aliquots of each elution fraction were analyzed by SDS-PAGE to evaluate yield and purity.

***Purification and concentration of proteasome peptides:*** A critical step in this procedure is the separation of peptides from the proteins eluted in the proteasomal pulldown. MAPP analyzes endogenously cleaved peptides, whereas the proteasome complex and associated proteins are physically excluded. Immunoprecipitated proteasomes and their encompassed peptides were loaded on tC18 cartridges (Waters) that were prewashed with 80 % acetonitrile (ACN) in 0.1 % TFA, then washed with 0.1 % TFA only. After loading, the cartridges were washed with 0.1 % TFA. Peptides were eluted with 30 % ACN in 0.1 % TFA. Protein fractions were eluted with 80 % ACN in 0.1 % TFA.

***Liquid chromatography mass spectrometry:*** ULC/MS grade solvents were used for all chromatographic steps. Each sample was loaded using split-less nano-Ultra Performance Liquid Chromatography (10 kpsi nanoAcquity; Waters, Milford, MA, USA). Peptides were reconstituted in 97:3 H₂O:ACN+0.1% formic acid. The mobile phase was: A) H₂O + 0.1% formic acid and B) acetonitrile + 0.1% formic acid. Desalting of the samples was performed online using a reversed-phase C18 trapping column (180 µm internal diameter, 20 mm length, 5 µm particle size; Waters).

***MAPP samples:*** MAPP peptides were solubilized in 97:3 H₂O:ACN then separated using a HSS T3 nano-column (75 µm internal diameter, 250 mm length, 80A pores), in cases where we observed residual intact proteins, such as in samples treated with epoxomicin, a 300A column was used. Peptides were separated using a 120 min gradient. The nanoUPLC was coupled online through a nanoESI emitter (10 µm tip; New Objective; Woburn, MA, USA) to a quadrupole Orbitrap mass spectrometer (Q Exactive Plus, Thermo Scientific, USA) using a FlexIon nanospray apparatus (Proxeon). Data was acquired in positive ionization, data dependent acquisition mode, using a Top10 method. For the ZsGreen experiment, the samples were analyzed in parallel reaction monitoring (PRM) mode using the Q Exactive mass spectrometer, targeting 11 molecular ions representing the ZsGreen peptides, of which 4 produced quantifiable signal. Raw data was then imported into Skyline(1-3) for extraction of fragment ion chromatograms and area under the curve calculation.

***Tryptic digest of whole cell lysates:*** Cell pellets were subjected to in-solution tryptic digestion using a modified Filter Aided Sample Preparation protocol FASP or S-trap. All chemicals are from Sigma Aldrich, unless stated otherwise. Reduction was performed using dithiothreitol and alkylation with iodoacetamide. Digestion of proteins was performed using trypsin at 37 °C overnight. Additional amount of trypsin was added and incubated for 4 hours at 37 °C. Digested proteins were then spun down to a clean collecting tube, 50 µl NaCl 0.5 M was added and spun down, acidified with trifloroacetic acid, desalted using HBL Oasis, vacuum centrifuged to dry and stored in -80°C until analysis. Resulting peptides were then separated using a HSS T3 nano-column (75 µm internal diameter, 250 mm length, 1.8 µm particle size, 80A pores; Waters) at 0.35 µL/minutes. Peptides were eluted from the column into the mass spectrometer using a 155min gradient. The nanoUPLC was coupled online through a nanoESI emitter (10 µm tip; New Objective; Woburn, MA, USA) to the a quadrupole Orbitrap mass spectrometer (Q Exactive Plus or HF, Thermo Scientific, USA) using a FlexIon nanospray apparatus (Proxeon). Data was acquired in positive ionization, data dependent acquisition mode, using a Top10 method.

***Protein eluate:*** Proteins eluted from the StageTips were subjected to in-solution tryptic digestion. All chemicals are from Sigma Aldrich, unless stated otherwise. Reduction was performed using dithiothreitol and alkylation with iodoacetamide. Digestion of proteins was performed using trypsin at 37 °C overnight. Digested proteins were then spun down to a clean collecting tube, 50 µl NaCl 0.5M was added and spun down, acidified with trifloroacetic acid, desalted using HBL Oasis, vacuum centrifuged to dry and stored in -80°C until analysis. Resulting peptides were then separated using a HSS T3 nano-column (75 µm internal diameter, 250 mm length, 1.8 µm particle size, 80A pores; Waters) at 0.35 µL/minutes. Peptides were eluted from the column into the mass spectrometer using a 50min gradient. The nanoUPLC was coupled online through a nanoESI emitter (10 µm tip; New Objective; Woburn, MA, USA) to a quadrupole Orbitrap mass spectrometer (Q Exactive Plus or HF, Thermo Scientific, USA) using a FlexIon nanospray apparatus (Proxeon). Data was acquired in positive ionization, data dependent acquisition mode, using a Top10 method.

***Mass spectrometry data analysis:*** Raw data were analyzed in MaxQuant software (version 1.6.0.16) with the default parameters for the analysis of the proteasomal peptides, except for the following: unspecific enzyme, LFQ minimum ratio count of 1, minimum peptide length for unspecific search of 6, maximum peptide length for unspecific search of 40, and match between runs enabled. A stringent false discovery rate (FDR) of 1% was applied for peptide identification (in accordance with the FDR reported in a previous peptidomics study). For the analysis of tryptic digests, the default parameters were set, apart from a minimum peptide length of 6. Masses were searched against the human proteome database from UniprotKB (last update September 2019).

***Bioinformatics analysis and label-free quantification:*** Peptides resulting from MaxQuant were initially filtered to remove reverse sequences and known MS contaminants. To decrease ambiguity, we allowed peptides that had at least two valid LFQ intensities out of three independent biological replicates, and we included razor peptides, which belong to a unique MaxQuant 'protein group'. MAPP protein intensities were inferred with MaxQuant. For graphical representation, intensities were log transformed, and in Python v3.6, zero intensity was imputed to a random value chosen from a normal distribution of 0.3 s.d. and downshifted 1.8 s.d. Statistical analyses were performed in R v 3.6.2 and GraphPad Prism v 7.04. Protein annotation and gene ontology analysis were performed with the PANTHER classification system v 10.0. gene-set enrichment analysis, GeneAnalytics, Corum and AmiGo 2 v 2.4.26. Protein Network visualization was performed in Cytoscape v 3.4.0. TCGA data was mined using the xenaPython package in Python 3.6. The results shown in this analysis are in whole or part based upon data generated by the TCGA Research Network. The full lung carcinoma cohort (both lung adenocarcinoma [LUAD] and lung squamous cell carcinoma [LUSC] designations) was used for this study unless indicated otherwise.

***Proteasome Cleavage Reporter Assay:*** A549 cells, stimulated with TNFα and IFNγ for the indicated times, were collected and flash frozen. Frozen cells were re-suspended in lysis buffer (25 mM sucrose, 50 mM TRIS pH 7.4, 5 mM MgCl₂, 0.5 mM EDTA, 2 mM ATP, 1 mM DTT). Lysates were passed 10 times through a 27 G needle, incubated on ice for 15 min and the centrifuged at 21130rcf for 10 min. Protein concentrations were measured using the Coomassie Plus (Bradford) Assay Kit (Pierce, ThermoFisher Scientific). 20 µg of cellular lysate was incubated with 0.1 mM suc-LLVY-AMC (SEQ ID NO: 1), ac-PAL-AMC (SEQ ID NO: 2), Z-LLE-AMC (SEQ ID NO: 3) (Biotest), ac-NPND-AMC (SEQ ID NO: 4) and Z-LLE-βNA (SEQ ID NO: 5) (bachem) as per protocol and fluorescence levels were measured over time using a BioTek Synergy H1 plate reader (Ex: 360 nm, Em: 460 nm). The background protease activity was determined for each condition from an identically prepared sample with the addition of Mg132 proteasome inhibitor (0.04 mM; Calbiochem). Each time point measurement was performed in three independent biological replicates.

### EXAMPLE 1

Eight tumor biopsies of NSCLC patients with their matching adjacent lung tissue from the Midgam biobank were analyzed. The standard MAPP protocol (see WO2017/158610) was adapted to solid tumors by first passing the biopsies through a mesh, creating a single cell suspension. In parallel, a bottom-up tryptic proteomics was performed from whole cell extract in order to assess the abundance of proteins in the tumors as well as their degradation profiles (Fig. 1).

Principal component analysis (PCA) based both on the WCE proteomics and the MAPP uncovered that the degradome almost entirely separated samples based on whether they were from the adjacent or tumor tissue (Figs. 2A-B). This indicates that proteins expressed and degraded in the cell are sufficiently different between adjacent and tumor tissue to classify samples. Indeed, unsupervised clustering using Pearson correlation classifies all of the data according to the samples type (e.g. tumor and adjacent) except for one control sample which was assigned as a tumor. Next, the present inventors sought to analyze the degradation profile for each patient by comparing its tumor-identified degradome to the adjacent control.

This generated a conserved degradation signature that categorizes the tumor and adjacent tissue. Intriguingly, 163 proteins were found that were significantly degraded more in the tumor samples and 46 proteins that were significantly more degraded in the adjacent controls (Fig. 3A). This common signature can also be observed in a patient-specific view (Fig. 3B) where the ratio of protein intensity (rows) between the tumor and adjacent controls were calculated for each patient (columns). The present inventors then examined the interactions between these proteins, highlighting a cluster of differentially degraded proteins involved in metabolic pathways, and another in lipid raft organization (Fig. 4). The differential degradation of components of these pathways in the context of NSCLC suggests a potential role for the proteasome in regulating the tumor environment.

After highlighting proteins which were differentially degraded in the tumor and adjacent tissues, the present inventors then set out to identify targets which are specific to the tumor or adjacent tissue. These would potentially represent proteins which could be used for targeted immunotherapies. To accomplish this, they ranked proteins by the number of tumor samples they were degraded in minus the number of adjacent samples (Figures 5A-B). They then selected proteins which were detected in at least 4 more tumor samples (orange box) or adjacent tissues (blue box). These selectively degraded proteins are potentially highly tumor specific at the level of their degradation. However, to then ascertain their protein and mRNA expression, the present inventors examined their levels in the WCE proteomics data and TCGA Lung Adenocarcinoma (LUAD) datasets (Figures 6A-C).

When compared to the WCE proteomics, the majority of proteins that were degraded in more tumor samples were also expressed more highly in the tumor. Nevertheless, these were not the most differentially expressed proteins in the WCE proteomics and only through the lens of MAPP were they able to identify them as differentially expressed. Furthermore, there were some proteins that were entirely undetected in WCE proteomics, such as the potentially antigenic target CASC5. Without the MAPP analysis it would not have been identified. Aside from the protein expression levels, the mRNA expression was also ascertained across the entire TCGA LUAD cohort. With the exception of one gene/protein, all of the other genes were identified in the TCGA.

CASC5, WDR48, MCPH1, TECPR2, PPP2R3C and CTNS are all selectively degraded in the tumor tissue but not detected in either tumor or adjacent tissue by WCE proteomics. The present inventors then examined the Immune Epitope Database and Analysis Resource (IEDB), a repository of experiments profiling peptides presented on MHC to see if these proteins are known to be presented (Fig. 7A). There was at least one presented peptide identified from each protein. In the case of CASC5, there are 75 different known presented epitopes. In addition, using the Xena package, the present inventors assessed whether there are identified mutations in any of the proteins in the TCGA LUAD cohort. Indeed, in 4 of the proteins, several mutations have been identified (Fig. 7B). Finally, again using the Xena python package to access the LUAD cohort, the odds ratio for each gene was calculated. This ratio represents whether the expression of the gene has a significant impact on survival (Fig. 8A, bars represent 95% confidence intervals, all genes presented had an odds ratio with a pvalue <=0.1). The impact of gene expression on survival is also displayed as Kaplan-Meier curves for DENR, CASC5 and AGER (Fig. 8B).

To identify novel targets which may be involved in NSCLC, the data was divided into several categories (Table 2).

**Table 2**

| ***Membrane -bound targets (e.g. blocking antibody, CAR-T) Supplememention, enhancment of expression*** | | | | | |
|---|---|---|---|---|---|
| Up-regulated in tumor | | | Down-regulated in tumor | | |
| Protein | FC | pVal | Protein | FC | pVal |
| MYOF | 8.08 | 0.03 | CDH5 | -8.08 | 0.00 |
| CTNS | 7.75 | 0.05 | PPAPDC2 | -8.22 | 0.01 |
| FCGR2B | 7.56 | 0.09 | AGER | -9.22 | 0.00 |
| PCDHGC5 | 7.22 | 0.01 | GYPA | -9.91 | 0.01 |
| POMGNT2 | 7.16 | 0.23 | CAV1 | -10.38 | 0.01 |
| ACSL1 | 6.90 | 0.20 | | | |
| CTAGE5 | 4.51 | 0.04 | | | |
| ADRB1 | 3.94 | 0.04 | | | |
| | | | | | |

| ***Appear only in MAPP (e.g. anti-cancer vaccine)*** | | | | | |
|---|---|---|---|---|---|
| Up-regulated in tumor | | | Down-regulated in tumor | | |
| Protein | FC | pVal | Protein | FC | pVal |
| TECPR2 | 9.02 | 0.04 | PPAPDC2 | -8.22 | 0.01 |
| CASC5 | 8.02 | 0.00 | MAGEE1 | -11.23 | 0.02 |
| CTNS | 7.75 | 0.05 | | | |
| PCDHGC5 | 7.22 | 0.01 | | | |
| WDR48 | 7.12 | 0.01 | | | |
| MCPH1 | 6.16 | 0.01 | | | |
| PPP2R3C | 6.02 | 0.01 | | | |
| ADRB1 | 3.94 | 0.04 | | | |
| JAG2 | 3.30 | 0.04 | | | |
| GEMIN7 | 2.87 | 0.04 | | | |
| PTPRB | 2.47 | 0.04 | | | |
| PRMT9 | 1.97 | 0.05 | | | |
| | | | | | |

| ***Additional interesting targets*** | | | | | |
|---|---|---|---|---|---|
| PSME4 - may affect HLA presentation, infiltration, metabolism, DNA damage response and cell cycle | | | | | |
| Ube2L3 - ubiquitin E2 conjugating enzyme enzyme inhibition | | | | | |
| TP53RK - kinase; enzyme inhbition | | | | | |
| PSME3 - PA28g | | | | | |

1) Proteins that were detected by MAPP and not by whole-cell extract proteomic analysis of the same samples. These proteins were separated based on their signal in MAPP, more degraded in the tumor or less. Proteins that were more degraded in the tumor and are upregulated in mRNA expression compared to normal controls (Fig. 6A-C; right panel) will be further examined for their potential utilization as anti-cancer vaccines (peptides, RNA delivery, etc.) as proteasome degradation serves as an upstream event to MHC presentation. 2) Transmembrane proteins that are differentially regulated may serve for targeting tumor cells by blocking antibodies or T-cell therapies. Specific targets that were down-regulated only in the cancer may be examined for the effect of supplementation on NSCLC growth rates (e.g. by changing miRNA, enhancing expression, protein supplementation). Likewise, certain classes of drugs may augment the function of the under expressed protein, thereby impeding tumorigenesis. A third group describes proteins of functional interest for their enzymatic influence on the protein environment. There are three proteins of the ubiquitin proteasome system that were detected as overexpressed in tumors - two subunits that were most differentially expressed among proteasome subunits and an E2 ubiquitin ligase UBE2L3. Finally, increased degradation of TP53RK - a critical modulator of the tumor suppressor TP53 was also identified.

Given the changes on the substrates targeted for degradation in the tumor and adjacent tissue, the present inventors wished to examine if there were any accompanying changes in the ubiquitin proteasome system which would explain the altered degradation. It was first noted that across all of the patients, there is a significant increase in the number of peptides identified in the tumor degradomes as compared to the degradome of the adjacent tissue (Figs. 9A-B, p = 0.016). It was then posited that this is due to increased numbers of proteasomes in the tumors. To examine this, the expression of proteasomes in the WCE proteomics across the samples was analyzed. Indeed, it was observed that most of the tumor samples had higher expression across all of the different proteasome subunits (Fig. 10). Specifically a significant increase in subunits of the 20S proteasome was observed (Fig. 11; PSMA1, PSMA6). This was not, however, true for the catalytic subunits of the immunoproteasome (Fig. 11; PSMB8-10). Surprisingly however, the subunits which most significantly increased were the proteasome regulators PSME3 (PA28γ) and PSME4 (PA200).

PSME4 is most highly expressed in testes tissue and has been associated with spermatogenesis, DNA damage repair and degradation of oxidized proteins. Using DepMap based on data from the Achilles project, it was found that Lung NSC and Adenocarcinoma in particular, are more dependent on PSME4 than other cancers (Fig. 12). This was determined through assessment of viability following knockdown of PSME4 expression.

In order to determine what the proteasome expression is across a wider subset of lung cancers, the present inventors examined the level of mRNA expression across the TCGA lung adenocarcinoma (LUAD) cohort (n-=1128 patients). There, different levels of PSME4 at mRNA level were found, suggesting that there might be variability in different tumors and expression levels of PSME4. Nevertheless, unsupervised clustering of the samples and proteasome subunits revealed different proteasome composition across the cohort (Fig. 13). In order to see how the different proteasome compositions related to one another, the Pearson correlation was calculated pairwise across the expression of all of the different proteasome subunits (Fig. 14). As can be seen there is highly correlated expression of the 3 immunoproteasome catalytic subunits (PSMB8-10) and the PA28αβ cap (PSME1-2). Conversely, PSME4 is the most anti-correlated gene to the immunoproteasome subunits (PSME4 vs PSMB10 rho = -0.44). This indicates that, across the LUAD cohort, tumors will either express immunoproteasomes of PSME4 capped proteasomes, but when expression of one increases the other decreases.

The present inventors then determined whether tumors with high expression of PSME4 would have a different expression profile than those with low expression. They therefore stratified the LUAD cohort by PSME4 expression, taking the top and bottom 20% (n = 115 samples each). Of these, over 10,000 genes were differentially expressed between the two subsets (Figure 15). Using Gene Set Enrichment Analysis (GSEA), the inventors examined which pathways the differently expressed genes belong to. Using the reactome and biocarta annotations sets, pathways were found that were specifically upregulated in the tumors with high PSME4 or low PSME4 expression. The high expressers had increased cell cycle effect as well as altered chromosome maintenance. Tumors with low PSME4 expression had increased inflammation pathways, NKT as well as increases in the complement cascade and GPCR signaling (Fig. 15).

Finally, since it was previously shown that PSME4 alters proteasome cleavage, increasing the post glutamyl proteasome activity, the present inventors wanted to ascertain if they also observed an altered cleavage motif in the tumor samples. Indeed, comparing the percentage of peptides with different carboxy-terminus residues in the tumor and adjacent tissues, they observed a significant increase in the percentage of peptides that ended in aspartic or glutamic acid (D or E; Figure 16). This was accompanied by significant changes in other terminal residues that were not previously reported to be associated with PSME4. Nevertheless, in the case of post-glutamyl activity, a high correlation between the abundance of PSME4 in the samples (as determined from the WCE proteomics) and the percent of peptides that ended in D or E in the sample (Figure 17) was found. In accordance with the changes observed in degradation of proteins involved in metabolic pathways, this change in cleavage may represent the tumor cells adapting to altered metabolism by altering amino acid recycling. Further, the post-glutamyl activity increase associated with PSME4 was only shown previously in an *in-vitro* context and the present inventors are the first to show that it holds true in the context of human lung cancer samples. Thus, PSME4 levels may be modulated to affect the immunopeptidome. As such, it may have broader implications in the context of other cancers and responsivity to immunotherapy.

Given that the most dominant DNA damaging agent involved in Lung Cancer is smoking, we examined if there is a significant correlation between patients who smoked and expression of PSME4 in the tumor. Using the TCGA LUAD dataset, the cohort was divided based on their smoking history. There was a significant increase in expression of PSME4 in those patients which smoked versus those who were non-smokers [FIGs. 18A-C; p < .0001]. Likewise, when examining those who have stopped smoking, the time since smoking significantly affected PSME4 levels [2 way anova, p = 8.18E-06]. Because this connection may be due to mutations that accumulated as a result of smoking, the present inventors then examined whether tumors with more mutations had higher expression of PSME4. Indeed, when dividing the LUAD cohort into tumors with high or low tumor mutational burden, PSME4 was significantly higher in the group with high TMB [Figures 18A-C]. Finally, expression of PSME4 was compared to a genetic signature of DNA repair, finding that increased expression of PSME4 in the tumor correlated highly with an increase in DNA repair [rho = 0.65].

In order to understand if the increase in PSME4 was a phenomenon specific to smoking induced lung cancer, the expression of PSME4 was examined across all the cancer types that had corresponding normal tissue [Figure 19B]. While in LUAD there was an increase in PSME4 in the tumor tissue, other cancer types, such as melanoma (SKCM) did not display the same trend. By contrast, if examining the immunoproteasome subunit PSMB10 [Figure 19A] there is no change in LUAD but a change between normal and tumor samples in SKCM.

Hypothesizing that there is an interplay between different proteasome compositions across cancer, the present inventors took the TCGA LUNG cohort and calculated the correlation in expression pairwise across all of the proteasome subunits. Strikingly, the PSME4 subunit was the most anticorrelated to the 3 immunoproteasome catalytic subunits and 2 immunoproteasome regulatory caps (PSMB8-10, PSME1+2) [Figure 20A]. Furthermore, out of all the genes expressed in the cancer cohort, not just those of the proteasome, PSME4 is one of the most anti correlated to PSMB10 (2.3 SD from the mean correlation). To examine this connection more functionally, splenocytes were taken from mice that are deficient in immunoproteasomes (LMP2 KO). Following stimulation of immunoproteasome expression with TNFα and IFNγ, it was seen that in the LMP2 KO, PSME4 increased more as compared to the WT mice. This suggests that immunoproteasome deficiency indeed leads to higher PSME4 expression, suggesting a balance between them.

In order to understand whether this balance has functional consequences, the TCGA LUAD database was divided into high and low PSME4 expressers, and subsequently GSEA pathway analysis was performed. Tumors with high PSME4 are more proliferative and have increase in pyrimidine metabolism. By contrast, low expressers have more inflammation and immune signaling [Figures 21A-B]. Thus, it was hypothesized that the balance between immunoproteasome and PSME4 is involved in the balance between tumor proliferation and inflammation/infiltration.

Given the effect PSME4 has on tumor growth and proliferation, the present inventors next assessed whether this impacts sensitivity to chemotherapy. Using the Cell miner database of IC50 of all FDA approved drugs across the NCI60 cell lines, the cell lines were divided into those with high and low PSME4 expression. Those with high PSME4 expression were more sensitive to 3 different drugs all of which disrupt the cell cycle through affecting microtubule formation [Figure 22].

In order to determine whether the level of PSME4 affects degradation of substrates differently, the degradation of every protein identified in MAPP was correlated with the abundance of PSME4 across the NSCLC cohort. Only those proteins whose degradation was significantly more correlated to PSME4 than any of the 20S or 19S subunits were selected [Figure 23]. These PSME4 enriched substrates include ATIC, a protein responsible for catalyzing 2 of the steps of the purine synthesis pathway, and several proteins involved in cell proliferation or histone organization.

To examine how differential degradation of the PSME4 substrates would affect cellular response to therapy, the connectivity map (CMAP) data was used. The dataset contains the IC50 of all FDA approved drugs across cells that have been knocked out for a large panel of human genes. It can be seen that when the genes in the signature are knocked out, the cells are desensitized to many drugs which cause DNA damage [Figure 24]. This indicates that PSME4 mediated degradation may aid in the cellular response to DNA damage.

To follow up on the role of PSME4 in tumor metabolism, a metabolite analysis performed on all the NCI60 cell lines (Ortmayr, K. et al (2019). Nature Communications*)* was examined. When comparing melanoma cell lines to lung cancer, it was found that melanoma had significant increases in many purine metabolites, while lung cancer had increased pyrimidine synthesis [Figure 25]. Furthermore, when examining all the NCI60, dividing them into cell lines with high or low PSME4 expression. Consistent with the transcriptome analysis of TCGA, pyrimidine metabolites were also increased in cell lines with high PSME4 expression.

When purine synthesis was blocked in A549 cells using mizoribine, a decrease in PSME4 expression was noted [Figure 26A-B].

Given the way PSME4 effects tumor inflammation and progression, responsiveness to immunotherapy was also examined. However, given that the proteasome functions as a complex, the expression of PSME4 in the context of the expression of PSMB10, (the immunoproteasome subunit which is most anti-correlated to PSMB10) was examined. Looking at 4 patients who responded to durvalamab therapy, and 4 non-responders, the expression of PSME4 and PSMB10 was examined using qPCR. As illustrated in Figure 27A-C, there is a significantly higher ratio of PSME4/PSMB 10 expression in patients who did not respond to the immunotherapy. This suggests the effects that PSME4 has on tumor inflammation and peptide processing (propagate to decreased responsiveness to immunotherapy).

The present inventors also examined the increase in PSME4 between tumor and normal tissue across cancer types in relationship to the PSMB10 ratio. LUAD, LUSC, STAD and COAD all increased in PSME4 compared to normal tissue from the same site, but decreased in PSMB10 [Figure 29].

In order to understand if peptides generated from the PSME4 protein itself are presented the IEDB database was examined and 87 peptides were found to present PSME4 [Figure 30].

### EXAMPLE 2

Analysis of serum from patients with lung adenocarcinoma or from healthy individuals showed that the patients with lung cancer have higher levels of PSME4. (Figure 31A) From tumor and adjacent tissues that were previously analyzed with MAPP, the amount of PSME4 was analyzed. It was found that the tumor expressed a significantly higher level of PSME4 (PA200) when compared to the adjacent control per patient (Figure 31B).

PSME4 (PA200) and PSMB10 (immunoproteasome) were immunostained in the same samples of responders and non-responders which were analyzed for Figures 27A-C, PSME4 and PSMB10 (immunoproteasome). The levels of each were scored in both the lymphocytes and epithelial cells. Representative images of the responders and non-responders with the PSME4 and PSMB10 staining are illustrated in Figure 32A. A heatmap of the scoring, and the ratio between PSME4 and PSMB10 from each tumor is illustrated in Figure 32B. The average ratio of the non-responders was found to be significantly higher than the responders (2-way ANOVA p = 0.0088) (Figure 32C).

Figure 33A-D illustrates that the increase in proteasome cleavage is induced by inflammatory cytokines TNFα and IFNγ. The effect is partially reduced when recombinant PSME4 is introduced.

Figures 34A-D illustrates that the reduction of activity caused by PSME4 occurs for only certain proteasome activities, namely chymotryptic cleavage (reported by suc-LLVY-AMC (SEQ ID NO: 1) peptide). However, addition of recombinant PSME4 induces the activity of the constitutive proteasome caspase like activity (NPND-AMC (SEQ ID NO: 4) + LLE-*β*NA (SEQ ID NO: 5)). This confirms our observations in Figure 16 that the samples with higher levels of PSME4 (namely the tumor samples) had increased D,E and N amino acid termini, produces by the caspase like activity of the proteasome. Peptides with negative c termini (like D and E) cannot bind to MHC molecules and therefore will not be presented effectively to the immune system CD8 T cells.

## Claims

1. A method of diagnosing non-small cell lung cancer in a subject comprising analyzing an amount and/or an activity of PSME4 and at least one immunoproteasome catalytic subunit selected from the group consisting of PSMB8, PSMB9 and PSMB10 present in a non-small cell lung cancer tumor sample of said subject, wherein an increase in a ratio of amount and/or activity of said PSME4 to amount and/or activity of said at least one immunoproteasome catalytic subunit as compared to said ratio in a non-tumor sample is indicative of said non-small cell lung cancer.

2. The method of claim 1, wherein said at least one immunoproteasome catalytic unit is PSMB8.

3. The method of claim 1, wherein said at least one immunoproteasome catalytic unit is PSMB10.

4. A method of selecting a treatment for a subject diagnosed with a cancer, the method comprising determining an amount of PSME4 protein and an amount of at least one immunoproteasome catalytic subunit selected from the group consisting of PSMB8, PSMB9, and PSMB10 present in cancer cells of said subject, wherein a ratio of amount of said PSME4 protein to amount of said at least one immunoproteasome catalytic subunit being below a predetermined threshold is indicative of suitability of said subject to treatment with an immune checkpoint inhibitor.

5. The method of claim 4, wherein said cancer is selected from the group consisting of colon adenocarcinoma, NSCLC and stomach adenocarcinoma.

6. The method of claim 1, wherein said non-small cell lung cancer tumor is selected from the group consisting of a primary tumor, a secondary tumor, a recurrent tumor, a refractory tumor and a combination thereof.

7. The method of claim 6, wherein said primary tumor is selected from the group consisting of a squamous cell carcinoma, an adenocarcinoma, a large cell carcinoma and a combination thereof.

8. The method of claim 6, wherein said secondary tumor is a metastatic tumor.

9. The method of claim 1, wherein a ratio of amount and/or activity of said PSME4 to amount and/or activity of said at least one immunoproteasome catalytic subunit greater than at least 2:1 is indicative of said non-small cell lung cancer.

10. The method of claim 1, wherein a ratio of amount and/or activity of said PSME4 to amount and/or activity of said at least one immunoproteasome catalytic subunit greater than at least 3:1 is indicative of said non-small cell lung cancer

11. The method of claim 1, wherein a ratio of amount and/or activity of said PSME4 to amount and/or activity of said at least one immunoproteasome catalytic subunit greater than at least 4:1 is indicative of non-small cell lung cancer.

12. The method of claim 4, wherein a ratio of amount of said PSME4 protein to amount of said at least one immunoproteasome catalytic subunit greater than at least 2:1 is indicative of suitability of said subject to treatment with said immune checkpoint inhibitor.

13. The method of claim 4, wherein a ratio of amount of said PSME4 protein to amount of said at least one immunoproteasome catalytic subunit greater than at least 3:1 is indicative of suitability of said subject to treatment with said immune checkpoint inhibitor.

14. The method of claim 4, wherein a ratio of amount of said PSME4 protein to amount of said at least one immunoproteasome catalytic subunit greater than at least 4:1 is indicative of suitability of said subject to treatment with said immune checkpoint inhibitor.

## Patentansprüche

1. Verfahren zum Diagnostizieren von nicht kleinzelligem Lungenkrebs bei einem Probanden, umfassend die Analyse einer Menge und/oder einer Aktivität von PSME4 und mindestens einer immunoproteasomalen katalytischen Untereinheit, ausgewählt aus der Gruppe, bestehend aus PSMB8, PSMB9 und PSMB10, die in einer Tumorprobe des nicht kleinzelligen Lungenkrebses des genannten Probanden vorhanden sind, wobei ein Anstieg des Verhältnisses der Menge und/oder Aktivität des genannten PSME4 zur Menge und/oder Aktivität der genannten mindestens einen immunoproteasomalen katalytischen Untereinheit im Vergleich zu dem genannten Verhältnis in einer Nicht-Tumorprobe den genannten nicht kleinzelligen Lungenkrebs anzeigt.

2. Verfahren nach Anspruch 1, wobei die genannte mindestens eine immunoproteasomale katalytische Einheit PSMB8 ist.

3. Verfahren nach Anspruch 1, wobei die genannte mindestens eine immunoproteasomale katalytische Einheit PSMB10 ist.

4. Verfahren zum Auswählen einer Behandlung für einen Probanden, bei dem Krebs diagnostiziert wurde, wobei das Verfahren das Bestimmen einer Menge an PSME4-Protein und einer Menge an mindestens einer immunoproteasomalen katalytischen Untereinheit umfasst, ausgewählt aus der Gruppe, bestehend aus PSMB8, PSMB9 und PSMB10, die in Krebszellen des genannten Probanden vorhanden sind, wobei ein Verhältnis der Menge des genannten PSME4-Proteins zur Menge der genannten mindestens einen immunoproteasomalen katalytischen Untereinheit, das unter einer vorbestimmten Schwelle liegt, die Eignung des genannten Probanden für eine Behandlung mit einem Immun-Checkpoint-Inhibitor anzeigt.

5. Verfahren nach Anspruch 4, wobei der genannte Krebs ausgewählt ist aus der Gruppe, bestehend aus Kolonadenokarzinom, NSCLC und Magenadenokarzinom.

6. Verfahren nach Anspruch 1, wobei der genannte nicht kleinzellige Lungenkrebstumor ausgewählt ist aus der Gruppe, bestehend aus einem Primärtumor, einem Sekundärtumor, einem rezidivierenden Tumor, einem refraktären Tumor und einer Kombination davon.

7. Verfahren nach Anspruch 6, wobei der genannte Primärtumor ausgewählt ist aus der Gruppe, bestehend aus einem Plattenepithelkarzinom, einem Adenokarzinom, einem großzelligen Karzinom und einer Kombination davon.

8. Verfahren nach Anspruch 6, wobei der genannte Sekundärtumor ein metastatischer Tumor ist.

9. Verfahren nach Anspruch 1, wobei ein Verhältnis der Menge und/oder Aktivität des genannten PSME4 zur Menge und/oder Aktivität der genannten mindestens einen immunoproteasomalen katalytischen Untereinheit von größer als mindestens 2:1 den genannten nicht kleinzelligen Lungenkrebs anzeigt.

10. Verfahren nach Anspruch 1, wobei ein Verhältnis der Menge und/oder Aktivität des genannten PSME4 zur Menge und/oder Aktivität der genannten mindestens einen immunoproteasomalen katalytischen Untereinheit von größer als mindestens 3:1 den genannten nicht kleinzelligen Lungenkrebs anzeigt.

11. Verfahren nach Anspruch 1, wobei ein Verhältnis der Menge und/oder Aktivität des genannten PSME4 zur Menge und/oder Aktivität der genannten mindestens einen immunoproteasomalen katalytischen Untereinheit von größer als mindestens 4:1 den genannten nicht kleinzelligen Lungenkrebs anzeigt.

12. Verfahren nach Anspruch 4, wobei ein Verhältnis der Menge des genannten PSME4-Proteins zur Menge der genannten mindestens einen immunoproteasomalen katalytischen Untereinheit von größer als mindestens 2:1 die Eignung des genannten Probanden für eine Behandlung mit dem genannten Immun-Checkpoint-Inhibitor anzeigt.

13. Verfahren nach Anspruch 4, wobei ein Verhältnis der Menge des genannten PSME4-Proteins zur Menge der genannten mindestens einen immunoproteasomalen katalytischen Untereinheit von größer als mindestens 3:1 die Eignung des genannten Probanden für eine Behandlung mit dem genannten Immun-Checkpoint-Inhibitor anzeigt.

14. Verfahren nach Anspruch 4, wobei ein Verhältnis der Menge des genannten PSME4-Proteins zur Menge der genannten mindestens einen immunoproteasomalen katalytischen Untereinheit von größer als mindestens 4:1 die Eignung des genannten Probanden für eine Behandlung mit dem genannten Immun-Checkpoint-Inhibitor anzeigt.

## Revendications

1. Méthode de diagnostic du cancer du poumon non à petites cellules chez un sujet comprenant l'analyse d'une quantité et/ou d'une activité de PSME4 et d'au moins une sous-unité catalytique d'immunoprotéasome choisie dans le groupe constitué par la PSMB8, la PSMB9 et la PSMB10 présentes dans un échantillon de tumeur du cancer du poumon non à petites cellules dudit sujet, dans laquelle une augmentation d'un rapport entre la quantité et/ou l'activité de ladite PSME4 et la quantité et/ou l'activité de ladite au moins une sous-unité catalytique d'immunoprotéasome par comparaison avec ledit rapport dans un échantillon non tumoral est indicative dudit cancer du poumon non à petites cellules.

2. Méthode selon la revendication 1, dans laquelle ladite au moins une unité catalytique d'immunoprotéasome est la PSMB8.

3. Méthode selon la revendication 1, dans laquelle ladite au moins une unité catalytique d'immunoprotéasome est la PSMB10.

4. Méthode de sélection d'un traitement pour un sujet diagnostiqué avec un cancer, la méthode comprenant la détermination d'une quantité de protéine PSME4 et d'une quantité d'au moins une sous-unité catalytique d'immunoprotéasome choisie dans le groupe constitué par la PSMB8, la PSMB9 et la PSMB10 présentes dans les cellules cancéreuses dudit sujet, dans laquelle un rapport entre la quantité de ladite protéine PSME4 et la quantité de ladite au moins une sous-unité catalytique d'immunoprotéasome étant inférieur à un seuil prédéterminé est indicatif de l'adéquation dudit sujet à un traitement avec un inhibiteur de point de contrôle immunitaire.

5. Méthode selon la revendication 4, dans laquelle ledit cancer est choisi dans le groupe constitué par l'adénocarcinome du côlon, le CPNPC (NSCLC) et l'adénocarcinome de l'estomac.

6. Méthode selon la revendication 1, dans laquelle ladite tumeur du cancer du poumon non à petites cellules est choisie dans le groupe constitué d'une tumeur primaire, d'une tumeur secondaire, d'une tumeur récurrente, d'une tumeur réfractaire et d'une combinaison de celles-ci.

7. Méthode selon la revendication 6, dans laquelle ladite tumeur primaire est choisie dans le groupe constitué par un carcinome à cellules squameuses, un adénocarcinome, un carcinome à grandes cellules et une combinaison de ceux-ci.

8. Méthode selon la revendication 6, dans laquelle ladite tumeur secondaire est une tumeur métastatique.

9. Méthode selon la revendication 1, dans laquelle un rapport entre la quantité et/ou l'activité de ladite PSME4 et la quantité et/ou l'activité de ladite sous-unité catalytique d'immunoprotéasome supérieur à au moins 2:1 est indicatif dudit cancer du poumon non à petites cellules.

10. Méthode selon la revendication 1, dans laquelle un rapport entre la quantité et/ou l'activité de ladite PSME4 et la quantité et/ou l'activité de ladite sous-unité catalytique d'immunoprotéasome supérieur à au moins 3:1 est indicatif dudit cancer du poumon non à petites cellules.

11. Méthode selon la revendication 1, dans laquelle un rapport entre la quantité et/ou l'activité de ladite PSME4 et la quantité et/ou l'activité de ladite sous-unité catalytique d'immunoprotéasome supérieur à au moins 4:1 est indicatif du cancer du poumon non à petites cellules.

12. Méthode selon la revendication 4, dans laquelle un rapport entre la quantité de ladite protéine PSME4 et la quantité de ladite au moins une sous-unité catalytique d'immunoprotéasome supérieur à au moins 2:1 est indicatif que le sujet est apte à recevoir un traitement avec ledit inhibiteur du point de contrôle immunitaire.

13. Méthode selon la revendication 4, dans laquelle un rapport entre la quantité de ladite protéine PSME4 et la quantité de ladite au moins une sous-unité catalytique d'immunoprotéasome supérieur à au moins 3:1 est indicatif que le sujet est apte à recevoir un traitement avec ledit inhibiteur du point de contrôle immunitaire.

14. Méthode selon la revendication 4, dans laquelle un rapport entre la quantité de ladite protéine PSME4 et la quantité de ladite au moins une sous-unité catalytique d'immunoprotéasome supérieur à au moins 4:1 est indicatif que le sujet est apte à recevoir un traitement avec ledit inhibiteur du point de contrôle immunitaire.
